# EUROPÄISCHE PATENTSCHRIFT

(11) **EP 2 623 086 B1**
(45) Veröffentlichungstag und Bekanntmachung des Hinweises auf die Patenterteilung: **10.07.2019**
(21) Anmeldenummer: 13000544.0
(22) Anmeldetag: 02.02.2013
(51) Int. Cl.: A61K 6/00, A61K 6/083

(54) **Härtbares Gemisch umfassend Weichmacher mit einem polyalicyclischen Strukturelement zur Anwendung bei der Herstellung dentaler Werkstoffe.**
Curable mixture comprising plasticisers with a polyalicyclic structure element for use in the production of dental materials.
Mélange durcissable comprenant un plastifiant avec un élément structurel polyalicyclique destiné à être utilisé dans la fabrication de matériaux dentaires

(30) Priorität: 02.02.2012 DE 102012001979
(43) Veröffentlichungstag der Anmeldung: 07.08.2013
(73) Patentinhaber: VOCO GmbH, 27472 Cuxhaven (DE)
(72) Erfinder: Blömker, Tobias, 20251 Hamburg (DE); Fontein, Nils, 27472 Cuxhaven (DE); Stepputtis, Manfred, 27449 Kutenholz (DE); Plaumann, Manfred Thomas, 27474 Cuxhaven (DE)

(56) Entgegenhaltungen:
- EP-A1- 1 978 048
- DE-C- 934 889
- US-A1- 2011 250 558
- US-B2- 6 642 286
- DATABASE WPI Week 198407 Thomson Scientific, London, GB; AN 1984-039676 XP002724654, & JP S59 1556 A (CHISSO CORP) 6. Januar 1984 (1984-01-06)

## Beschreibung

Die vorliegende Erfindung betrifft dentale härtbare Gemische oder Massen umfassend Weichmacher mit einem polyalicyclischen Strukturelement zur Anwendung in einem therapeutischen Verfahren bei der Herstellung dentaler Werkstoffe.

Die aus den härtbaren Dentalmassen erhältlichen Werkstoffe sind besonders geeignet als Füllungsmaterialien, Stumpfaufbaumaterialien, provisorische Kronen- und Brückenmaterialien, Befestigungszemente, Unterfütterungsmaterialien, Konditioniermittel, zahntechnische Werkstoffe, Unterfüllungsmaterialien, Abdeckmassen zum Gingivaschutz, Prothesenmaterialien, als Materialien einer provisorischen Suprakonstruktion für ein dentales Implantat oder eines Kerns für eine provisorische Suprakonstruktion oder als Inlays, Onlays und Verblendungen.

Je nach Indikation können die Dentalmassen in Form von dünnfließenden Materialien bis zu hochpastösen Formulierungen eingesetzt werden. Dentalmassen härten in der Regel radikalisch aus und enthalten neben den vernetzbaren Monomeren und den Initiatoren/Katalysatoren noch Füllstoffe sowie spezielle Additive, die ganz unterschiedliche Funktionen übernehmen. Neben der radikalischen Härtung gibt es auch andere Formen der Härtung, beispielsweise die Trocknung in oralen Abgabe- oder Lacksystemen zur Herstellung eines Trägers für dentale Wirkstoffe oder die Abbindung eines zunächst flüssigen Wundverbands zum Schutz der Schleimhaut.

Die härtbare dentale Masse beziehungsweise das härtbare dentale Gemisch wird nach der Härtung zum dentalen Werkstoff.

Ganz spezielle Additive in härtbaren Dentalmassen stellen die gegenüber den Reaktivkomponenten chemisch inerten organischen Substanzen, auch inerte Lösungsmittel oder Weichmacher genannt, dar. Je nach Einsatzgebiet, Struktur und Menge erfüllen die inerten organischen Substanzen oder Weichmacher unterschiedliche Anforderungen.

Die Verwendung von Weichmachern in härtbaren Dentalmassen ist aus dem Stand der Technik gut bekannt.

Unter dem Begriff "(Meth)acryl" ist im Rahmen des vorliegenden Textes sowohl "Acryl" als auch "Methacryl" zu verstehen.

DE 101 47 125 A1 beschreibt Harzzusammensetzungen für ein weiches Unterfüllungsmaterial. Wurden bisher als Komponente zum Weichmachen eines gehärteten Materials in den Harzzusammensetzungen für ein weiches Unterfütterungsmaterial Weichmacher auf Phthalatbasis eingesetzt, so werden in der Druckschrift alternative Weichmacher vorgeschlagen, da es heutzutage bekannt ist, dass Phthalate als endokrine Disruptoren den menschlichen Körper möglicherweise negativ beeinflussen können. In der Anmeldung werden als Weichmacher Säureester, ausgewählt aus der Gruppe bestehend aus Trimellitsäureestern, Fettsäureestern, Essigsäureestern, Maleinsäureestern, Fumarsäureestern und Zitronensäureestern offenbart. Explizit benannt sind Tri-2-ethylhexyltrimellitat, Dimethyladipat, Dibutyladipat, Diisobutyladipat, Diisonorbornyladipat, Di-2-ethylhexyladipat, Diisodecyladipat, Diethylenglykoladipat, Dibutyldiglykoladipat, Di-2-ethylhexylazelat, Dimethylsebacat, Dibutylsebacat, Di-2-ethylhexylsebacat, Methylacetylricinolat, epoxydiertes Sojaöl, Glyceryltriacetat, 2-Ethylhexylacetat, Dimethylmaleat, Dibutylmaleat, Di-2-ethylhexylmaleat, Dibutylfumarat, Di-2-ethylhexylfumarat, Trimethylcitrat, Triethylcitrat, Tripropylcitrat und Triisobutylcitrat.

Bevorzugt sind von diesen Verbindungen Diisobutyladipat, Diisobornyladipat, Dibutylsebacat und Tributylcitrat. Diese Verbindungen können einzeln oder in Gemischen zur Herstellung eines weichen Unterfüllungsmaterials verwendet werden, ohne dass sie in Körpern von Lebewesen als endokrine Disruptoren wirken können. Die Menge des Weichmachers soll zwischen 20 und 80 Gew.-% bezogen auf die Gesamtmasse des weichen Unterfüllungsmaterials betragen. Liegt die Menge unter 20 Gew.-%, dann wird der Masse keine ausreichende Weichheit nach der Polymerisation verliehen, liegt die Masse über 80 Gew.-% wird die Masse für eine weiches Unterfüllungsmaterial zu weich.

Die Harzzusammensetzungen werden in Form Pulver/Flüssigkeit, Flüssigkeit/Flüssigkeit oder als Paste System in unterschiedlichen Mischungsverhältnissen eingesetzt.

EP 1 194 110 B1 offenbart zweikomponentige Paste/Paste Systeme als provisorische Kronen und Brückenmaterialien, die im Mischungsverhältnis von Basis- zu Katalysatorpaste von 10:1 formuliert sind. Auch solche Systeme enthalten weichmachende Additive. Die polymerisierbare Dentalmasse umfasst Weichmacher in Mengen von 1 bis 30 Gew.-%, vorzugsweise 1 bis 20 Gew.-% und insbesondere 1 bis 15 Gew.-% bezogen auf die Gesamtmasse der Bestandteile. Geeignete Weichmacher sind Polyethylenglykolderivate, Polypropylenglykole, niedermolekulare Polyester, Dibutyl-, Dioctyl-, Dinonyl-, Diphenylphthalat, Di(iso-nonyl)adipat, Tricresylphosphat und Siliconöle.

In den Beispielen dieser Druckschrift wird das 2,2-Bis-4-(2-hydroxyethoxyphenyl)-propan-bis-acetat als Weichmacher in den Katalysatorpasten eingesetzt.

In DE 32 46 654 A1 werden nichtadhäsive dentale Abdruckmassen beschrieben. In diesen Systemen wird die Startkomponente der Abdruckmasse zusammen mit Weichmachern verwendet, wobei als Weichmacher beispielsweise Phthalsäureester, acylierte Zitronensäureester, Polyglykole, Dibenzyltoluol oder polyethoxylierte Sorbitanester geeignet sein sollen.

DE 101 26 476 A1 (oder die DE 102 35 990 A1) betrifft N-Alkylaziridinoprepolymere, die in der Zahnmedizin für Produkte zur Abformung eingesetzt werden. Die Abformmaterialien enthalten auch Weichmacher. So werden in der Druckschrift Weichmacher vom Estertyp wie C₁₂ - C₁₅ - Alkyllactate, Ethyl- oder Butylester der Zitronensäure oder der Acetylzitronensäure, Phthalsäureester längerer verzweigter Alkohole wie Bis(2-ethylhexyl)phthalat oder Phthalsäurepolyester, C₂ bis C₁₈ - Dialkylester von C₂- bis C₆-Dicarbonsäuren, wie Bis(2-ethylhexyl)adipat, Dioctylmalat, Diisopropyladipat, aromatische und aliphatische Sulfonsäureester, wie C₂ bis C₂₀-Alkylsulfonsäureester des Phenols oder von C₁ bis C₁₈- Alkanolen und typische aromatische Weichmacher wie Polyphenyle, Dibenzyltoluol sowie Isomerengemische von C₂₀- bis C₃₀-Aromaten beschrieben. Bevorzugt soll die Verwendung von Gemischen aus Weichmachern des Estertyps und des aromatischen Typs sein. Ein bevorzugtes Gemisch stellt eine Zusammensetzung aus Acetyltributylcitrat und Dibenzyltoluol dar.

In der Patentanmeldung werden zusätzliche Weichmacher mit Molmassen über 2000 g/mol angegeben. Diese Weichmacher gehören unterschiedlichen Verbindungstypen wie dem Polyethertyp, Polyestertyp, Polycarbonattyp, Polyolefintyp, an, wobei als Endgruppen Hydroxyl-, Ether-, Alkyl-, und Acylgruppen bevorzugt sind.

Durch die Benutzung von Weichmachern lassen sich bei vielen Indikationen extreme Mischungsverhältnisse vermeiden.

Ein weiter Vorteil des Einsatzes von Weichmachern ist der Umstand, dass sich, wie oben bereits beschrieben, feste Starterkomponenten in den Weichmachern leicht lösen lassen.

In der DE 197 11 514 B4, die ebenfalls auf Abformmassen zielt, wird zudem festgestellt, dass übliche Weichmacher mit Polyethermaterialien im Allgemeinen gut verträglich sind, so dass ihre Verwendung nicht nur aus wirtschaftlichen Gründen, sondern auch zur Verbesserung der Eigenschaften, insbesondere zur Vermeidung oder Verringerung der Kristallisation, ratsam sei. Geeignet seien beispielsweise Phthalsäureester, Glykolderivate, polymere Weichmacher, Sorbitanester, etc. Übliche Weichmacher seien beispielsweise in "Polyethers, Part I", herausgegeben von Norman G. Gaylord, Interscience Publishers (1963) beschrieben. Der Zusatz größerer Mengen Weichmacher zu den Abdruckmassen könne allerdings die Wasseraufnahme, Quellung und Dimensionsänderung in einem Maß beeinflussen, dass der Abdruck unbrauchbar werde.

In der DE 39 02 417 A1 werden Formmassen zur Herstellung von dentalen Gussmodellen offenbart. Auch hier kommen Weichmacher zum Einsatz. Die Druckschrift spricht von einer "chemisch inerten" Verbindung, die den Massen beigegeben wird, wobei unter "chemisch inert" zu verstehen sei, dass die Verbindung keine Reaktionen mit der organischen polymerisierbaren Masse eingeht. Die "chemisch inerte" Verbindung oder - in unserer Terminologie - der Weichmacher weist somit keine gegenüber den polymerisierbaren Monomeren reaktionsfähigen funktionellen Gruppen, also keine Doppelbindungen, wie polymerisierbare Vinylgruppen, auf.

In der Druckschrift werden als geeignete Weichmacher Biphenyl, 1,2-Diphenylethan, Decanol, 2,4,6-Trimethylnaphthalin, Hexamethylbenzol, Diphenylmethan, 1,1-Diphenylethan, Pentadecan, 2,3-Dimethylbiphenyl, Zimtalkohol, Dibenzylether, Hexaethylbenzol sowie Phthalsäurediethylester angegeben.

DE 199 61 341 C2 ist auf provisorisch K&B Materialien auf Kompositbasis gerichtet, wobei eine Verbesserung der Bruchanfälligkeit bei gleichzeitig hoher Formstabilität dieser Materialien angestrebt ist. Die erfindungsgemäßen Zusammensetzungen enthalten auch Weichmacher in Mengen von 1- 40 Gew.-%, bevorzugt 2 - 30 Gew.-%. Die Weichmacher sollten bevorzugt Viskositäten kleiner als 10 Pas bei 23°C (Kegel-Platte-Viskosimeter) aufweisen. Genannt werden wiederum Polyethylenglykolderivate, Polypropylenglykole, niedermolekulare Polyester, Dibutyl-, Dioctyl-, Dinonyl-, Diphenylphthalat, Di(iso-nonyl)adipat, Tricresylphosphat, Paraffinöle und Siliconöle.

DE 197 54 029 A1 beschreibt ein elastisches Zahnrestaurationsmaterial und Verfahren zur Herstellung von Zahnprothesenmaterial unter Verwendung desselben. Auch in dieser Anmeldung kommen Weichmacher zum Einsatz. Bevorzugt verwendet werden Phthalatweichmacher. Beispiele schließen Phthalatderivate, wie Phthalsäuredimethylester, Phthalsäuredibutylester und Phthalsäuredioctylester ein.

DE 60 2004 009 552 T2 offenbart ein orales Abgabesystem mit einem antibakteriellen und einem entzündungshemmenden Mittel, das für die Behandlung dentaler Krankheiten, insbesondere von periodontalen Krankheiten geeignet ist. Um die Flexibilität des Systems zu verbessern, wird ihr ein Weichmacher oder eine Mischung von Weichmachern zugegeben. Die Art und Menge des Weichmachers soll dabei die Flexibilität der Zusammensetzung bestimmen. Als Beispiele geeigneter Weichmacher werden genannt: Phthalate wie Dimethylphthalat, Dibutylphthalat, Diethylphtalat, Dibutylsebacat, Triethylcitrat, Tributylcitrat, acetylierte Monoglyceride, Acetyltributylcitrat, Triacetin, Benzylbenzoat, GlykolDerivate wie Glycerol, Polyethylenglykole, Propylenglykolbutyl, und/oder Glykolester von Fettsäuren, raffinierte Mineralöle, Ölsäure, Castoröl (Rizinusöl), Kornöl, Kampher, und Zuckeralkohole wie Sorbitol. Bevorzugte Weichmacher sind Sorbitol und Glycerin, wobei Glycerin der am meisten bevorzugte Weichmacher ist. Die bevorzugte Menge an Weichmacher liegt zwischen 1 und 15 Gew.-% und weiter bevorzugt zwischen 4 und 10 Gew.-%.

Die Patentschrift beansprucht einen Weichmacher, der ausgewählt ist aus der Gruppe aus Glykolderivaten, Phthalaten, Citratderivaten, Benzoaten, Butyl- oder Glykolestern von Fettsäuren, hochraffinierten Mineralölen, Kampher, Ölsäure, Castoröl, Maiskeimöl und Zuckeralkoholen.

DE 10 2008 283 306 A1 offenbart ein zweikomponentiges, chemisch härtendes, lagerstabiles dentales Kompositmaterial mit Nanodiamant. Das erfindungsgemäße Material kann zur Einstellung bestimmter Eigenschaften auch Weichmacher enthalten. Genannt sind Polyethylenglykole, Polypropylenglykole, ungesättigte Polyester, Phthalate, Adipate, Sebacate, Phosphorsäureester, Phosphonsäureester und/oder Zitronensäureester.

DE 699 21 231 T2 beschreibt eine Dentalmasse und einen daraus hergestellten künstlichen Zahn. Auch diese Dentalmasse besteht aus einem sogenannten Verbundwerkstoff (Kompositmaterial), d.h. sie enthält sowohl eine organische als auch eine anorganische Phase. Der Verbund zwischen der organischen Harzmatrix und den anorganischen Füllstoffoberflächen wird mit Hilfe von Haftvermittlern sichergestellt. Beispiele für haftvermittelnde Substanzen sind organofunktionelle Silane, Haftvermittler auf Titanat-Basis und auf Zircoaluminat-Basis. In DE 699 21 231 T2 werden etliche Verbindungen für die verschiedenen Typen von Haftvermittlern angegeben. Die Menge des der Dentalmasse zugesetzten Haftvermittlers liegt zwischen 0,1 bis 25 Gewichtsteilen auf 100 Gewichtsteile des Monomers. Beträgt die Menge mehr als 25 Gewichtsteile, so soll ein Überschuss des Haftvermittlers als Weichmacher wirken.

DE 692 31 737 T2 betrifft ein Wurzelkanalfüllmittel und eine Klebezusammensetzung. Das hitzehärtbare Material auf Harzbasis der erfindungsgemäßen Wurzelkanalzusammensetzung enthält auch einen Weichmacher in einer Menge zwischen 0,1 und 30 Gew.-%. Der Zweck des Weichmachers besteht hier darin, das Harz zu erweichen bzw. plastisch zu machen, so dass sich das Material leicht in einen Wurzelkanal einbringen lässt. Als geeignete Weichmacher werden u.a. Dibutoxyethoxyethyladipat, Dioctylphthalat, Dibutylphthalat, Butylbenzylphthalat, Alkylbenzylphthalat, Dialkyladipat, 2-Ethylhexyldiphenylphosphat, Isodecyldiphenylphosphat, Triphenylphosphat sowie weitere Ester genannt.

DE 690 17 484 T2 zielt auf eine chlorhexidinhaltige Zusammensetzung zur Behandlung periodontaler oder anderer Krankheiten, wobei das Chlorhexidin verzögert freigesetzt werden soll. Auch hier wird ein Weichmacher zur Regulierung der Flexibilität der endgültigen getrockneten Zusammensetzung eingesetzt. Der Weichmacher muss dabei in ausreichender Menge zur Verfügung gestellt werden, um zu verhindern, dass die Endzusammensetzung zu spröde ist. Der Weichmacher sollte in einer Menge von 0,01 bis 15 Gew.-% vor dem Trocknen in der Zusammensetzung vorhanden sein. Nach dem Verdampfen von 90% des Lösungsmittels sollen solche Zusammensetzungen 0,01 bis 41 Gew.-% Weichmacher enthalten. Explizit als Weichmacher benannt sind Phthalatester, Phosphatester, Glykolderivate, Kohlenwasserstoffe, Öle oder Fettsäuren, wobei sich Glycerin und Sorbitol als bevorzugte Weichmacher erwiesen haben. Am meisten bevorzugt ist das Glycerin.

Auch die DE 690 33 994 T2 offenbart flüssige Polymerzusammensetzungen zur Verhütung und Behandlung von dentalen oder dermatologischen Krankheitszuständen. Auch hier werden Weichmacher offenbart, etwa Polyethylenglykol 400 bis 4000, Glycerin, Sorbit oder Mineralöl, die in Konzentrationen von etwa 1 Gew.-% in den Zusammensetzungen enthalten sein können.

DE 697 25 380 T2 beschreibt flüssige, lichthärtbare Zusammensetzungen. Solche Zusammensetzungen werden laut Druckschrift im weiten Umfang als Beschichtungsmaterialien, als Photoresits, als dentales Material oder dergleichen eingesetzt. Auch für diese Zusammensetzungen werden Weichmacher offenbart. Illustrative Beispiele schließen Dialkylester von Phthalsäure wie n-Dioctylphthalat, Dimethylphthalat, Diethylphthalat, Dibutylphthalt, Diheptylphthalat, und Di-2-ethylhexylphthalat, Triorganoester von Phosphorsäure wie Tributylphosphat, Tri-2-ethylhexylphophat, Triphenylphosphat und Tricresylphosphat, Dialkylester von Adipinsäure wie Dibutyladipat und Di-n-octyladipat und dergleichen ein. Die hier offenbarte photohärtbare Harzmasse kann erfindungsgemäß einen oder eine Vielzahl der angegebenen Weichmacher enthalten.

DE 698 01 010 T2 betrifft ein prothetisches Restaurationsteil mit ausgezeichneter Bioverträglichkeit, ausreichender Härte und mechanischer Festigkeit sowie Verfahren zu seiner Herstellung. Auch für diese Systeme werden hier Weichmacher offenbart: Phthalatester (DOP, DEP, DBP), Adipinsäureester, Trimellitsäureester und Sebacinsäureester werden genannt.

DE 20 2010 014 676 U1 beschreibt eine Dubliermasse, die auch zur Herstellung von Negativabdrücken im Dentalbereich eingesetzt werden kann. Erfindungsgemäß enthält diese Masse 30 bis 60 Gew.-% mindestens eines mehrwertigen Alkohols. Dieser dient als Weichmacher und soll zu den vorteilhaften elastischen Eigenschaften der hergestellten Negativabdrücke beitragen. Der mindestens eine mehrwertige Alkohol ist vorzugsweise ausgewählt aus der Gruppe umfassend Glycerin, Zuckeralkohole (z.B. Sorbit oder Mannit), Propylenglykol, Polyethylenglykol und Mischungen hiervon, wobei die Verwendung von Glycerin besonders bevorzugt ist.

Besonders vorteilhaft soll es sein, wenn die Dubliermasse 45 bis 55 Gew.-% des mindestens einen mehrwertigen Alkohols, insbesondere Glycerin, umfasse.

DE 199 41 738 B4 zielt auf Füllstoffe zur Kunststoff-Formulierungen auf Polyurethanbasis. Neben den erfindungsgemäßen Füllstoffen können mit den Füllstoffen formulierte Dentalmassen zur Erhöhung der Flexibilität der Massen zusätzlich auch Weichmacher enthalten. Gut geeignet sollen beispielsweise Dibutyl-, Dioctyl- und Dinonylphthalate oder -adipate sowie höhermolekulare Polyphthalsäureester und Adipinsäureester sein.

DE 10 2009 046 251 A1 offenbart ein reaktives einkomponentiges System. Solche Systeme finden besondere Bedeutung in den Bereichen Dichtstoffe und Klebstoffe. Sie können, laut Druckschrift auch im medizinischen Bereich, wie z.B. im Dentalbereich, bei Beschichtungen wie Lacken oder bei Reaktionsharzen wie z.B. Straßenmarkierungen oder Industriefußböden Anwendung finden. Als Weichmacher werden für diese einkomponentigen Systeme vorzugsweise Ester, Polyole, Öle, niedermolekulare Polyether oder Phthalate genannt.

DE 2420351 C3 betrifft die Verwendung einer Zusammensetzung als Wurzelkanalfüllkomponente. Diese dentale Masse benötigt gemäß Druckschrift ein inertes Lösungsmittel (Weichmacher), um die Komponenten zu lösen und später ein Polymerisat zu ergeben, das bei Revision wieder entfernbar ist. Als geeignete Verbindungen werden Wasser, Glycerin und dessen Ester, Propylenglykol, 1,2-Propandiolcarbonat, Pentaerythrit, Diacetin, Monoacetin, Ethylenglykol, Diethylenglykol und Dipropylenglykol angegeben. In den Beispielen dieser Patentschrift wird indes ausschließlich Diacetin (= Glycerindiacetat oder 1,2,3-Propantriol-1,3-diacetat) benutzt.

In der WO 00/78853 A1 werden zur Verwendung als Weichmacher zur Herstellung toxikologisch günstig zu bewertender Kunststoffe eine ganze Reihe von Cyclohexanpolycarbonsäuren sowie deren Derivate angegeben. Neben den Cyclohexanpolycarbonsäuren an sich und deren Derivate werden insbesondere Mono-, Di-oder gegebenenfalls Tri- oder Tetraester sowie Anhydride der Cyclohexanpolycarbonsäuren zur Verwendung in Kunststoffen vorgeschlagen. Die eingesetzten Ester sind Alkyl-, Cycloalkyl- sowie Alkoxyalkylester, wobei die Alkyl-, Cycloalkyl- sowie Alkoxyalkylgruppen in der Regel 1 bis 30, vorzugsweise 2 bis 20 und besonders bevorzugt 3 bis 18 Kohlenstoffatome umfassen und verzweigt oder linear sein können.

Die WO 00/78704 betrifft ausgewählte Cyclohexan-1,3- und -1,4-dicarbonsäureester, ihre Verwendung als Weichmacher in Kunststoffen sowie ihre Herstellung mittels Hydrierung der entsprechenden Isophthalsäure- und Terephthalsäureester durch Inkontaktbringen einer oder mehrerer solcher Isophthalsäure- oder Terephthalsäureester mit einem wasserstoff enthaltenden Gas in Gegenwart eines speziellen Katalysators.

Die EP 1 042 273 B1 beschreibt Verfahren zur Hydrierung von Benzolpolycarbonsäuren oder Derivaten davon unter Verwendung eines Makroporen aufweisenden Katalysators.

Die DE 28 23 165 gibt Verfahren zur Herstellung von cycloaliphatischen Carbonsäureestern an.

Im Stand der Technik zu dentalen härtbaren Massen, die inerte Substanzen, bzw. Weichmacher enthalten und zur Anwendung bei der Herstellung dentaler Werkstoffe oder bei der Herstellung toxikologisch günstig zu bewertender Kunststoffe, die aufgrund ihrer geringen Toxizität ebenfalls als dentale Werkstoffe geeignet wären, vorgesehen sind, ist somit eine Vielzahl an Verbindungen beschrieben.

Bezüglich ihrer Verwendung im dentalen Bereich werden die Weichmacher in einkomponentigen und mehrkomponentigen härtbaren Systemen für die unterschiedlichsten Indikationen benutzt. Sie werden eingesetzt in radikalisch vernetzbaren Systemen ebenso wie in trocknenden oder abbindenden Zusammensetzungen. Man findet sie in photohärtenden und chemisch härtenden dentalen Massen. Sie werden eingesetzt, um mehrkomponentige dentale Massen besser (genauer) abzumischen und um die Eigenschaften des resultierenden Werkstoffs optimaler einzustellen. Der weichmachende, flexibilisierende Effekt dieser inerten Verbindungen kommt zustande, indem die freien, nichtreaktiven Kettenenden der Weichmacher einen regelmäßigen Netzaufbau verhindern. Mit ihren langen Ketten wird das Strukturgerüst verzerrt und aufgeweitet. Sie können sich zwischen die Polymerketten legen und die Ausbildung eines Großteils von Wechselwirkungen zwischen den einzelnen Molekülverbänden stören und verhindern. Ihre Wirkweise ähnelt mitunter einem Gleitmittel, das sich zwischen die einzelnen Polymerschichten legt und an dem die Polymere vorbeigleiten können. Man benutzt die Weichmacher somit als elastifizierende Komponente in Dentalmassen, die das Elastizitätsmodul (sowie die Glasübergangstemperatur) absenken. In diesem Zusammenhang sei darauf hingewiesen, dass das Ausmaß der Abnahme der mechanischen Festigkeit der Werkstoffe bei der Verwendung von vielen Weichmachern aus dem Stand der Technik nachteilig ist.
Darüberhinaus werden Weichmacher auch eingesetzt, um aktiven Einfluss auf die Reaktionskinetik einer härtbaren Masse zu nehmen. Beispielsweise wird für provisorische K&B-Materialien selbst bei Systemen mit Mischungsverhältnissen von 1:1 Weichmacher benötigt, da man eine ausreichend lange elastische Phase der angemischten Masse braucht, die ohne die Verwendung von Weichmachern nur äußerst schwierig einzustellen wäre.

Primäre Aufgabe der vorliegenden Erfindung ist es, härtbare Gemische oder Massen mit weichmachenden Eigenschaften zur Anwendung in einem therapeutischen Verfahren bei der Herstellung eines dentalen Werkstoffs zur Verfügung zu stellen. Die Weichmacher sollen ausgeprägte hydrophobe Eigenschaften aufweisen, die sich u.a. in einer sehr geringen Wasseraufnahme und einer sehr geringen Löslichkeit der unter der Verwendung der Weichmacher erhältlichen dentalen Werkstoffen zeigt. Weiterhin sollen sich die unter Verwendung der Weichmacher erhältlichen dentalen Werkstoffe durch eine gute mechanische Stabilität auszeichnen, die sich u.a. in einer hohen Biegefestigkeit zeigt. Der Einsatz der Weichmacher soll somit keine negativen Effekte auf die mechanischen Werte ausüben. Vorzugsweise sollen sich unter Verwendung der Weichmacher solche dentalen Werkstoffe herstellen lassen, die sowohl eine geringe Wasseraufnahme, eine geringe Löslichkeit als auch eine hohe Biegefestigkeit aufweisen. Zudem sollen die erfindungsgemäßen Kompositmaterialien solche reaktionskinetischen Eigenschaften aufweisen, dass ihre Verarbeitungszeiten für die jeweiligen Indikationen vorteilhaft sind.

Diese Aufgaben werden gelöst durch ein härtbares Gemisch umfassend
(a) eine oder mehrere während des Härtens mit den Bestandteilen (b) radikalisch nicht polymerisationsfähige Verbindung(en) der Struktur Q - [(Y)ₙ - X]ₒ, wobei hier und nachfolgend gilt:
   - Q bedeutet ein polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten Yₙ - X substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind;
   - Y bedeutet Methylen (-CH₂-);
   - n = 0 oder 1;
   - X bedeutet -O-Z, -N-(Z)₂, -NH-Z, -O-C(=O)-Z, -C(=O)-O-Z, -O-C(=O)-NH-Z, -NH-C(=O)-O-Z, -NH-C(=O)-NH-Z, -C(=O)-NH-Z, -NH-C(=O)-Z, -C(=O)-N-(Z)₂, -N-(Z)-C(=O)-Z, -O-C(=O)-N(Z)-C(=O)-NH-Z, -NH-C(=O)-N(Z)-C(=O)-NH-Z, -N(C(=O)-NH-Z)₂, -C(=O)-N(Z)-C(=O)-NH-Z, -N(C(=O)-NH-Z)(C(=O)-Z), -N(C(=O)-NH-Z)(C(=O)-O-Z),
      wobei die jeweils im Formelbild links angeordnete Bindung dem Strukturelement Q näher ist und wobei X so gewählt wird, dass Z möglichst wenige Atome zählt,
   - Z bedeutet ein organischer Rest mit mindestens einem Kohlenstoffatom, wobei verschiedene Z unterschiedlich sein können,
   - o = 2, 3, 4;
   als Weichmacher
   und
(b) weitere Bestandteile gewählt aus der Gruppe bestehend aus
   (b-1) ein oder mehrere verschiedene Monomer(e), bevorzugt gewählt aus der Gruppe bestehend aus (Meth)acrylaten,
   (b-2) einen oder mehrere Füllstoffe,
   (b-3) einen oder mehrere Photoinitiator(en) und/oder einen oder mehrere Initiator(en) für die chemische Aushärtung,
   (b-4) gegebenenfalls einen oder mehrere Polymerisationsinhibitor(en) und
   (b-5) gegebenenfalls ein oder mehrere Lösungsmittel
   zur Anwendung in einem therapeutischen Verfahren bei der Herstellung eines dentalen Werkstoffs.

Verbindungen, die mit Monomeren radikalisch nicht polymerisierbar sind, sind dem Fachmann bekannt. Diese Verbindungen polymerisieren nicht in Gegenwart von sogenannten Initiatorsubstanzen, wie beispielsweise Peroxiden, Hydroperoxiden, Carbonsäureperoxiden oder Azoverbindungen, die unter den Bedingungen dentaler Anwendungen (Normaldruck, Umgebungstemperatur oder Mundtemperatur) zerfallen und freie Radikale bilden. Weiter unten werden gängige Initiatorsysteme, auch photochemisch wirksame, benannt. Diese Verbindungen enthalten keine aktivierten ethylenisch ungesättigten Bindungen, wie (Meth)acrylatfunktionen, Vinyletherfunktionen, Allylfunktionen oder Doppelbindungen, die von Estergruppen flankiert sind, wie sie sich bei ungesättigten Polyestern finden. Hierzu zählen auch Vinyl- und Vinylidengruppen, bei denen die Doppelbindung durch Halogenatome, Amin-, Thiol-, Ester, Säure-, Cyan- oder Arylgruppen durch direkte Substitution aktiviert ist und somit in Gegenwart von Initiatoren unter den Bedingungen dentaler Anwendungen mit Monomeren radikalisch reagieren kann.

Selbstverständlich reagieren diese Verbindungen auch nicht in kettenverlängernden Schritten mit kohlenstoff- oder heteroatomzentrierten Radikalen.

Die unter Verwendung der Weichmacher erhältlichen dentalen Werkstoffe weisen eine ausgeprägte Hydrophobie auf, die sich u.a. in einer sehr geringen Wasseraufnahme und in einer sehr geringen Löslichkeit der Polymerisate zeigt. Daneben zeichnen sich die unter der Verwendung der Weichmacher erhältlichen dentalen Werkstoffe durch eine hohe mechanische Stabilität aus, die sich u.a. in einer hohen Biegefestigkeit der Polymerisate zeigt. Die Verwendung der Weichmacher führt zu Polymerisaten, die sowohl eine geringe Wasseraufnahme, eine geringe Löslichkeit als auch eine hohe Biegefestigkeit aufweisen. Das polyalicyclische Strukturelement Q trägt zu der hohen Hydrophobie bei, die sich u.a. in einer sehr geringen Wasseraufnahme und sehr geringen Löslichkeit der Polymerisate zeigt.

Es wurde weiterhin gefunden, dass die Verwendung der Weichmacher zu gut verarbeitbaren Gemischen führt Die unter der Verwendung der Weichmacher formulierten härtbaren Zusammensetzungen lassen sich einfach herstellen, wobei die ausgehärteten Polymerisate bzw. Formstoffe einen geringen Schrumpf, eine gute Haftung auf verschiedenen Untergründen, eine hohe Hydrolysebeständigkeit, eine geringe Wasseraufnahme, eine geringe Löslichkeit und eine hohe mechanische Festigkeit aufweisen. Die genannten Eigenschaften sind im Bereich der Dentaltechnik äußerst wichtig.

Vorzugsweise werden die Weichmacher in Zusammensetzungen verwendet, die radikalisch vernetzbar sind. Es wurde beobachtet, dass sich die Weichmacher stabil in den entsprechenden organischen Matrices der radikalisch härtbaren Zusammensetzungen sowohl in den flüssigen oder pastösen, noch nicht gehärteten Zusammensetzungen als auch in den vernetzten Polymerisaten halten und keine Tendenz zum Auswandern zeigen. Möglicherweise bilden die funktionellen Gruppen, die am polyalicyclischen Kern der Weichmacher anbinden und polare Elemente aufweisen, eine beständige und dauerhaft übergeordnete Domänenstruktur, die für die Integrität des Weichmachers im Strukturumfeld der vorzugsweise radikalisch härtbaren Zusammensetzungen aufkommt.

Bei dem "polyalicyclischen" Strukturelement Q handelt es sich um einen bicyclischen, tricyclischen, tetracyclischen, pentacyclischen oder hexacyclischen Kohlenwasserstoffrest, wie oben definiert. Die Bezeichnungen "bicyclisch", "tricyclisch", tetracyclisch", "pentacyclisch" und "hexacyclisch" entsprechen dabei der IUPAC-Nomenklatur.

Ein geeigneter Weichmacher umfasst mindestens ein polyalicyclisches Strukturelement Q, das von einem entsprechenden polyalicyclischen Kohlenwasserstoff abgeleitet ist. Dies bedeutet im Rahmen des vorliegenden Textes, dass o Wasserstoffatome des Kohlenwasserstoffs durch Substituenten Yₙ - X ersetzt sind, und optional ein, zwei oder mehr der nicht durch Substituenten Yₙ - X substituierten Wasserstoffatome durch Alkylgruppen, Alkoxygruppen, Halogenatome oder Trifluormethylgruppen substituiert sind.

Die Struktur unsubstituierter Bicyclen ist wie folgt: 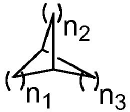 wobei n₁, n₂ und n₃ jeweils unabhängig voneinander eine natürliche Zahl von 1 bis 8 bedeuten können, vorzugsweise eine natürliche Zahl von 1 bis 4.

Exemplarisch genannt seien hier:

| | |
|---|---|
| Für n₁ = n₂ = 1; n₃ = 2 | Bicyclo[2.1.1]hexan |
| für n₁ =1; n₂ = n₃ = 2 | Bicyclo[2.2.1]heptan |
| für n₁ = n₂ = 1; n₃ = 3 | Bicyclo[3.1.1]heptan |
| für n₁ = n₂ = n₃ = 2 | Bicyclo[2.2.2]octan |
| für n₁ = n₂ = 1; n₃ = 4 | Bicyclo[4.1.1]octan |
| für n₁ =1; n₂ = 2; n₃ = 3 | Bicyclo[3.2.1]octan |
| für n₁ =1; n₂ = 2; n₃ = 4 | Bicyclo[4.2.1]nonan |
| für n₁ = n₂ = 2; n₃ = 4 | Bicyclo[4.2.2]decan |

Nachfolgend werden exemplarisch einige disubstituierte Bicyclen gezeigt: 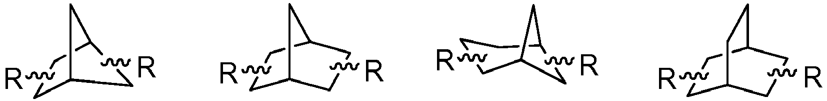 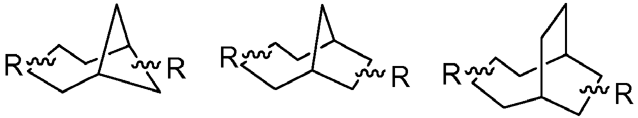 wobei R jeweils die sonstigen Reste der Verbindung bedeutet.

Beispiele für bicyclische Strukturelemente Q sind das Bicyclo[1.1.1]pentan-, das Bicyclo[2.1.1]hexan-, das Bicyclo[2.2.1]heptan-, das Bicyclo[3.1.1]heptan-, das Bicyclo[2.2.2]octan-, das Bicyclo[4.1.1]octan-, das Bicyclo[3.2.1]octan-, das Bicyclo[4.2.1]nonan-, das Bicyclo[3.3.1]nonan-, das Bicyclo[5.1.1]nonan-, das Bicyclo[3.2.2]nonan-, das Bicyclo[6.1.1]decan-, das Bicyclo[5.2.1]decan-, das Bicyclo[4.2.2]decan-, das Bicyclo[3.3.2]decan-, das Bicyclo[7.1.1]undecan-, das Bicyclo[6.2.1]undecan-, das Bicyclo[5.2.2]undecan-, das Bicyclo[4.3.2]undecan-, das Bicyclo[3.3.3]undecan-, das Bicyclo[8.1.1]dodecan-, das Bicyclo[7.2.1]dodecan-, das Bicyclo[6.2.2]dodecan-, das Bicyclo[5.3.2]dodecan-, das Bicyclo[4.3.3]dodecan-, das Bicyclo[4.4.2]dodecan-, das Bicyclo[5.4.1]dodecanstrukturelement sowie noch höhere Strukturelemente wie die entsprechenden Tridecane, Tetradecane, Pentadecane, etc.

Für unsubstituierte Tricyclen sind z.B. folgende Strukturen möglich: 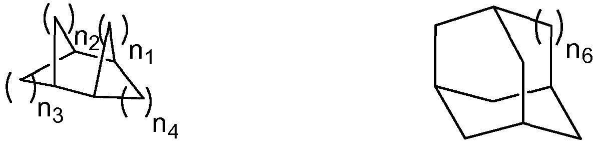 wobei n₁, n₂, n₃, n₄ bzw. n₆ jeweils unabhängig voneinander eine natürliche Zahl von 0 bis 5 bedeuten können.

Exemplarisch genannt seien:

| | |
|---|---|
| Für n₁ = 2; n₂ = 0; n₃ = 2; n₄ = 3 | Tricyclo[4.3.2.0^{2,5}]undecan |
| für n₁ = 0; n₂ = 1; n₃ = 2; n₄ = 3 | Tricyclo[5.2.1.0^{2,6}]decan |
| für n₁ = 0; n₂ = 2; n₃ = 2; n₄ = 3 | Tricyclo[5.2.2.0^{2,6}]undecan |
| für n₁ = 2; n₂ = 0; n₃ = 2; n₄ = 2 | Tricyclo[4.2.2.0^{2,5}]decan |
| für n₆ = 1 | Tricyclo[3.3.1.1^{3,7}]decan |

Nachfolgend werden exemplarisch einige di- oder trisubstituierte Tricyclen gezeigt: 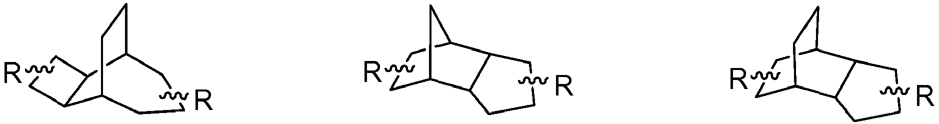 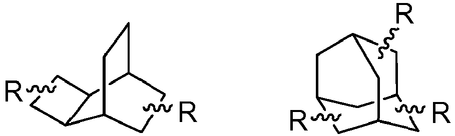 wobei R jeweils die sonstigen Reste der Verbindung bedeutet.

Beispiele für tricyclische Strukturelemente Q sind das Tricyclo[3.2.1.0^{2,6}]octan-, das Tricyclo[4.2.1.0^{2,6}]nonan-, das Tricyclo[5.2.1.0^{2,6}]decan-, das Tricyclo[6.2.1.0^{2,6}]undecan-, das Tricyclo[7.2.1.0^{2,6}]dodecan-, oder das Tricyclo[4.2.1.1^{2,5}]decan-, das Tricyclo[4.3.1.1 ^{2,5}]decan-, das Tricyclo[4.4.1.1 ^{2,5}]decan-, das Tricyclo[2.2.1.0^{2,6}]heptan-, das Tricyclo[2.2.2.0^{2,6}]octan-, das Tricyclo[3.2.2.0^{2,6}]nonan-, das Tricyclo[3.3.1.1^{3,7}]decan-, das Tricyclo[3.2.1.1^{3,7}]nonan-, das Tricyclo[4.2.2.2^{2,5}]dodecan-, das Tricyclo[4.3.2.2^{2,5}]tridecan-, das Tricyclo[4.4.2.2^{2,5}]tetradecan-, das Tricyclo[4.2.1.0^{3,7}]nonan-, das Tricyclo[4.4.1.1^{1,5}]dodecan-, das Tricyclo[6.2.1.0^{2,7}]undecan-, das Tricyclo[5.2.2.0^{2,6}]undecan, das Tricyclo[6.2.2.0^{2,7}]dodecan-, Tricyclo[4.3.2.0^{2,5}]undecan-, das Tricyclo[4.2.2.0^{2,5}]decan-oder das Tricyclo[5.5.1.0^{3,11}]tridecanstrukturelement.

Für unsubstituierte Tetracyclen ist z.B. die folgende Struktur möglich: 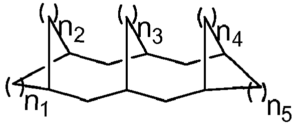 wobei n₁, n₂, n₃, n₄ und n₅ jeweils unabhängig voneinander eine natürliche Zahl von 1 bis 5 bedeuten können.

Exemplarisch genannt seien hier:

| | |
|---|---|
| Für n₁ = n₂ = n₃ = n₄ = 2; n₅ = 5 | Tetracyclo[9.6.2^{3,9}.2^{13,16}]tricosan |
| für n₁ = n₅ = 2; n₂ = n₃ = n₄ = 1 | Tetracyclo[6.6.1³⁶.1^{10,13}]heptadecan |
| für n₁ = n₂ = n₃ = n₄ = n₅ = 2 | Tetracyclo[6.6.2^{3,6}.2^{10,13}]eicosan |

Nachfolgend werden exemplarisch einige disubstituierte Tetracyclen gezeigt 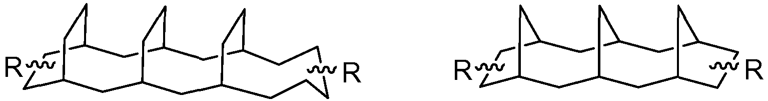 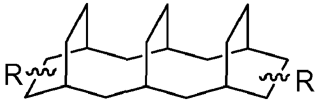 wobei R jeweils die sonstigen Reste der Verbindung bedeutet.

Beispiele für tetracyclische Strukturelemente Q sind das Tetracyclo[4.4.2.2^{2,5}.1^{7,10}]pentadecan-, das Tetracyclo[5.5.2.2^{2,6}.1^{8,12}]heptadecan-, das Tetracyclo[6.6.2.2^{2,7}.1^{9,14}]nonadecan-, das Tetracyclo[4.4.2.2^{2,5}.2^{7,10}]hexadecan-, das Tetracyclo[5.4.2.2^{2,6}.1^{8,11}] hexadecan-, das Tetracyclo[6.2.1.1^{3,6}.0^{2,7}]dodecan, das Tetracyclo[9.6.2^{3,9}.2^{13,16}]tricosan-, das Tetracyclo[9.6.2^{3,9}.2^{13,16}]tricosan-, das Tetracyclo[6.6.1^{3,6}.1^{10,13}]heptadecan-, das Tetracyclo[6.6.2^{3,6}.2^{10,13}]cosan-, oder das Tetracyclo[5.3.2.1^{2,4}.0^{3,6}]tridecanstrukturelement.

Beispiele für pentacyclische Strukturelemente Q sind das Pentacyclo[4.2.0.0^{2,5}.0^{3,8}.0^{4,7}]octan-, das Pentacyclo[13.7.4.3^{3,8}.0^{18,20}.1^{13,28}]triacontan-, das Pentacyclo[8.6.6.5^{2,9}.1^{23,26}]octacosan oder das Pentacyclo[3.3.0.0^{2,4}.0^{3,7}.0^{6,8}]octan-Strukturelement.

Ein Beispiel eines hexacyclischen Strukturelementes Q ist das Hexacyclo[15.3.2.2^{3,7}.1^{2,12}.0^{13,21}.0^{11,25}]pentacosan.

Bevorzugte Verbindungen zur Herstellung der Weichmacher sind beispielsweise:
Carbonsäuresubstituierte polyalicyclische Kohlenwasserstoffe:
   Bicyclo[3.2.2]nonan-6,7-dicarbonsäure,
   Bicyclo[3.3.1]nonan-1,5-dicarbonsäure,
   Tricyclo[3.2.2.0^{2,4}]non-8-en-6,7-dicarbonsäure,
   Bicyclo(2.2.1)-2,5-heptadien-2,3-dicarbonsäure,
   Bicyclo[2.2.1]heptan-5,6-dicarbonsäure,
   Norbornan-2,3-dicarbonsäure,
   Bicyclo[2.2.1]hept-5-en-2,3-dicarbonsäure,
   Pentacyclo[4.4.0.0^{2,5}.0^{3,8}.0^{4,7}]decan-2,4-dicarbonsäure,
   Bicyclo[2.2.2]octan-1,4-dicarbonsäure, etc.
Alkoholsubstituierte polyalicyclische Kohlenwasserstoffe:
   Bicyclo(2.2.1)heptan-2,7-diol,
   [5-(Hydroxymethyl)-6-bicyclo[2.2.1]hept-2-enyl] -methanol,
   Tricyclo[3.3.1.1^{3,7}]decan-1,3-diethanol,
   Tetracyclo[6.3.0.0^{2,6}.0^{5,9}]undecan-3,11-diol,
   [6-(Hydroxymethyl)-6-bicyclo[2.2.1]hept-2-enyl]methanol,
   Tricyclo[3.3.^{13,7}]decan-1,3-diol,
   Bis(hydroxymethyl)tricyclo[5.2.1.0^{2,6}]decan, etc.
Isocyanatsubstituierte polyalicyclische Kohlenwasserstoffe:
   Bis(2-isocyanatoethyl)-5-norbornen-2,3-dicarboxylate,
   2,5 (2,6)-Bis(isocyanatomethyl)bicyclo[2.2.1]heptan,
   Bis(isocyanatomethyl)tricyclo[5.2.1.0^{2,6}]decan, etc.
Gemischt- oder Aminosubstituierte polyalicyclische Kohlenwasserstoffe:
   Tricyclo[2.2.1.0^{2,6}]heptan-1,3-dicarbonsäure-3-amino,
   Tricyclo[3.3.1.1^{3,7}]decan-1-ol-3-amino,
   Pentacyclo[4.3.0.0^{2,5}.0^{3,8}.0^{4,7}]nonan-2,4-diamin,
   Tricyclo[3.3.1.1^{3,7}]decan-1-ol-3-amino,
   Bis(aminomethyl)tricyclo[5.2.1.0^{2,6}]decan, etc.

In einer bevorzugten Ausführungsform leitet sich die Struktur des polyalicyclischen Strukturelements Q von einem bicyclischen [a.c.d] Kohlenwasserstoff ab. Die Buchstaben a, c und d sind natürliche Zahlen und haben die Bedeutung der IUPAC-Nomenklatur. Die Summe von a, c und d liegt vorzugsweise im Bereich von 3 bis 13, bevorzugt im Bereich von 4 bis 7.

In einer weiteren bevorzugten Ausführungsform leitet sich die Struktur des polyalicyclischen Strukturelements von einem tricyclischen [a.c.d.f]-Kohlenwasserstoff ab. Die Summe von a, c, d und f liegt vorzugsweise im Bereich von 6 bis 12, bevorzugt im Bereich von 7 bis 9.

In einer bevorzugten Ausführungsform leitet sich die Struktur des polyalicyclischen Strukturelements von einem tricyclischen [a.2.1,0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen[a.2.2.0^{2,(a+1)}] Kohlenwasserstoff ab, wobei a jeweils die Zahl 3, 4, 5, 6 oder 7 bedeuten kann.

In einer weiteren bevorzugten Ausführungsform leitet sich die Struktur des polyalicyclischen Strukturelements von einem tricyclischen[a.3.1.1] Kohlenwasserstoff ab, wobei a jeweils die Zahl 3, 4, 5, 6 oder 7 bedeuten kann.

Bevorzugt ist ein härtbares Gemisch zur Anwendung in einem therapeutischen Verfahren bei der Herstellung eines dentalen Werkstoffs, wobei die radikalisch nicht polymerisationsfähigen Verbindung der Struktur Q - [(Y)ₙ - X]ₒ als Weichmacher so aufgebaut ist, dass Z ausgewählt ist aus der Gruppe bestehend aus:
a.) Kohlenwasserstoffresten [-R₁], wobei die Anzahl der C-Atome 1 bis 30, vorzugsweise 1 bis 15 und besonders bevorzugt 1 bis 9 beträgt und wobei die Reste linear oder verzweigt sein können und
b.) Kohlenwasserstoffetherresten [(-R₂ - O)_{q} - R₃], wobei die Anzahl der C-Atome für R₂ 2 bis 6, vorzugsweise 2 bis 4 und besonders bevorzugt 2 bis 3 beträgt und wobei die Anzahl der C-Atome für R₃ 1 bis 20, vorzugsweise 1 bis 10 und besonders bevorzugt 1 bis 5 beträgt und wobei R₂ und R₃ linear oder verzweigt sein können und wobei q = 1 bis 15, vorzugsweise 1 bis 10 und besonders bevorzugt 1 bis 5 ist und
c.) Kohlenwasserstoffesterresten [- R₄ - (C=O) - O - R₅] und [-R₄ - O - (C=O) - R₅], wobei die Anzahl der C-Atome für R₄ und R₅ 1 bis 15, vorzugsweise 1 bis 9 und besonders bevorzugt 1 bis 4 beträgt und wobei R₄ und R₅ linear oder verzweigt sein können und
d.) Kohlenwasserstoffesterresten [- R₄ - ((C=O) - O - R₅)₂] und [-R₄ - (O - (C=O) - R₅)₂] und
e.) Kohlenwasserstoffaminoresten [- R₄ - N - (R₅)₂] und
f.) alkoxylierten Kohlenwasserstoffesterresten [(-R₂ - O)_{q} - R₄ - (C=O) - O - R₅] und [( -R₂ - O)_{q} - R₄ - O - (C=O) - R₅] und
g.) substituierten alkoxylierten Kohlenwasserstoffesterresten [(-R₂ - O)_{q} - CH₂ - O - (C=O) - NH - R₄ - (C=O) - O - R₅] und
h.) Kohlenwasserstoffalkoholresten mit endständiger Hydroxylgruppe [- R₄ - OH] und
i.) Kohlenwasserstoffalkoholresten mit einer, zwei oder mehreren nicht endständiger/endständigen Hydroxylgruppe(n) und bis zu 30 C-Atomen und
j.) alkoxylierten Kohlenwasserstoffalkoholresten [(- R₂ - O)_{q} - R₄ - OH] und
k.) Ketonresten [- R₆ - (C=O) - R₇], wobei die Anzahl der C-Atome für R₆ 1 bis 30, vorzugsweise 1 bis 20 und besonders bevorzugt 1 bis 10 beträgt und wobei die Anzahl der C-Atome für R₇ 1 bis 30, vorzugsweise 1 bis 10 und ganz bevorzugt 1 beträgt und wobei und wobei R₆ und R₇ linear oder verzweigt sein können.

Besonders bevorzugt ist ein härtbares Gemisch zur Anwendung in einem therapeutischen Verfahren bei der Herstellung eines dentalen Werkstoffs, wobei die radikalisch nicht polymerisationsfähigen Verbindung der Struktur Q - [(Y)ₙ - X]ₒ als Weichmacher so zusammengesetzt ist, dass Z ausgewählt ist aus der Gruppe bestehend aus:
a.) Kohlenwasserstoffresten [-R₁], wobei die Anzahl der C-Atome 1 bis 9 beträgt und wobei die Reste linear oder verzweigt sein können und
b.) Kohlenwasserstoffetherresten [(-R₂ - O)_{q} - R₃], wobei die Anzahl der C-Atome für R₂ 2 bis 3 beträgt und wobei die Anzahl der C-Atome für R₃ 1 bis 5 beträgt und wobei R₂ und R₃ linear oder verzweigt sein können und wobei q = 1 bis 5 ist und
c.) Kohlenwasserstoffesterresten [- R₄ - (C=O) - O - R₅] und [-R₄ - O - (C=O) - R₅], wobei die Anzahl der C-Atome für R₄ und R₅ 1 bis 4 beträgt und wobei R₄ und R₅ linear oder verzweigt sein können und
d.) Kohlenwasserstoffesterresten [- R₄ - ((C=O) - O - R₅)₂] und [-R₄ - (O - (C=O) - R₅)₂] und
e.) Kohlenwasserstoffaminoresten [- R₄ - N - (R₅)₂] und
f.) alkoxylierten Kohlenwasserstoffesterresten [(-R₂ - O)_{q} - R₄ - (C=O) - O - R₅] und [( -R₂ - O)_{q} - R₄ - O - (C=O) - R₅] und
g.) substituierten alkoxylierten Kohlenwasserstoffesterresten [(-R₂ - O)_{q} - CH₂ - O - (C=O) - NH - R₄ - (C=O) - O - R₅] und
h.) Kohlenwasserstoffalkoholresten [- R₄ - OH] und
i.) Kohlenwasserstoffalkoholresten mit einer, zwei oder mehreren nicht endständiger/endständigen Hydroxylgruppe(n) und bis zu 30 C-Atomen und
j.) alkoxylierten Kohlenwasserstoffalkoholresten [(- R₂ - O)_{q} - R₄ - OH] und
k.) Ketonresten [- R₆ - (C=O) - R₇], wobei die Anzahl der C-Atome für R₆ 1 bis 10 beträgt und wobei die Anzahl der C-Atome für R₇ 1 beträgt und wobei R₆ linear oder verzweigt sein kann.

Bei einem bevorzugten erfindungsgemäßen härtbaren Gemisch zur Anwendung in einem therapeutischen Verfahren bei der Herstellung eines dentalen Werkstoffs handelt es sich um eine chemisch und/oder durch Licht induziert und/oder thermisch induziert härtbare dentale Zusammensetzung.

Ein ganz besonders bevorzugtes härtbares Gemisch zur Anwendung in einem therapeutischen Verfahren bei der Herstellung eines dentalen Werkstoffs setzt sich aus den folgenden Bestandteilen zusammen:

### Bestandteil (a) - Weichmacher

Die radikalisch nicht polymerisationsfähige Verbindung der Struktur Q - [(Y)ₙ - X]ₒ als Weichmacher ist ausgewählt aus der Gruppe bestehend aus 3(4),8(9)-Bis(acyloxymethyl)tricyclo[5.2.1.0^{2,6}]decan, dem alkoxylierten 3(4),8(9)-Bis(acyloxymethyl)tricyclo[5.2.1.0^{2,6}]decan, 2,3-Bis(acyloxymethyl)bicyclo[2.2.1]heptan, dem alkoxylierten 2,3-Bis(acyloxymethyl)bicyclo[2.2.1]heptan, 1,3,5-Triacyloxytricyclo[3.3.1.1^{3,7}]decan, dem alkoxylierten Triacyloxytricyclo[3.3.1.1^{3,7}]decan, Tricyclo[5.2.1,0^{2,6}]decan-3(4),8(9)-dicarbonsäureester, dem michaelartigen Adduct aus 3(4),8(9)-Bis(aminomethyl)tricyclo[5.2.1.0^{2,6}]decan und Acrylsäureester, 3(4), 8(9)-Bis-(alkyloxymethyl)tricyclo[5.2.1.0^{2,6}]decan, dem alkoxylierten 3(4), 8(9)-Bis-(alkyloxymethyl)tricyclo[5.2.1.0^{2,6}]decan, dem Additionsprodukt aus 3(4), 8(9)-Bis(hydroxymethyl)tricyclo[5.2.1.0^{2,6}]decan mit Isocyanat, dem Additionsprodukt aus alkoxyliertem 3(4), 8(9)-Bis(hydroxymethyl)tricyclo[5.2.1.0^{2,6}]decan mit Isocyanat, dem Additionsprodukt aus 3(4), 8(9)-Bis(aminomethyl)tricyclo[5.2.1.0^{2,6}]decan mit Isocyanat beziehungsweise dem Reaktionsprodukt aus 3(4), 8(9)-Bis(isocyanatomethyl)tricyclo[5.2.1.0^{2,6}]decan mit Aminen, dem Additionsprodukt aus alkoxyliertem 3(4), 8(9)-Bis(aminomethyl)tricyclo[5.2.1.0^{2,6}]decan mit Isocyanat, dem Additionsprodukt aus 3(4), 8(9)-Bis(isocyanatomethyl)tricyclo[5.2.1.0^{2,6}]decan mit Alkohol, dem Additionsprodukt aus 3(4), 8(9)-Bis(isocyanatomethyl)tricyclo[5.2.1.0^{2,6}]decan mit alkoxyliertem Alkohol, dem Umsetzungsprodukt aus 3(4), 8(9)-Bis(carbonsäurehalogenid)tricyclo[5.2.1.0^{2,6}]decan mit einem Amin, beziehungsweise dem Umsetzungsprodukt aus 3(4), 8(9)-Bis(carbonsäure)tricyclo[5.2.1.0^{2,6}]decan mit einem Isocyanat, dem Umsetzungsprodukt aus 3(4), 8(9)-Bis(isocyanatomethyl)tricyclo[5.2.1.0^{2,6}]decan mit einem Aminoalkohol, dem Umsetzungsprodukt aus 3(4), 8(9)-Bis(isocyanatomethyl)tricyclo[5.2.1.0^{2,6}]decan mit einem Aminoalkohol und anschließender Alkoxylierung des resultierenden Alkohols, dem Umsetzungsprodukt aus 3(4), 8(9)-Bis(isocyanatomethyl)tricyclo[5.2.1.0^{2,6}]decan mit einem Aminoalkohol, anschließender Alkoxylierung des resultierenden Alkohols und Reaktion dieser Verbindung mit Carbonsäure/Carbonsäureanhydrid, dem Umsetzungsprodukt aus 3(4), 8(9)-Bis(aminomethyl)tricyclo[5.2.1.0^{2,6}]decan mit Alkylhalogeniden, dem Umsetzungsprodukt aus 3(4), 8(9)-Bis(aminomethyl)tricyclo[5.2.1.0^{2,6}]decan mit Säurehalogeniden, beziehungsweise dem Umsetzungsprodukt aus 3(4), 8(9)-Bis(isocyanatomethyl)tricyclo[5.2.1.0^{2,6}]decan mit Carbonsäuren, dem Umsetzungsprodukt von 3(4), 8(9)-Bis(hydroxymethyl)tricyclo[5.2.1.0^{2,6}]decan, beziehungsweise von alkoxyliertem 3(4), 8(9)-Bis(hydroxymethyl)tricyclo[5.2.1.0^{2,6}]decan, mit Fettsäuren sowie deren weitere Umsetzungen im Falle von Reaktionsprodukten ungesättigter Fettsäuren zu den entsprechenden Ketonen gemäß dem Wacker Prozess, dem Umsetzungsprodukt aus 3(4), 8(9)-Bis(aminomethyl)tricyclo[5.2.1.0^{2,6}]decan mit Epoxiden und deren weiteres Reaktionsprodukt mit Carbonsäuren, dem alkoxylierten 3(4), 8(9)-Bis(hydroxymethyl)tricyclo[5.2.1.0^{2,6}]decan und dem alkoxylierten 3(4), 8(9)-Bis(aminomethyl)tricyclo[5.2.1.0^{2,6}]decan,

### Bestandteil (b-1) - polymerisierbare Monomere

Die polymerisierbaren Monomere sind vorzugsweise radikalisch polymerisierbare Monomere, die bevorzugt eine, zwei oder mehr ethylenische Gruppen aufweisende Substanzen sind wie beispielsweise, ohne darauf beschränkt zu sein, die in der Dentalchemie, üblicherweise eingesetzten (Meth)acrylatmonomere.

Die (Meth)acrylatmonomere können monofunktionell sowie polyfunktionell sein. Vorzugsweise eingesetzte monofunktionelle (Meth)acrylatmonomere stellen die Ester der (Meth)acrylsäure mit Alkylgruppen von 1 bis 12 C-Atomen sowie Ester der (Meth)acrylsäure, die aromatische Gruppen mit 6 bis 12 C-Atomen enthalten, wobei die Alkylgruppen und aromatischen Gruppen, die die Ester bilden, Substituenten wie Hydroxylgruppen und Etherbindungen enthalten können, dar.

In der Patentliteratur ist eine Vielzahl weiterer Verbindungen genannt (beispielsweise auch in der DE 39 41 629 A1), die allesamt Ester der Acryl- oder Methacrylsäure sind und sich zum Einsatz in einem härtbaren Gemisch eignen.

Die radikalisch polymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von (Meth)acrylaten eingesetzt werden.

Auch können als Beispiele polyfunktioneller (Meth)acrylatmonomere Di(meth)acrylate von Alkylenglykol mit 2 bis 20 C-Atomen, Di(meth)acrylate von Oligomeren des Alkylenglykols, Polyalkylenglykoldi(meth)acrylat, Di(meth)acrylate von Bisphenol A bzw. vom Diglycidylether von Bisphenol A genannt werden.

Besonders bevorzugt sind weiterhin radikalisch härtbare Verbindungen, die auf ein zentrales polyalicyclisches Strukturelement beruhen, wie beispielsweise 3(4), 8(9)-Bis((meth)acryloyloxymethyl)tricyclo[5.2.1.0^{2,6}]decan, alkoxyliertes 3(4), 8(9)-Bis((meth)acryloyloxymethyl)tricyclo[5.2.1.0^{2,6}]decan, 2,3-Bis((meth)acryloyloxymethyl)-bicyclo[2.2.1]heptan, alkoxyliertes 2,3-Bis((meth)acryloyloxymethyl)bicyclo[2.2.1]heptan, 1,3,5-Tri(meth)acryloyloxytricyclo[3.3.1.1^{3,7}]decan, alkoxyliertes Tri(meth)acryloyloxytricyclo-[3.3.1.1^{3,7}]decan sowie (Meth)acrylsäureester von Tricyclo[5.2.1,0^{2,6}]decan-3(4),8(9)-dimethanol, alkoxyliertem Tricyclo[5.2.1.0^{2,6}]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind.

Angaben zur Herstellung dieser substituierten (Meth)acrylsäureester finden sich in den noch nicht offengelegten Patentanmeldungen EP 11 183 333, EP 11 183 328, EP 11 183 345, EP 11 183 338, EP 11 183 342 und EP 11 188 086 sowie in den in diesen Dokumenten zitierten Schriftstücken.

Bevorzugt sind ebenfalls Urethan(meth)acrylatester, Reaktionsprodukte aus 2 Mol eines (Meth)acrylats mit einer Hydroxylgruppe und einem Mol eines Diisocyanats.

Weiterhin können auch radikalisch härtbare Monomere mit ethylenischen Doppelbindungen auf Basis von Polysiloxanen, wie sie beispielsweise in der DE 199 03 177 oder in der DE 44 16 857 beschrieben sind, verwendet werden.

In einem bevorzugten erfindungsgemäßen härtbaren Gemisch enthält Bestandteil (b-1) ein oder mehrere (Meth)acrylat-Monomere gewählt aus der Gruppe bestehend aus
3(4), 8(9)-Bis((meth)acryloyloxymethyl)tricyclo[5.2.1.0^{2,6}]decan, alkoxyliertem 3(4), 8(9)-Bis((meth)acryloyloxymethyl)tricyclo[5.2.1.0^{2,6}]decan, 2,3-Bis((meth)acryloyloxymethyl)-bicyclo[2.2.1]heptan, alkoxyliertem 2,3-Bis((meth)acryloyloxymethyl)bicyclo[2.2.1]heptan, 1,3,5-Tri(meth)acryloyloxytricyclo[3.3.1.1^{3,7}]decan, alkoxyliertem Tri(meth)acryloyloxytricyclo-[3.3.1.1^{3,7}]decan, (Meth)acrylsäureester von Tricyclo[5.2.1.0^{2,6}]decan-3(4),8(9)-dimethanol, alkoxyliertem Tricyclo[5.2.1.0^{2,6}]decan-3(4),8(9)-dimethanol, Bicyclo[2.2.1]heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind, Ethylenglykoldi(meth)acrylat, 1,6-Hexandioldi(meth)acrylat (HEDMA), Triethylenglykoldi(meth)acrylat (TEDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglykoldi(meth)acrylat, 7,7,9-Trimethyl-4,13-dioxo-5,12-diazahexadecan-1,16-dioxydi(meth)acrylat (UDMA), Butandioldi(meth)acrylat, Tetraethylenglykoldi(meth)acrylat, Neopentylglykoldi(meth)acrylat, 2-Hydroxypropyl-1,3-di(meth)acrylat, 3-Hydroxypropyl-1,2-di(meth)acrylat, Pentaerythritoldi(meth)acrylat, Di(meth)acrylaten des Dihydroxymethyltricyclo[5.2.1.0^{2,6}]decans, 2-Hydroxyethyl(meth)acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, 2,2-Bis[4-[3-(meth)acryloyloxy-2-hydroxypropoxy]phenyl]propan (Bis-GMA), Trimethylolpropantri(meth)acrylat, Trimethylolethantri(meth)acrylat, Pentaerythritoltri(meth)-acrylat, Trimethylolmethantri(meth)acrylat, Pentaerythritoltetra(meth)acrylat, Ditrimethylolpropantetra(meth)acrylat, Pentaerythritolhexa(meth)acrylat, Butylenglykoldi(meth)acrylat, Propylenglykoldi(meth)acrylat, Nonandioldi(meth)acrylat, Decandioldi(meth)acrylat, Glycerolmono(meth)acrylat, Glyceroldi(meth)acrylat, Trimethylolpropanmono(meth)acrylat, Trimethylolpropandi(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)-acrylat, Allyl(meth)acrylat, Glycidyl(meth)acrylat, 2-Ethoxyethyl(meth)acrylat, Methoxypolyethylenglykol(meth)acrylat, Isobornyl(meth)acrylat, 2-(N,N-Dimethylamino)ethyl(meth)acrylat, N-Methylol(meth)acrylamid, Diaceton(meth)acrylamide, 2,2-Bis[4-(meth)acryloyloxyphenyl]-propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxyphenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxyphenyl]-2-[4-(meth)acryloyloxytriethoxyphenyl]propan, 2-[4-(Meth)acryloyloxdipropoxyphenyl]-2-[4-(meth)acryloyloxytriethoxyphenyl]propan, 2,2-Bis[4-(meth)acryloyloxyisopropoxy phenyl]propan, Hydroxypivalinsäureneopentylglykoldi(meth)acrylat, Acetoacetoxyethyl-(meth)acrylat, Polypropylenglykoldi(meth)acrylat, Glycerolalkoxylatdimethacrylat, Neopentylglykol(meth)acrylat, N,N-(1,2-Dihydroxyethylen)bisacrylamid, 2,2-Bis[4-(meth)acryloyloxypentaethoxyphenyl]propan, 2,2-Bis[4-(meth)acryloyloxypolyethoxyphenyl]-propan, Diethylenglykoldi(meth)acrylat, Dipentaerythritoltetra(meth)acrylat, Dipentaerythritolhexa(meth)acrylat, N,N-(2,2,4-Trimethylhexamethylen)bis[2-(aminocarboxy)propan-1,3-diol]-tetra(meth)acrylat, das Kondensationsprodukt von 3, (4)- (Meth)acryloxymethyl-8, (9) - hydroxymethyltricyclo- [5.2.1.0^{2,6}]decan mit Dicarbonsäuren, 2-Ethylhexyl(meth)acrylat, Tridecyl(meth)acrylat, Stearyl(meth)acrylat, Cyclohexyl(meth)acrylat, Benzyl(meth)acrylat, Methoxydiethylenglykol(meth)acrylat, Dicyclopentenyl(meth)acrylat, Phenyl(meth)acrylat, Pentaerythritolmono(meth)acrylat, Dipentaerythritolmono(meth)acrylat, Caprolacton modifiziertes Tetrahydrofurfuryl(meth)acrylat sowie lichthärtbare Monomere auf Basis von Polysiloxanen,

### Bestandteil (b-2) - Füllstoffe

Als Bestandteil (b-2) können organische und/oder anorganische Füllstoffe eingesetzt werden.

Anorganische Füllstoffe können allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Die mittlere Partikelgröße d₅₀ der erfindungsgemäß einzusetzenden Füllstoffpartikel der Füllstoffkomponente (b-2) eines erfindungsgemäßen Gemisches wird mittels Lichtstreuung (Laserbeugung) bestimmt, vorzugsweise mit einem Partikelgrößenmessgerät Beckman Coulter LS 13320.

Als anorganische Füllstoffe kommen kompakte Gläser und unterschiedliche Kieselsäuren in verschiedenen Größen und Zuständen (monodispers, polydispers) zum Einsatz.

Geeignete anorganische Bestandteile sind beispielsweise amorphe Materialien auf der Basis von Mischoxiden aus SiO₂, ZrO₂ und/oder TiO₂, mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können die dentalen härtbaren Gemische unterschiedliche Kieselsäuren, bevorzugt pyrogene Kieselsäuren, enthalten.

Daneben können verstärkend wirkende Materialien, wie Glasfasern, Polyamid- oder Kohlenstofffasern eingesetzt werden. Die dentalen härtbaren Gemische können zusätzlich feinteilige Splitter oder Perlpolymerisate enthalten, wobei die Perlpolymerisate Homo- oder Copolymere organischer härtbarer Monomerer sein können.

Bevorzugt enthalten die dentalen härtbaren Gemische nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Sulfiden, Seleniden und Telluriden von Metallen, Mischmetallen und deren Mischungen. Besonders bevorzugt sind nanoskalige Teilchen von SiO₂, TiO₂, ZrO₂, ZnO, SnO₂ und Al₂O₃ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc. Bevorzugt werden nanoskalige anorganische Feststoffteilchen, die organisch oberflächenmodifiziert sind.

In einer bevorzugten Ausgestaltung liegen die nanoskaligen Teilchen in nicht agglomerierter und/oder nicht aggregierter Form vor, beispielsweise dispergiert in einem Medium, vorzugsweise in monodisperser Form.

Um eine gute Einbindung der Nanopartikel in die Polymermatrix eines erfindungsgemäßen härtbaren Gemisches oder Erzeugnisses zu erreichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan, wodurch silanisierte Nanopartikel gebildet werden. Als Haftvermittler eignet sich besonders das Methacryloxypropyltrimethoxysilan.

In einer weiteren bevorzugten Ausgestaltung sind die nanoskaligen Teilchen nicht agglomerierte und/oder nicht aggregierte, organisch oberflächenmodifizierte Nanopartikel mit einer mittleren Partikelgröße kleiner 200 nm, vorzugsweise kleiner 100 nm, besonders bevorzugt kleiner 70 nm), die wiederum bevorzugt silanisiert sind.

### Bestandteil (b-3) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung eines erfindungsgemäßen härtbaren Gemisches bewirken können.

Das Absorptionsmaximum von Campherchinon (CC) liegt bei ca. 470 nm und somit im Bereich des blauen Lichts. Campherchinon (CC) zählt zu den Pl₂-Initiatoren und wird regelmäßig zusammen mit einem Coinitiator eingesetzt.

Vorzugsweise enthält eine erfindungsgemäßes härtbares Gemsich die Kombination eines alpha-Diketons und eines aromatischen tertiären Amins, bevorzugt ist die Kombination von Campherchinon (CC) und Ethyl-*p*-*N*,*N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in erfindungsgemäßen härtbaren Gemischen wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Gemischen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

### Bestandteil (b-3) - Initiatoren für die chemische Aushärtung

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht-und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil (b-4) - Polymerisationsinhibitoren

Die erfindungsgemäßen härtbaren Gemische enthalten vorzugsweise einen oder mehrere Inhibitoren, auch Stabilisatoren genannt. Diese werden einem härtbaren Gemisch zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität des härtbaren, insbesondere lichthärtbaren, dentalen Gemisches. Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben. Alternative Inhibitoren sind in der DE 101 19 831 A1 oder in der EP 1 563 821 A1 angegeben.

### Bestandteil (b-5) - Lösungsmittel

Geeignet sind die üblicherweise verwendeten Lösungsmittel, wie beispielsweise Kohlenwasserstoffe, Ketone und Ester wie beispielsweise Toluol, Xylol, Isooctan, Aceton, Butanon, Methylisobutylketon, Ethylacetat, Butylacetat, Tetrahydrofuran, N-Methylpyrrolidon, Dimethylacetamid und Dimethylformamid. Man kann auch Alkohole wie Ethanol, Propanole, Butanole, Pentanole, Hexanole, Cyclohexanol, Heptanole, Octanole, Nonanole, Decanole, etc. einsetzen. Ebenfalls geeignet sind cycloaliphatische oder arylaliphatische Alkohole.

Besonders bevorzugt ist, dass die Stöchiometrie der Umsetzungen bei der Synthese des Weichmachers (a) von
- 3(4), 8(9)-Bis(hydroxymethyl)tricyclo[5.2.1.0^{2,6}]decan mit Isocyanat oder von alkoxyliertem 3(4), 8(9)-Bis(hydroxymethyl)tricyclo[5.2.1.0^{2,6}]decan mit Isocyanat von 1:1 bis 1:2 reicht, so dass die Reaktionsprodukte Urethane, Allophanate sowie deren Gemische, in denen die Urethane nicht vollständig zu den Allophanaten abreagiert sind, enthalten können,
- 3(4), 8(9)-Bis(aminomethyl)tricyclo[5.2.1.0^{2,6}]decan mit Isocyanat oder von alkoxyliertem 3(4), 8(9)-Bis(aminomethyl)tricyclo[5.2.1.0^{2,6}]decan mit Isocyanat von 1:1 bis 1:2 reicht, so dass die Reaktionsprodukte Harnstoffe, Biurete sowie deren Gemische, in denen die Harnstoffe nicht vollständig zu den Biureten abreagiert sind, enthalten können,
- 3(4), 8(9)-Bis(carbonsäure)tricyclo[5.2.1.0^{2,6}]decan mit Isocyanat von 1:1 bis 1:2 reicht, so dass die Reaktionsprodukte Amide, Acylharnstoffe sowie deren Gemische, in denen die Amide nicht vollständige zu den Acylharnstoffen abreagiert sind, enthalten können,
- 3(4), 8(9)-Bis(carbonsäurehalogenid)tricyclo[5.2.1.0^{2,6}]decan mit Amin 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann,
- 3(4), 8(9)-Bis(isocyanatomethyl)tricyclo[5.2.1.0^{2,6}]decan mit Alkohol oder mit alkoxyliertem Alkohol 1:1 beträgt und ein Urethan ergibt und die zweite Umsetzungsstufe zum Allophanat mit einem Monoisocyanat in einer Stöchiometrie bis 1:1 erfolgt, so dass das Reaktionsprodukt Urethane, Allophanate sowie deren Gemische enthalten kann,
- 3(4), 8(9)-Bis(isocyanatomethyl)tricyclo[5.2.1.0^{2,6}]decan mit Amin 1:1 beträgt und einen Harnstoff ergibt und die zweite Umsetzungsstufe zum Biuret mit einem Monoisocyanat in einer Stöchiometrie bis 1:1 erfolgt, so dass das Reaktionsprodukt Harnstoffe, Biurete sowie deren Gemische enthalten kann,
- 3(4), 8(9)-Bis(isocyanatomethyl)tricyclo[5.2.1.0^{2,6}]decan mit Carbonsäure 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann,
- 3(4), 8(9)-Bis(aminomethyl)tricyclo[5.2.1.0^{2,6}]decan mit Säurehalegoniden 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann.

Bevorzugte erfindungsgemäße härtbare Gemische zur Anwendung in einem therapeutischen Verfahren bei der Herstellung eines dentalen Werkstoffs enthalten an Komponenten
(a) 0,5 bis 20 Gew.-%, vorzugsweise 2 bis 10 Gew.-%
(b-1) 5 bis 90 Gew.-%, vorzugsweise 25 bis 80 Gew.-%
(b-2) 1 bis 85 Gew.-%, vorzugsweise 20 bis 75 Gew.-%
(b-3) 0,05 bis 8 Gew.-%, vorzugsweise 0,5 bis 4 Gew.-%
(b-4) 0 bis 1 Gew.-%, vorzugsweise 0,01 bis 0,2 Gew.-%
(b-5) 0 bis 85 Gew.-%, vorzugsweise 0 bis 75 Gew.-%.

Besonders bevorzugte erfindungsgemäße härtbare Gemische zur Anwendung in einem therapeutischen Verfahren bei der Herstellung eines dentalen Werkstoffs enthalten an Komponenten
(a) 2 bis 10 Gew.-%
(b-1) 25 bis 80 Gew.-%
(b-2) 20 bis 75 Gew.-%
(b-3) 0,5 bis 4 Gew.-%
(b-4) 0,01 bis 0,2 Gew.-%
(b-5) 0 bis 75 Gew.-%.

Ein besonders bevorzugtes erfindungsgemäßes härtbares Gemisch zur Anwendung in einem therapeutischen Verfahren bei der Herstellung eines dentalen Werkstoffs enthält
(a) 2 bis 10 Gew.-% eines Weichmachers, ausgewählt aus der Gruppe bestehend aus 3(4),8(9)-Bis(acetyloxymethyl)tricyclo[5.2.1.0^{2,6}]decan, dem alkoxylierten 3(4),8(9)-Bis(acetyloxymethyl)tricyclo[5.2.1.0^{2,6}]decan, 1,3,5-Triacetyloxytricyclo[3.3.1.1^{3,7}]decan, dem alkoxylierten Triacetyloxytricyclo[3.3.1.1^{3,7}]decan, Tricyclo[5.2.1.0^{2,6}]decan-3(4),8(9)-dicarbonsäureethylester und dem michaelartigen Adduct aus 3(4),8(9)-Bis(aminomethyl)tricyclo[5.2.1.0^{2,6}]decan und Acrylsäuremethylester,
(b-1) 25 bis 80 Gew.-% eines radikalisch härtbaren Monomers, ausgewählt aus der Gruppe bestehend aus 7,7,9-Trimethyl-4,13-dioxo-5,12-diazahexadecan-1,16-dioxydi(meth)acrylat (UDMA), Triethylenglykoldi(meth)acrylat (TEDMA) und 3(4), 8(9)-Bis(methacryloyloxymethyl)tricyclo[5.2.1.0^{2,6}]decan,
(b-2) 20 bis 75 Gew.-% eines Füllstoffs, ausgewählt aus der Gruppe bestehend aus Glaskeramik, Kieselsäuren, röntgenopaken Füllstoffen, nanoskaligen Füllstoffen unter 200 nm, wobei letztere bevorzugt nicht-agglomeriert und/oder nicht-aggregiert sind sowie organischen Polymerisaten,
(b-3) 0,5 bis 4 Gew.-% eines Photoinitoators, ausgewählt aus der Gruppe bestehend aus Campherchinon/Amin und Phosphinoxid und/oder eines chemischen Initiators ausgewählt aus der Gruppe bestehend aus Peroxid/Amin und Barbitursäure/Barbitursäurederivaten in Kombination mit Schwermetallsalzen.

Ein besonders bevorzugtes erfindungsgemäßes härtbare dentale Gemisch zur Anwendung in einem therapeutischen Verfahren bei der Herstellung eines dentalen Werkstoffs stellt ein Zweikomponentensysten dar, wobei Bestandteil (b-3) ein Redoxsystem umfasst, enthaltend ein Reduktionsmittel und ein Oxidationsmittel und wobei das erfindungsgemäße dentale Kompositmaterial in zwei räumlich voneinander getrennten Komponenten in Form von Pasten vorliegt und wobei das Reduktionsmittel in der ersten Komponente enthalten ist und das Oxidationsmittel in der zweiten Komponente enthalten ist und wobei die Bestandteile (a), (b-1), (b-2), gegebenenfalls (b-4) und gegebenenfalls (b-5) in der ersten und/oder zweiten Komponente vorliegen und wobei die Pasten in Mischungsverhältnissen von erster Komponente zu zweiter Komponente im Verhältnis von 10:1 bis 1:10 vorliegen.

Im Folgenden wird die Erfindung zunächst für Verbindungen umfassend tricyclische Strukturelemente Q am Beispiel von Tricyclo[5.2.1.0^{2,6}]decan (TCD) - Derivaten im Detail erläutert. Die Umsetzungen können gleichermaßen auch mit anderen Verbindungen, umfassend ein bicyclisches, tricyclisches, tetracyclisches, pentacyclisches oder hexacyclisches Strukturelement Q, durchgeführt werden.

Die Synthese der erfindungsgemäßen Weichmacher erfolgt nach den klassischen Verfahren der organischen Chemie.

### I. Synthese der Weichmacher ausgehend vom Alkohol

Bis(hydroxymethyl)tricyclo[5.2.1.0^{2,6}]decan ist kommerziell erhältlich, beispielsweise als Dicidolgemisch der isomeren Verbindungen 3,8-Bis(hydroxymethyl)tricyclo[5.2.1.0^{2,6}]decan und 4,8-Bis(hydroxymethyl)tricyclo[5.2.1.0^{2,6}]decan sowie 3,9- Bis(hydroxymethyl)-tricyclo[5.2.1.0^{2,6}]decan und 4,9- Bis(hydroxymethyl)tricyclo[5.2.1.0^{2,6}]decan.

Die Bis(hydroxymethyl)tricyclo[5.2.1.0^{2,6}]decane können, ausgehend von Dicyclopentadien (Tricyclo[5.2.1.0^{2,6}]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0^{2,6}]decan. Je nach Synthesepfad können gezielt an unterschiedlichen Positionen substituierte Bis(hydroxymethyl)tricyclo[5.2.1.0^{2,6}]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0^{2,6}]decan herstellbar ist. Die DE 103 52 260 B3 beschreibt dagegen Verfahren zur Herstellung von 3(4), 8(9)-Bis(hydroxymethyl)tricyclo[5.2.1.0^{2,6}]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäßer Monomere einsetzbare Bis(hydroxymethyl)tricyclo[5.2.1.0^{2,6}]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die basisch katalysierte Umsetzung von Bis(hydroxymethyl)tricyclo[5.2.1.0^{2,6}]decan mit Ethylenoxid führt nach wässriger Aufarbeitung zu dem gezeigten ethoxylierten TCD-Polyether. Die alkoxylierten TCD-diole eignen sich gut als Weichmacher in dentalen Kunststoffmatrices.

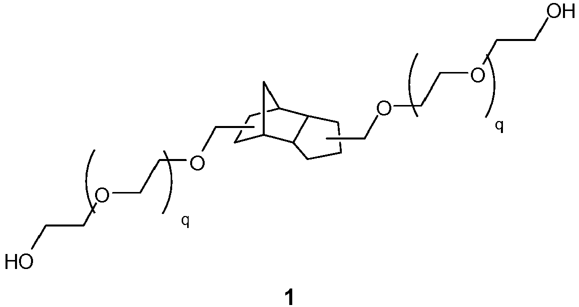

Die terminale Hydroxyfunktion kann anschließend beispielsweise mit Essigsäureanhydrid verestert werden und man erhält den ethoxylierten Ester des oben beschriebenen TCD-Polyethers.

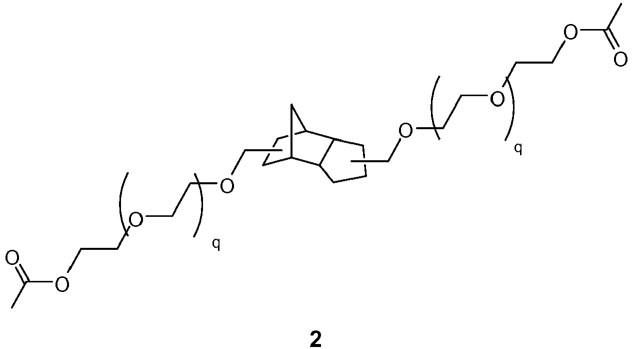

Geeignete Weichmacher können auch durch Veresterung des Bis(hydroxymethyl)tricyclo[5.2.1.0^{2,6}]decans mit Aleuritinsäure dargestellt werden. Die entsprechende Struktur ist unten dargestellt.

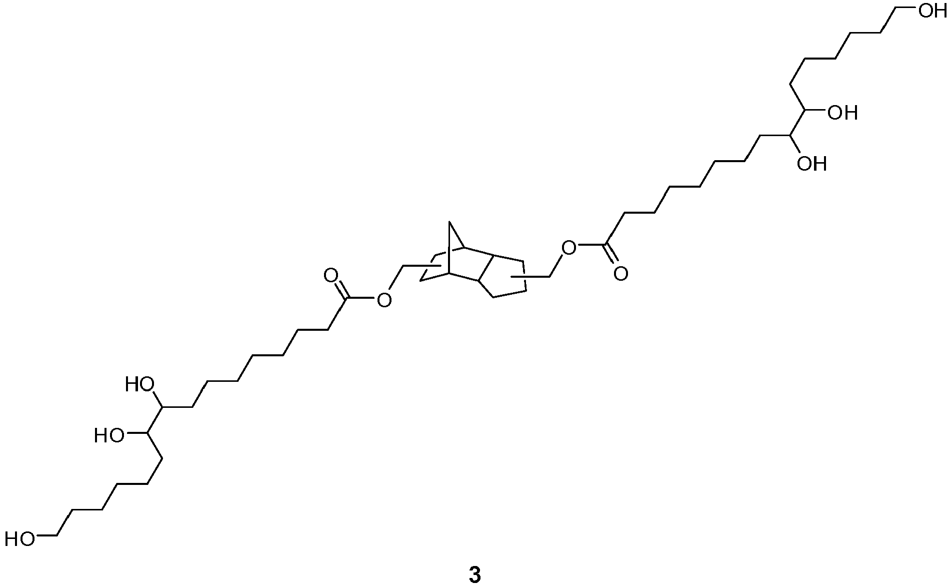

Reaktion von Bis(hydroxymethyl)tricyclo[5.2.1.0^{2,6}]decan mit zwei Äquivalenten 9-Decensäure führt zum entsprechenden Diester, der dann in Anlehnung an den sogenannten Wacker-Prozess Palladium-katalysiert zum entsprechenden Diketon oxidiert werden kann.

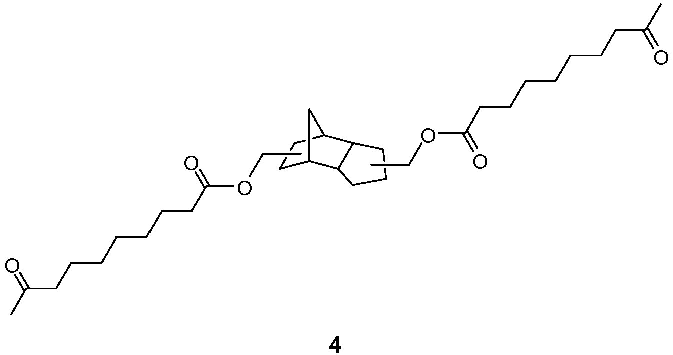

Umsetzung des Bis(hydroxymethyl)tricyclo[5.2.1.0^{2,6}]decans mit Essigsäureanhydrid in Gegenwart katalytischer Mengen konzentrierter Schwefelsäure ergibt das 3(4), 8(9)-Bis(acetyloxymethyl)tricyclo[5.2.1.0^{2,6}]decan.

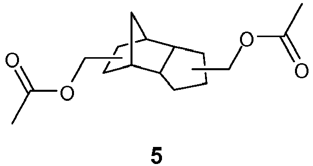

Umsetzung des Bis(hydroxymethyl)tricyclo[5.2.1.0^{2,6}]decans bzw. seiner alkoxylierten Verbindung mit einem Alkylhalogenid in Gegenwart einer starken Base liefert den 3(4), 8(9)-Bis-alkylymethylether des Tricyclo[5.2.1.0^{2,6}]decans bzw. sein alkoxyliertes Pendant.

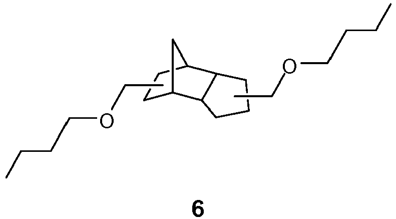

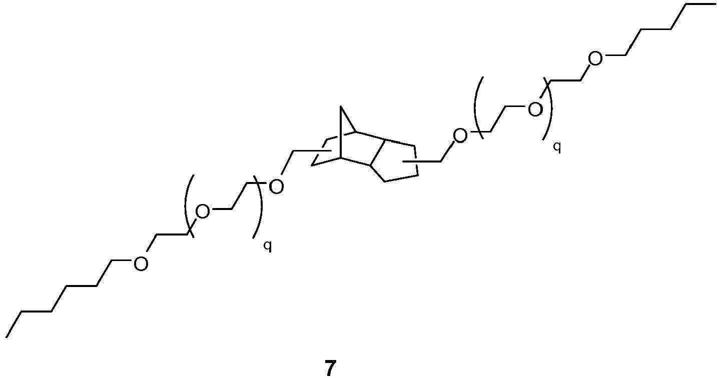

Umsetzung des Bis(hydroxymethyl)tricyclo[5.2.1.0^{2,6}]decans bzw. seiner alkoxylierten Verbindung mit einem Isocyanat, beispielsweise mit Ethyl-3-isocyanatopropionat unter den Bedingungen des Isocyanat-Alkohol Additionsverfahrens führt bei stöchiometrischer Reaktionsführung zum entsprechenden Urethan bzw. seines alkoxyliertes Pendants.

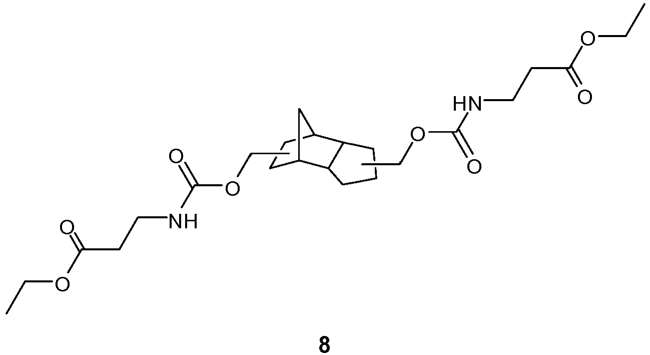

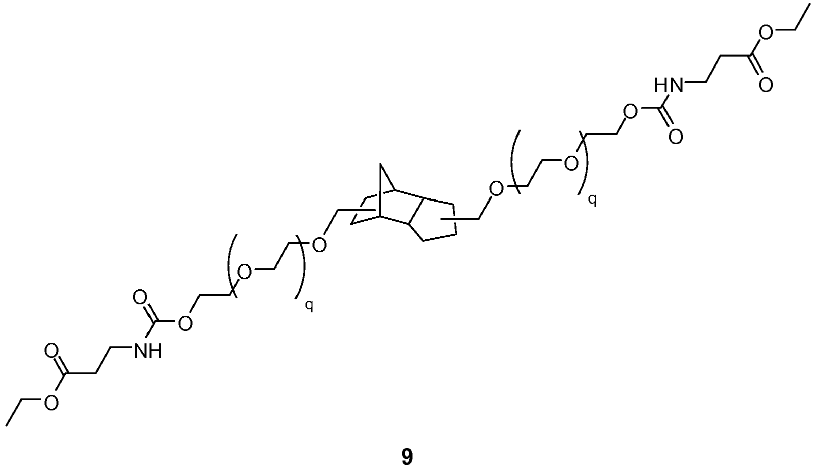

Als geeignete Komponenten in den Isocyanatadditionsverfahren können beispielsweise auch die folgenden, kommerziell erhältlichen Isocyanate eingesetzt werden: Ethyl-2-isocyanatoacetat, Methyl-6-isocyanatohexanoat, Ethyl-2-isocyanato-3-methylbutyrat, 2-Isocyanatoglutarsäurediethylester, Butyl-4-isocyanatobenzoat, Ethyl-2-isocyanatobenzoat, 2-Isocyanatoethyl-propionat, 1-Isocyanato-2-methoxyethan, 1-Isocyanato-3-methoxypropan, 1-Isocyanato-3-isopropoxypropan, 2-(Isocyanatomethyl)tetrahydrofuran, (2-Isocyanatoethyl)-dimethylamin, 3-Isocyanatopropanoylchlorid, 3-(Isocyanatomethyl)dihydropyrimidin-2,4(1H,3H)-dion.

Die stöchiometrischen Umsetzungsprodukte des Bis(hydroxymethyl)tricyclo[5.2.1.0^{2,6}]decans bzw. seiner alkoxylierten Verbindung mit Ethyl-3-isocyanatopropionat unter den Bedingungen des Isocyanat-Alkohol Additionsverfahrens führen zu Urethanen, die noch reaktive Wasserstoffatome in der Urethanfunktion -O-C(=O)-NH- aufweisen. Diese Wasserstoffatome können in einer zweiten Umsetzungsstufe mit Isocyanaten weiter abreagiert werden. Formal betrachtet verlaufen diese Additionsreaktionen so, dass ein Protonenübergang von der H-aktiven Verbindung an den Stickstoff der Isocyanatgruppe erfolgt und sich dann das resultierende negative Ion des Addenden mit dem positiv polarisierten Carbonylkohlenstoff des Isocyanats verbindet.

Wird also das Bis(hydroxymethyl)tricyclo[5.2.1.0^{2,6}]decan bzw. seine alkoxylierte Verbindung beispielsweise mit Ethyl-3-isocyanatopropionat doppelt stöchiometrisch umgesetzt und die Temperaturführung angepasst, da die Weiterreaktion der zweiten Umsetzungsstufe in der Regel bei höheren Temperaturen erfolgt, wird das entsprechende Allophanat gebildet.

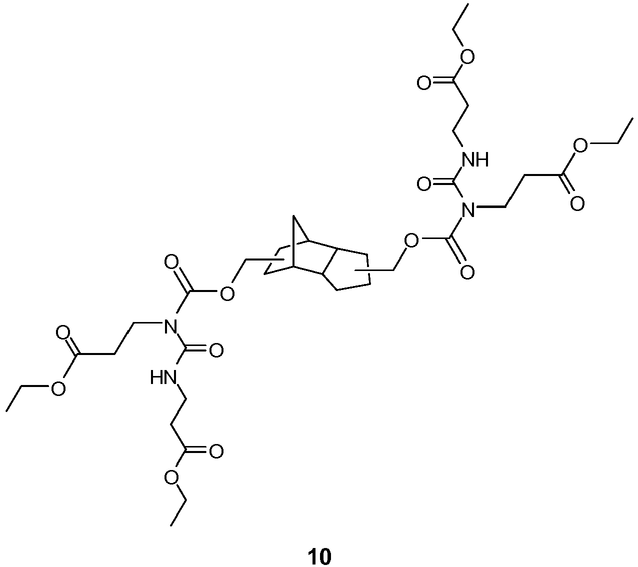

Besonders bevorzugte Weichmacher sind solche, die aus Ansätzen resultieren, deren Stöchiometrie zwischen der Umsetzung des Alkohols mit dem Isocyanat zu einem Urethan und einem Allophanat liegt.

Betrachtet man die Struktur des Allophanats, so findet man auch dort noch weitere reaktive H-Atome, die zu Umsetzungen mit Isocyanaten befähigt sind. Produkte dieser dritten Umsetzungsstufen der Isocyanat-Alkohol Reaktionen sind im Rahmen dieser Erfindung nicht bevorzugt.

Einfache Umsetzung von Bis(hydroxymethyl)tricyclo[5.2.1.0^{2,6}]decan beispielsweise mit Alkylisocyanat führt zum entsprechenden Urethan, das durch weitere Reaktion beispielsweise mit dem Alkylisocyanat das entsprechende Allophanat liefert.

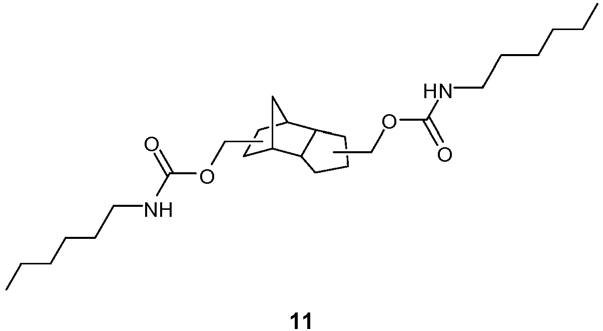

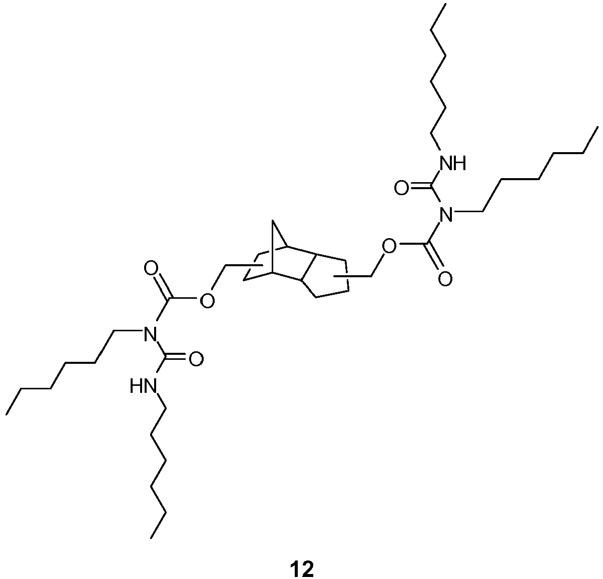

Auch hier gilt wiederum: Solche Gemische sind als Weichmacher besonders bevorzugt, die aus Ansätzen resultieren, deren Stöchiometrie zwischen der Umsetzung des Alkohols mit dem Isocyanat zu einem Urethan und einem Allophanat liegt, beziehungsweise bei denen das Urethan in einer zweiten Umsetzungsstufe nicht vollständig zum Allophanat abreagiert wird.

### II. Synthese der Weichmacher ausgehend von Carbonsäurederivaten

Das 3(4), 8(9)-Bis(carbonsäure)tricyclo[5.2.1.0^{2,6}]decan ist durch einfache Oxidation des kommerziell erhältlichen 3(4), 8(9)-Bis(formyl)tricyclo[5.2.1.0^{2,6}]decans herstellbar. Umsetzung der Dicarbonsäure mit Ethanol in Gegenwart katalytischer Mengen konzentrierter Schwefelsäure führt zum Tricyclo[5.2.1.0^{2,6}]decan-3(4),8(9)-dicarbonsäureethylester.

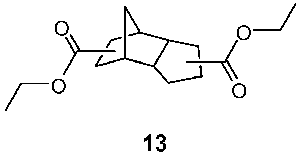

Umsetzung des 3(4), 8(9)-Bis(carbonsäure)tricyclo[5.2.1.0^{2,6}]decans beispielsweise mit Ethyl-3-isocyanatopropionat unter den speziellen Bedingungen dieses Additionsverfahrens ergibt das entsprechende Amid.

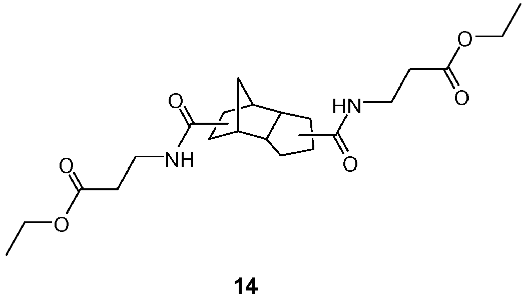

Wird das Amid, das aus der stöchiometrischen Reaktion des 3(4), 8(9)-Bis(carbonsäure)tricyclo[5.2.1.0^{2,6}]decans mit Ethyl-3-isocyanatopropionat hervorgeht, in einer zweiten Umsetzungsstufe, ebenfalls stöchiometrisch, weiter beispielsweise mit dem Isocyanat abreagiert, so gelangt man zum entsprechenden Acylharnstoff.

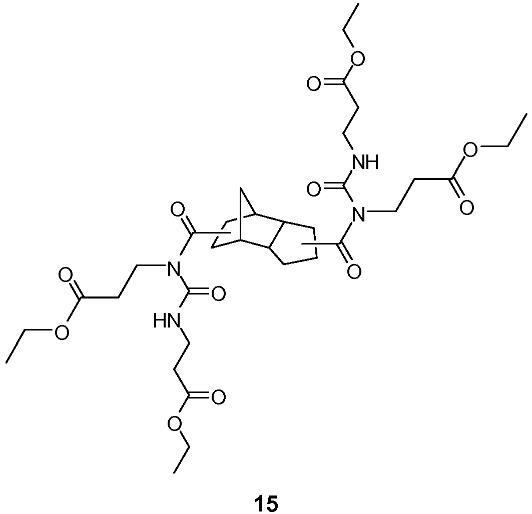

Auch hier gilt wiederum, dass solche Gemische als Weichmacher besonders bevorzugt sind, die aus Ansätzen resultieren, deren Stöchiometrie zwischen der Umsetzung einer Carbonsäure mit einem Isocyanat zu einem Amid und zu einem Acylharnstoff liegt.

Umsetzung des 3(4), 8(9)-Bis(carbonsäure)tricyclo[5.2.1.0^{2,6}]decans mit Thionylchlorid zum Carbonsäurechlorid und weitere Reaktion des Carbonsäurechlorids mit einem Amin ergibt unter Abspaltung von HCl das entsprechende Amid.

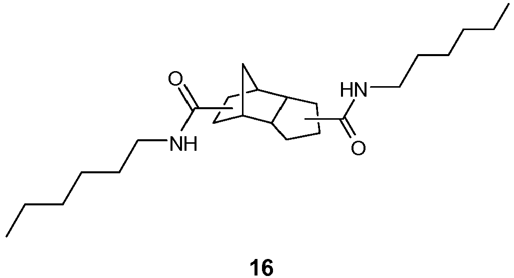

Alternativ ist das Amid durch Reaktion des 3(4), 8(9)-Bis(carbonsäure)tricyclo[5.2.1.0^{2,6}]decans mit einem Alkylisocyanat erhältlich.

Weitere Umsetzung des Amids beispielsweise mit einem Alkylisocyanat führt zum entsprechenden Acylharnstoff.

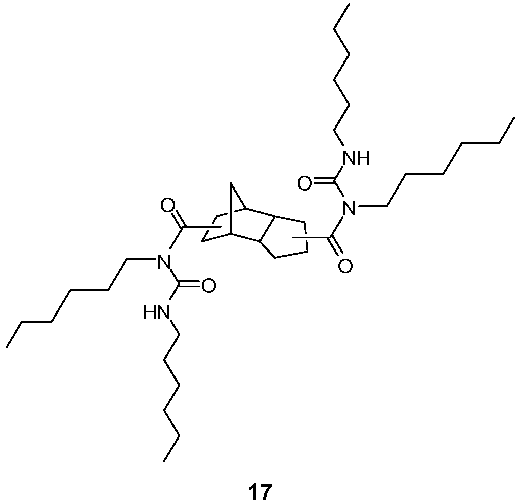

### III. Synthese der Weichmacher ausgehend von Aminen

Das 3(4), 8(9)-Bis(aminomethyl)tricyclo[5.2.1.0^{2,6}]decan ist an sich bekannt oder kann beispielsweise durch Umsetzung der entsprechenden Tosylate mit Ammoniak hergestellt werden. Wird das Amin langsam mit Acrylsäuremethylester umgesetzt, so gelangt man, je nach Reaktionsstöchiometrie dieser vinylogen Addition zu den entsprechenden michaelähnlichen Addukten.

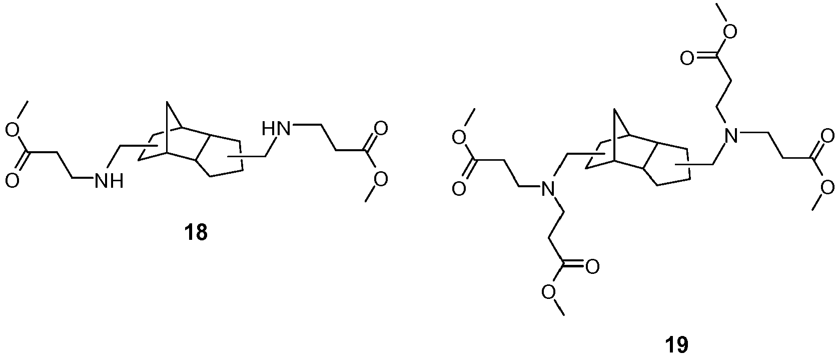

Umsetzung des 3(4), 8(9)-Bis(aminomethyl)tricyclo[5.2.1.0^{2,6}]decans beispielsweise mit Ethyl-3-isocyanatopropionat unter den Bedingungen des Isocyanat Additionsverfahrens führt zum entsprechenden Harnstoff.

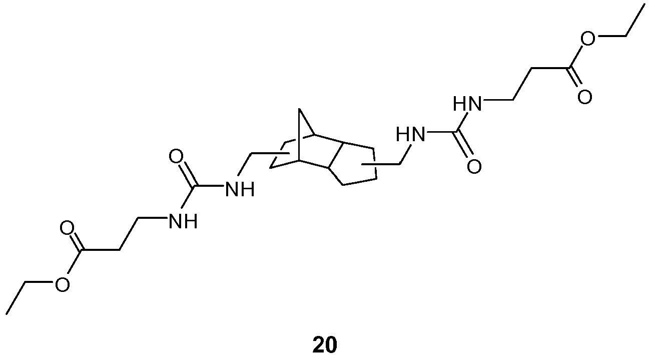

Wird das 3(4), 8(9)-Bis(aminomethyl)tricyclo[5.2.1.0^{2,6}]decan mit Ethyl-3-isocyanatopropionat doppelt stöchiometrisch umgesetzt, erhält man die entsprechenden Biurete.

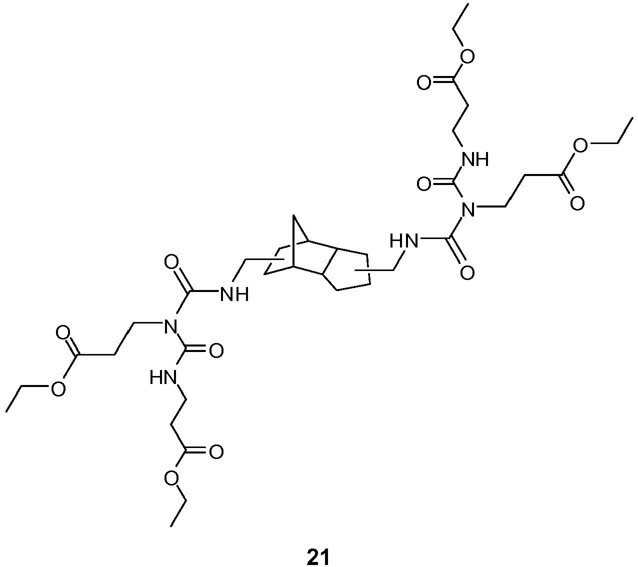

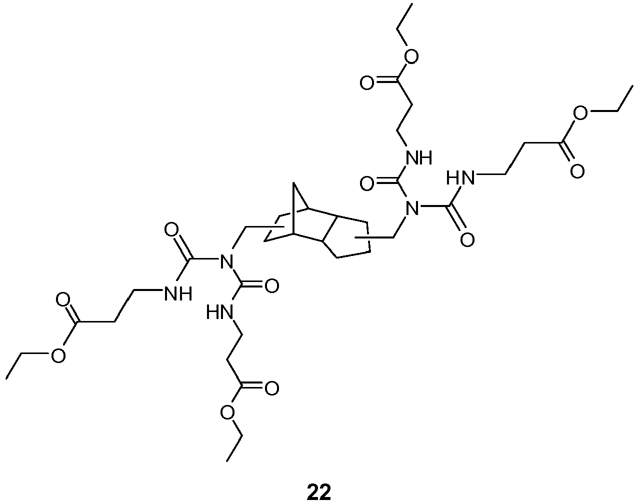

Auch hier gilt, dass solche Gemische als Weichmacher besonders bevorzugt sind, die aus Ansätzen resultieren, deren Stöchiometrie zwischen der Umsetzung eines Amins mit einem Isocyanat zu einem Harnstoff und zu einem Biuret liegt.

Zur weiteren Illustration geeigneter Weichmacher kommen beispielsweise die Umsetzungsprodukte des 3(4), 8(9)-Bis(aminomethyl)tricyclo[5.2.1.0^{2,6}]decans mit Alkylhalogeniden unter basischen Bedingungen infrage. Je nach Stöchiometrie des Ansatzes gelangt man so zu den entsprechenden Aminen.

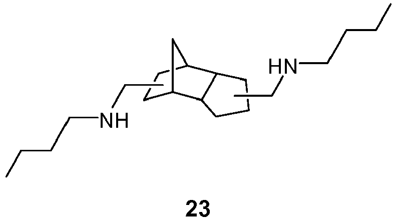

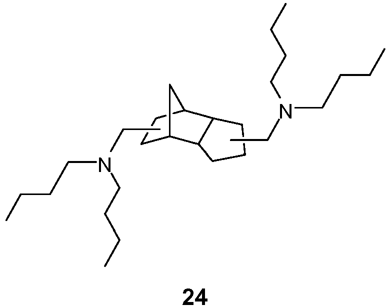

Wird 3(4), 8(9)-Bis(aminomethyl)tricyclo[5.2.1.0^{2,6}]decan mit Säurehalogeniden umgesetzt so gelangt man zu den entsprechenden Amiden. Alternativ sind diese erfindungsgemäßen Weichmacher erhältlich durch Reaktion des 3(4), 8(9)-Bis(isocyanatomethyl)tricyclo[5.2.1.0^{2,6}]decans mit den entsprechenden Carbonsäuren.

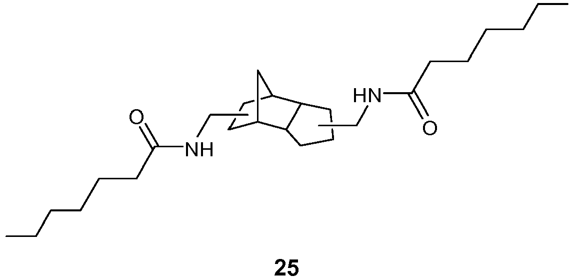

Wird obiges Amid beispielsweise mit einem Alkylisocyanat umgesetzt, so ergibt sich der entsprechende Acylharnstoff.

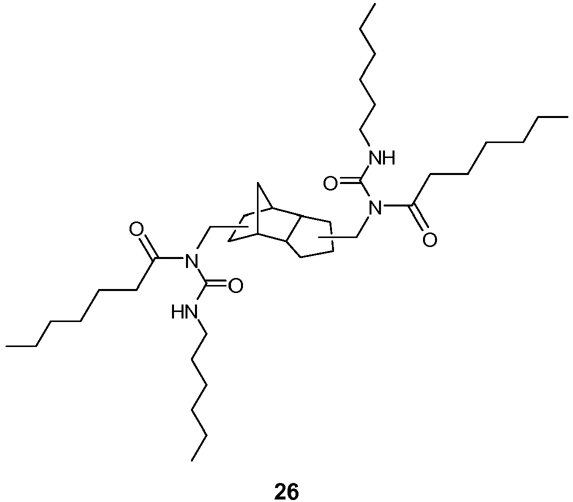

Reaktion des 3(4), 8(9)-Bis(aminomethyl)tricyclo[5.2.1.0^{2,6}]decans mit einem Alkylisocyanat führt, wie auch die Reaktion des 3(4), 8(9)-Bis(isocyanatomethyl)tricyclo[5.2.1.0^{2,6}]decans mit dem entsprechenden Amin, zu den Harnstoffen, die dann weiter beispielsweise mit Alkylisocyanat umsetzbar sind.

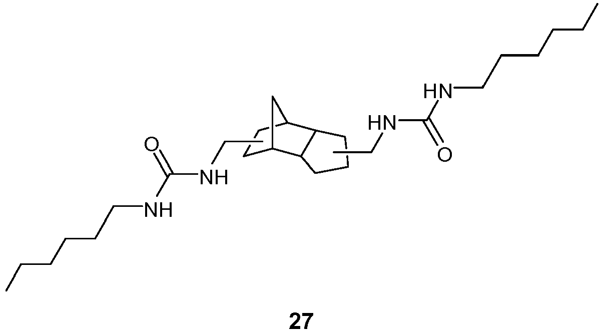

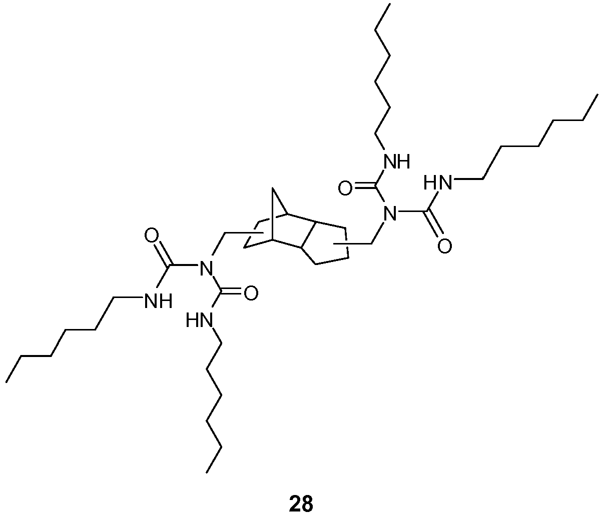

Reaktion des 3(4), 8(9)-Bis(aminomethyl)tricyclo[5.2.1.0^{2,6}]decans beispielsweise mit Ethylenoxid ergibt den einfach und doppelt substituierten alkoxylierten Aminoalkohol, der anschließend beispielsweise durch Umsetzung mit Essigsäureanhydrid verestert werden kann.

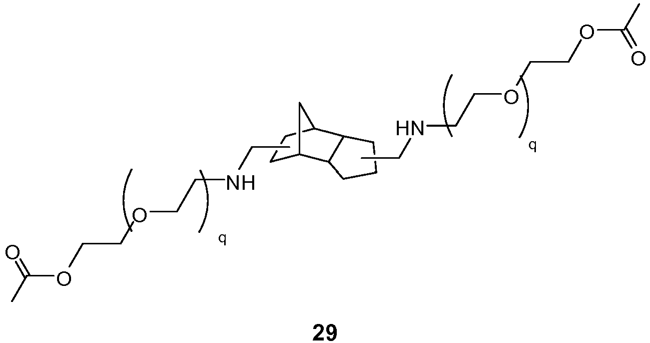

### IV. Synthese der Weichmacher ausgehend von Isocyanaten

Das 3(4), 8(9)-Bis(isocyanatomethyl)tricyclo[5.2.1.0^{2,6}]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Umsetzung dieser Verbindung unter den Bedingungen des Isocyanats-Alkohol Additionsverfahrens beispielsweise mit 3-Hydroxypropansäuremethylester führt zum entsprechenden Urethan.

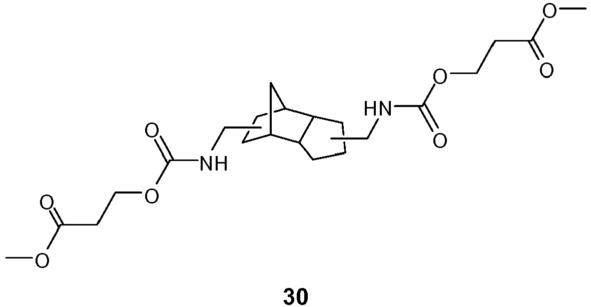

Wird anstelle des 3-Hydroxypropansäuremethylesters eine alkoxylierte Variante eingesetzt, so erhält man das entsprechend alkoxylierte Urethan.

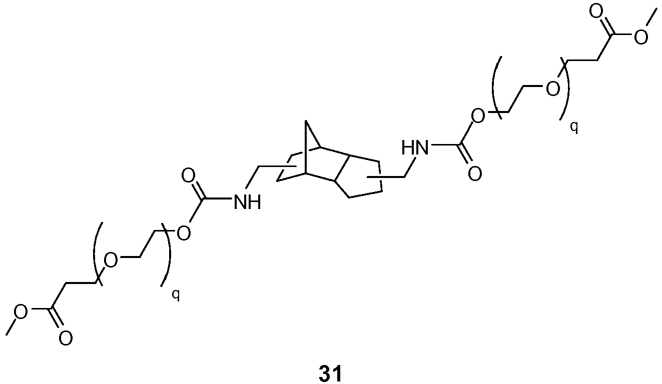

Wird beispielsweise ein Aminoalkohol mit dem 3(4), 8(9)-Bis(isocyanatomethyl)tricyclo[5.2.1.0^{2,6}]decan umgesetzt, der resultierende Alkohol alkoxyliert und diese Verbindung dann mit Essigsäureanhydrid umgesetzt, bekommt man die entsprechend alkoxylierte Harnstoffverbindung.

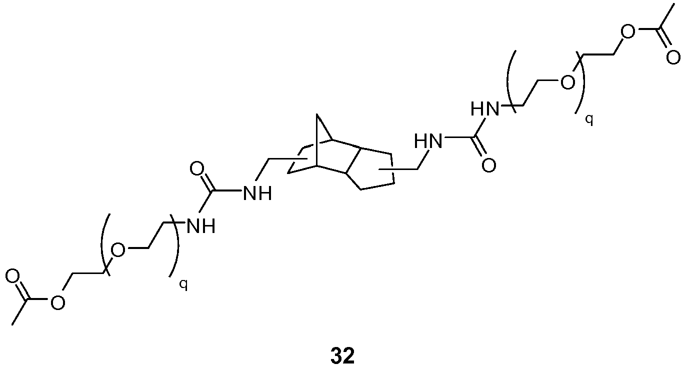

Umsetzung des 3(4), 8(9)-Bis(isocyanatomethyl)tricyclo[5.2.1.0^{2,6}]decans beispielsweise mit dem kommerziell erhältlichen Bernsteinsäuremonomethylester unter den speziellen Bedingungen der Additionsreaktion einer Carbonsäure mit einem Isocyanat ergibt das entsprechende Amid.

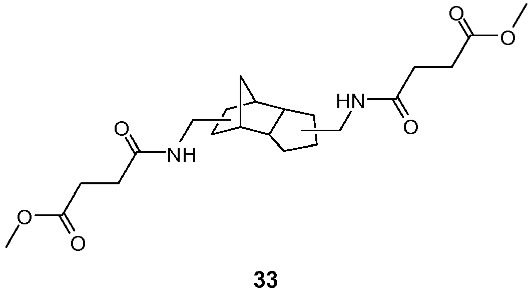

Alternativ kann dann auch das Urethan, das aus der stöchiometrischen Reaktion des 3(4), 8(9)-Bis(isocyanatomethyl)tricyclo[5.2.1.0^{2,6}]decans mit 3-Hydroxypropansäuremethylester bzw. seines alkoxylierten Vertreters erhältlich ist, mit weiterem Isocyanat, beispielsweise mit Ethyl-3-isocyanatopropionat, zu den entsprechenden Allophanaten abreagiert werden.

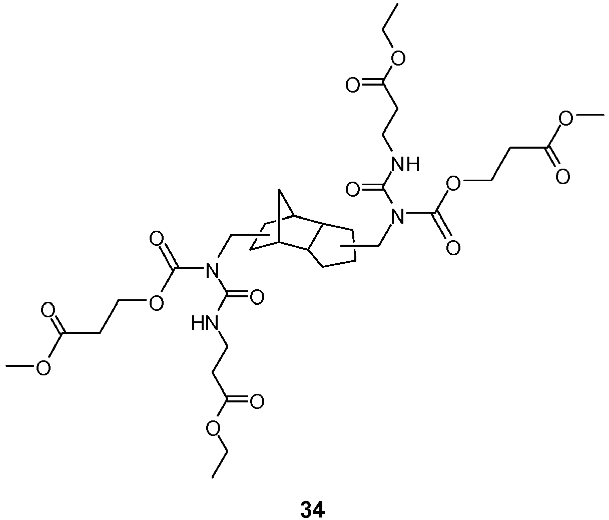

Auch hier sind solche Gemische als Weichmacher besonders bevorzugt, die aus Ansätzen resultieren, deren Stöchiometrie zwischen der Umsetzung des Alkohols mit dem Isocyanat zu einem Urethan und einem Allophanat liegt, beziehungsweise bei denen das Urethan in einer zweiten Umsetzungsstufe nicht vollständig zum Allophanat abreagiert wird.

Analog kann auch das Amid, das aus der Umsetzung des 3(4), 8(9)-Bis(isocyanatomethyl)tricyclo[5.2.1.0^{2,6}]decans mit Bernsteinsäuremonomethylester resultiert, in einer zweiten Umsetzungsstufe mit einem Isocyanat, beispielsweise dem Ethyl-3-isocyanatopropionat, weiter zum entsprechenden Acylharnstoff abreagiert werden.

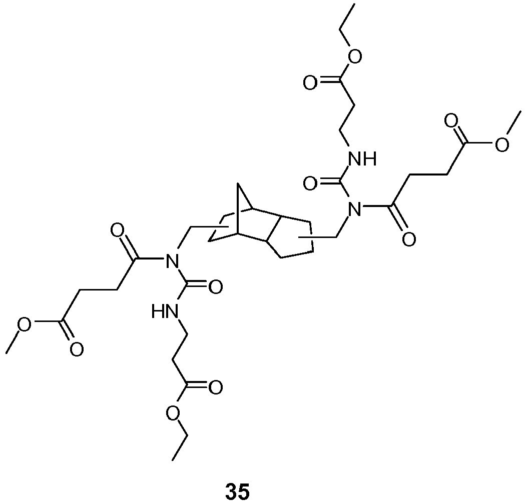

Auch hier gilt wiederum, dass solche Gemische als Weichmacher besonders bevorzugt sind, die aus Ansätzen resultieren, deren Stöchiometrie zwischen der Umsetzung einer Carbonsäure mit einem Isocyanat zu einem Amid und zu einem Acylharnstoff liegt.

### Beispiele

### 3(4),8(9)-Bis(acetyloxymethyl)tricyclo[5.2.1.0^{2,6}]decan (Weichmacher "TCD")

98.15 g (0.5 mol) Tricyclo[5.2.1,0^{2,6}]decan-3(4),8(9)-dimethanol und 102.09 g (1.0 mol) Essigsäureanhydrid werden mit 10 Tropfen konzentrierter Schwefelsäure versetzt und es wird solange am Rotationsverdampfer (ohne Vakuum) gerührt, bis eine homogene Lösung entstanden ist. Anschließend wird für 2 h unter Rückfluss auf 100°C erhitzt. Nach dem Abkühlen wird die Reaktionsmischung auf 300 ml Eiswasser gegeben und es wird mit Dichlormethan extrahiert. Die vereinigten organischen Phasen werden mit NatriumcarbonatLösung entsäuert und mit Wasser gewaschen. Es wird über Magnesiumsulfat getrocknet, filtriert und das Lösungsmittel im Vakuum entfernt. Es werden 136.09 g (0.49 mol; 97.1%) eines blassgelben Öls erhalten.
C₁₆H₂₄O₄ (280.36 g/mol)
Viskosität: 136.8 cSt
*n*^{D}₂₀: 1.487
IR (Film; cm⁻¹): 2946 (m, v_{C-H}); 1735 (vs, v_{C=O}); 1446 (w, δ_{C-H}); 1367 (m, δ_{C-H, Acetyl}); 1229 (vs, v_{C-O}); 1029 (s)

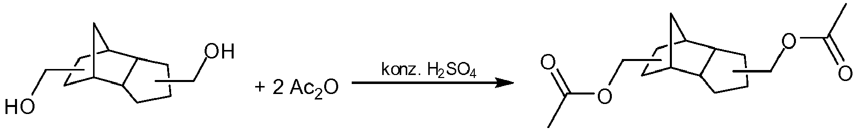

### 2,3-Bis(acetyloxymethyl)bicyclo[2.2.1]heptan (Weichmacher "Norbornan")

60.10 g (0.5 mol) Bicyclo[2.2.1]heptan-2,3-dimethanol und 102.09 g (1.0 mol) Essigsäureanhydrid werden mit 10 Tropfen konzentrierter Schwefelsäure versetzt und es wird solange am Rotationsverdampfer (ohne Vakuum) gerührt, bis eine homogene Lösung entstanden ist. Anschließend wird für 2 h unter Rückfluss auf 100°C erhitzt. Nach dem Abkühlen wird die Reaktionsmischung auf 300 ml Eiswasser gegeben und es wird mit Dichlormethan extrahiert. Die vereinigten organischen Phasen werden mit NatriumcarbonatLösung entsäuert und mit Wasser gewaschen. Es wird über Magnesiumsulfat getrocknet, filtriert und das Lösungsmittel im Vakuum entfernt. Es werden 115.84 g (0.48 mol; 96.4%) eines blassgelben Öls erhalten.
C₁₃H₂₀O₄ (240.30 g/mol)
*n*^{D}₂₀: 1.487
IR (Film; cm⁻¹): 2945 (m, v_{C-H}); 1734 (vs, v_{C=O}); 1456 (w, δ_{C-H}); 1366 (m, δ_{C-H, Acetyl}); 1228 (vs, v_{C-O}); 1029 (s)

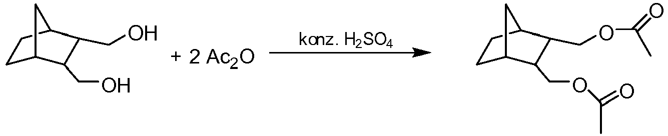

### 1,3,5-Triacetyloxytricyclo[3.3.1.1^{3,7}]decan (Weichmacher "Adamantan")

92.12 g (0.5 mol) 1,3,5-Adamantantriol werden in 153.14 g (1.5 mol) Essigsäureanhydrid suspendiert. Anschließend werden 10 Tropfen konzentrierte Schwefelsäure zugegeben und es wird solange am Rotationsverdampfer (ohne Vakuum) gerührt, bis eine homogene Lösung entstanden ist. Anschließend wird für 2 h unter Rückfluss auf 100°C erhitzt. Nach dem Abkühlen wird die Reaktionsmischung auf 300 ml Eiswasser gegeben und es wird mit Dichlormethan extrahiert. Die vereinigten organischen Phasen werden mit NatriumcarbonatLösung entsäuert und mit Wasser gewaschen. Es wird über Magnesiumsulfat getrocknet, filtriert und das Lösungsmittel im Vakuum entfernt. Es werden 150.50 g (0.48 mol; 97.0%) eines farblosen Öls erhalten.
C₁₆H₂₂O₆ (310.35 g/mol)
*n*^{D}₂₀: 1.485
IR (Film; cm⁻¹): 2935 (m, v_{C-H}); 1730 (vs, v_{C=O}); 1463 (w, δ_{C-H}); 1434 (w, δ_{C-H}); 1366 (m, δ_{C-H, Acetyl}); 1220 (vs, v_{C-O}); 1018 (s)

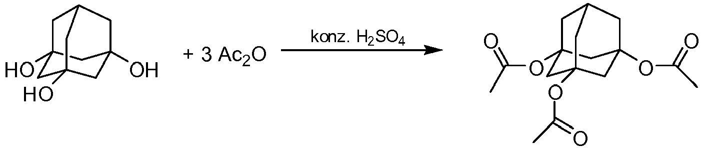

### Tricyclo[5.2.1.0^{2,6}]decan-3(4),8(9)-dicarbonsäureethylester (Weichmacher "Ester")

112.13 g (0.5 mol) Tricyclo[5.2.1.0^{2,6}]decan-3(4),8(9)-dicarbonsäure und 80.62 g (1.75 mol) Ethanol werden mit 5 g konz. Schwefelsäure in 100 ml Chloroform unter Rückfluss am Wasserabscheider erhitzt, bis sich kein Wasser mehr abscheidet. Nach dem Abkühlen wird die Reaktionslösung mit Wasser, Hydrogencarbonatlösung und Wasser gewaschen. Es wird über Magnesiumsulfat getrocknet, filtriert und das Lösungsmittel im Vakuum entfernt. Es werden 126.21 g (0.45 mol; 90.0%) eines farblosen Öls erhalten.
C₁₆H₂₄O₄ (280.36 g/mol)
*n*^{D}₂₀: 1.487
IR (Film; cm⁻¹): 2940 (m, v_{C-H}); 1740 (vs, v_{C=O}); 1452 (w, δ_{C-H}); 1377 (m, δ_{C-H}); 1221 (vs, v_{C-O}); 1025 (s)

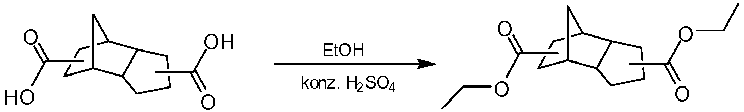

### Michael-Addukt aus 3(4),8(9)-Bis(aminomethyl)tricyclo[5.2.1.0^{2,6}]decan und Acrylsäuremethylester (Weichmacher "Michael")

97.16 g (0.5 mol) 3(4),8(9)-Bis(aminomethyl)tricyclo[5.2.1.0^{2,6}]decan werden langsam mit 172.18 g (2.0 mol) Acrylsäuremethylester versetzt und für 2 h bei Raumtemperatur gerührt. Die Reaktionskontrolle erfolgt durch IR (Verschwinden der Acrylat-Bande bei 1630 cm⁻¹).
C₂₈H₄₆O₈N₂ (538.68 g/mol)
*n*^{D}₂₀:1.497
IR (Film; cm⁻¹): 2935 (s, v_{C-H}); 1733 (vs, v_{C=O}); 1439 (m, δ_{C-H}); 1359 (m, δ_{C-H}); 1171 (vs, v_{C-O}); 1016 (m)

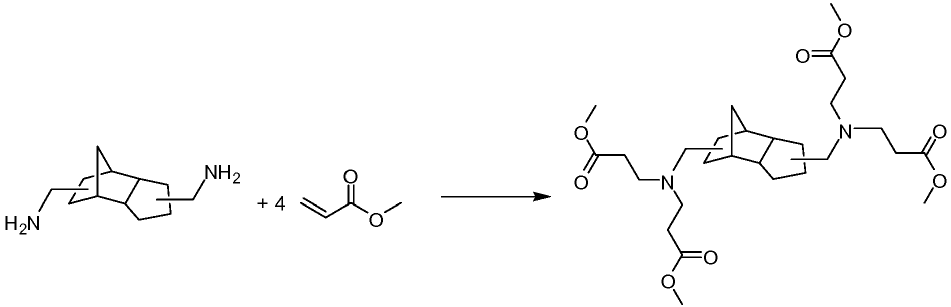

### Umsetzungsprodukt aus Tricyclo[5.2.1.0^{2,6}]decan-3(4),8(9)-dimethanol und Ethyl-3-isocyanatopropionat (Weichmacher "Urethan")

Es werden 143.14 g (1.0 mol) Ethyl-3-isocyanatopropionat und 2.99 g (0.015 mol) Phenothiazin in 250 ml Toluol gelöst und auf 70°C erwärmt. Dann werden 3.51 g (0.01 mol) Zinkoctat zugegeben. Anschließend werden 98.15 g (0.5 mol) Tricyclo[5.2.1.0^{2,6}]decan-3(4),8(9)-dimethanol in 150 ml Tetrahydrofuran langsam zugetropft. Danach werden 6.76 g (0.05 mol) Dimethylbenzylamin zugegeben und es wird über Nacht bei 70°C gerührt. Die Reaktionskontrolle erfolgt durch IR (Verschwinden der Isocyanat-Bande bei 2270 cm⁻¹). Das Lösungsmittel wird im Vakuum entfernt und es werden 236.65 g (0.49 mol, 97.2%) eines farblosen Öls erhalten.
C₂₄H₄₂N₂O₈ (486.60 g/mol)
*n*^{D}₂₀:1.498
IR (Film; cm⁻¹): 3356 (m, v_{N-H}), 2945 (s, v_{C-H}); 1712 (vs, v_{C=O}), 1695 (vs, v_{C=O}), 1522 (m, δ_{N-H}), 1452 (m, δ_{C-H}); 1374 (m, δ_{C-H}); 1243 (s, v_{C-O}), 1182 (vs, v_{C-O}); 1017 (s)

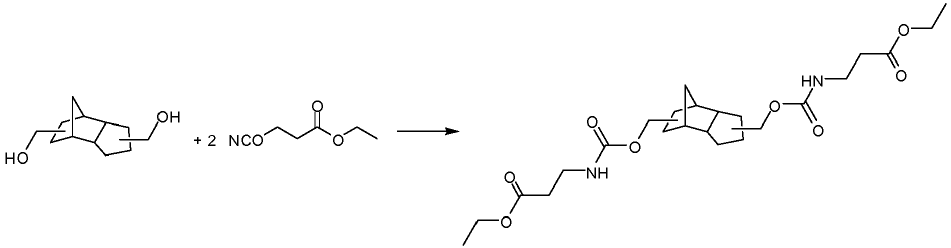

### Umsetzungsprodukt aus 3(4),8(9)-Bis(aminomethyl)tricyclo[5.2.1.0^{2,6}]decan und Ethyl-3-isocyanatopropionat (Weichmacher "Harnstoff")

Es werden 143.14 g (1.0 mol) Ethyl-3-isocyanatopropionat und 2.99 g (0.015 mol) Phenothiazin in 250 ml Toluol gelöst und auf 70°C erwärmt. Dann werden 3.51 g (0.01 mol) Zinkoctat zugegeben. Anschließend werden 97.16 g (0.5 mol) 3(4),8(9)-Bis(aminomethyl)tricyclo[5.2.1.0^{2,6}]decan in 150 ml Toluol langsam zugetropft. Danach werden 6.76 g (0.05 mol) Dimethylbenzylamin zugegeben und es wird über Nacht bei 70°C gerührt. Die Reaktionskontrolle erfolgt durch IR (Verschwinden der Isocyanat-Bande bei 2270 cm⁻¹). Das Lösungsmittel wird im Vakuum entfernt und es werden 241.33 g (0.48 mol, 95.2%) eines farblosen Öls erhalten.
C₂₄H₄₄N₄O₆ (506.64 g/mol)
*n*^{D}₂₀:1.498
IR (Film; cm⁻¹): 3333 (m, v_{N-H}), 2938 (s, v_{C-H}); 1732 (s, V_{C=O}, Ester); 1631 (s, v_{C=O}, Harnstoff), 1564 (vs, δ_{N-H}), 1460 (w, δ_{C-H}); 1374 (w, δ_{C-H}); 1247 (s, v_{C-O}), 1179 (vs, v_{C-O}); 1030 (m)

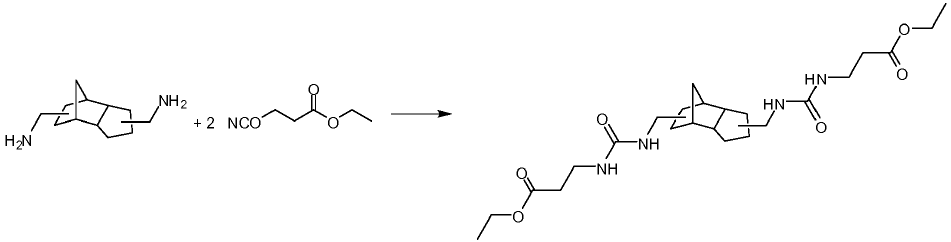

### Abkürzungen:

UDMA: Urethandimethacrylat
BisEMA: Ethoxyliertes Bisphenol-A-dimethacrylat
TCD-DMA: Bis(methacryloyloxymethyl)tricyclo[5.2.1.0^{2,6}]decan
BisGMA: Bisphenol-A-glycidyldimethacrylat
TEDMA: Triethylenglycoldimethacrylat
BAP: 2,2-Bis-4-(2-acetyloxyethoxyphenyl)-propan
GK: silanisierte Glaskeramik (GM27884, Schott)
DEPT: N,N-Dihydroxyethyl-p-toluidin
BHT: 3,5-Di-*tert*.-butyl-4-hydroxytoluol
BPO: Dibenzoylperoxid

### Provisorisches K&B-Material (10:1)

**Tabelle 1: Basis-Pasten (provisorisches K&B-Material)**

| | Basis 1 | Basis 2 |
|---|---|---|
| UDMA | 19.00 | 28.40 |
| BisEMA | 38.00 | 0.00 |
| TCD-DMA | 0.00 | 28.40 |
| GK | 35.95 | 35.95 |
| Aerosil A300 | 1.50 | 1.50 |
| Aerosil R812 | 3.00 | 3.00 |
| DEPT | 2.50 | 2.70 |
| BHT | 0.05 | 0.05 |

**Tabelle 2: Katalysator-Pasten (provisorisches K&B-Material)**

| | Kat 1 | Kat 2 | Kat 3 | Kat 4 |
|---|---|---|---|---|
| BAP | 53.50 | 0.00 | 0.00 | 0.00 |
| Weichmacher "TCD" | 0.00 | 53.50 | 0.00 | 0.00 |
| Weichmacher "Norbornan" | 0.00 | 0.00 | 53.50 | 0.00 |
| Weichmacher "Adamantan" | 0.00 | 0.00 | 0.00 | 53.50 |
| GK | 36.60 | 36.60 | 36.60 | 36.60 |
| Aerosil A300 | 1.50 | 1.50 | 1.50 | 1.50 |
| Aerosil R812 | 3.00 | 3.00 | 3.00 | 3.00 |
| BPO | 5.40 | 5.40 | 5.40 | 5.40 |

| | Kat 5 | Kat 6 | Kat 7 | Kat 8 |
|---|---|---|---|---|
| Weichmacher "Ester" | 53.50 | 0.00 | 0.00 | 0.00 |
| Weichmacher "Michael" | 0.00 | 53.50 | 0.00 | 0.00 |
| Weichmacher "Urethan" | 0.00 | 0.00 | 53.50 | 0.00 |
| Weichmacher "Harnstoff" | 0.00 | 0.00 | 0.00 | 53.50 |
| GK | 36.60 | 36.60 | 36.60 | 36.60 |
| Aerosil A300 | 1.50 | 1.50 | 1.50 | 1.50 |
| Aerosil R812 | 3.00 | 3.00 | 3.00 | 3.00 |
| BPO | 5.40 | 5.40 | 5.40 | 5.40 |

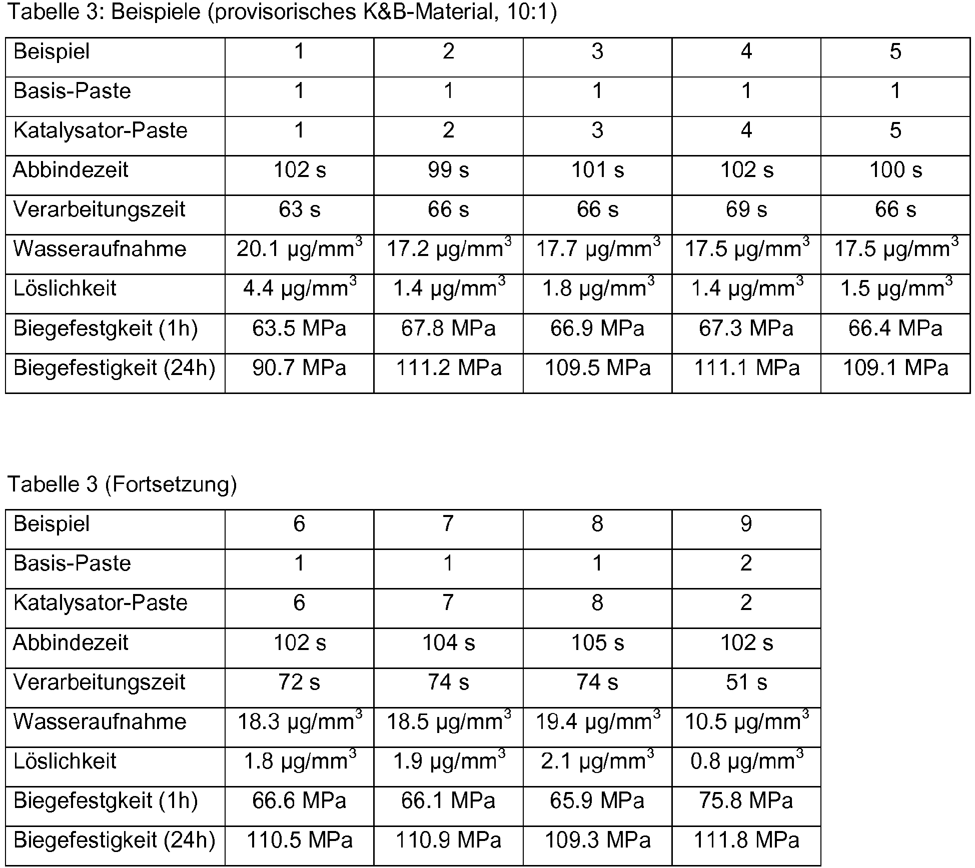

### Unterfütterungsmaterial (10:1)

**Tabelle 4: Basis-Pasten (Unterfütterungsmaterial)**

| | Basis 3 | Basis 4 |
|---|---|---|
| BisGMA | 18.00 | 0.00 |
| TEDMA | 10.00 | 3.00 |
| UDMA | 10.00 | 12.95 |
| TCD-DMA | 0.00 | 22.00 |
| GK | 55.85 | 55.85 |
| Aerosil R812 | 4.20 | 4.20 |
| Aerosil A300 | 1.40 | 1.40 |
| DEPT | 0.50 | 0.55 |
| BHT | 0.05 | 0.05 |

**Tabelle 5: Katalysator-Pasten (Unterfütterungsmaterial)**

| | Kat 9 | Kat 10 |
|---|---|---|
| BAP | 53.00 | 0.00 |
| Weichmacher "TCD" | 0.00 | 53.00 |
| GK | 35.60 | 35.60 |
| Aerosil R812 | 4.80 | 4.80 |
| Aerosil A300 | 1.60 | 1.60 |
| BPO | 5.00 | 5.00 |

**Tabelle 6: Beispiele Unterfütterungsmaterial (10:1)**

| Beispiel | 10 | 11 | 12 |
|---|---|---|---|
| Basis-Paste | 3 | 3 | 4 |
| Katalysator-Paste | 9 | 10 | 10 |
| Abbindezeit | 168 s | 168 s | 148 s |
| Verarbeitungszeit | 339 s | 327 s | 297 s |
| Wasseraufnahme | 20.2 µg/mm³ | 17.7 µg/mm³ | 11.3 µg/mm³ |
| Löslichkeit | 3.3 µg/mm³ | 1.6 µg/mm³ | 1.0 µg/mm³ |
| Biegefestigkeit (2x2) | 71.3 MPa | 80.0 MPa | 85.4 MPa |
| Haftung auf Paladur | 4.6 MPa | 7.7 MPa | 7.9 MPa |

Für die Herstellung der Basis- und Katalysator-Pasten der provisorischen K&B-Materialien sowie der Unterfütterungsmaterialien wurden die Bestandteile jeweils eingewogen an einem SpeedMixer™ DAC 600.1 VAC-P (Hauschild & Co KG, Hamm, Deutschland) homogenisiert, an einem Dreiwalzenstuhl (Exakt, Norderstedt, Deutschland) gewalzt und anschließend am SpeedMixer™ DAC 600.1 VAC-P bei -0,9 bar Vakuum entlüftet.
Die provisorischen K&B-Materialien und Unterfütterungsmaterialien wurden in S50-Kartuschen (50 ml, 10:1, CS 050-10-68, PSA 53-10-SI, PSB 53-10-SI) der Firma Sulzer Mixpac AG (Haag, Schweiz) abgefüllt, wobei die Basis-Paste jeweils in die große Kammer und die Katalysator-Paste jeweils in die kleine Kammer dieser 10:1-Kartuschen gefüllt wurde. Zum Mischen wurden die passenden, statischen Mischer MBX 3.2-16-S der Firma Sulzer Mixpac AG auf die Kartuschen aufgesetzt und die beiden Komponenten mit einem Dispenser (DS 50-10-00) ausgedrückt und homogen gemischt.

In den obigen Referenzbeispielen wurde der in dentalen Gemischen zurzeit gängig verwendete Weichmacher BAP als Verbindung aus dem Stand der Technik eingesetzt. Die Beispiele zum provisorischen K&B-Material zeigen, dass bei der erfindungsgemäßen Verwendung von dentalen Gemischen, umfassend Weichmacher mit der Struktur Q - [(Y)ₙ - X]ₒ dentale Werkstoffe erhältlich sind, die sich gegenüber dem Stand der Technik durch eine verringerte Wasseraufnahme und Löslichkeit bei gleichzeitig höherer mechanischer Festigkeit auszeichnen. Zudem wird kein nachteiliger Einfluss auf die Verarbeitungseigenschaften der dentalen Massen beobachtet. Ganz besonders vorteilhaft sind die Eigenschaften bei Verwendung eines Gemischs mit einer TCD-DMA/UDMA Harzmatrix.

Die Ergebnisse der Beispiele zum Unterfütterungsmaterial weisen genau in die gleiche Richtung wie die der Beispiele zum provisorischen K&B-Material. Zusätzlich zu den Vorteilen einer verringerten Wasseraufnahme und Löslichkeit bei verbesserten mechanischen Festigkeiten treten hier noch erhöhte Haftwerte der Unterfütterungsmaterialien auf einen Prothesenkunststoff auf.

**Infrarotspektroskopie (IR):** Die IR-Spektren wurden mit einem FT-IR Spektrometer Spectrum 100 (Perkin Elmer, Rodgau, Deutschland) gemessen.

**Brechungsindex** (***n***^{D}₂₀): Die Brechungsindices wurden an einem Refraktometer RE 40 (Mettler Toledo, Giessen, Deutschland) gemessen.

**Viskosität:** Die Viskositäten wurden mit einem Ubbelohde-Viskosimeter CS Lauda/CD15 Lauda/Kapillare IIc (Schott Instruments Analytics GmbH, Mainz, Deutschland) bei 20°C gemessen.

**Abbindezeit:** Die Abbindezeiten wurden an einem Thermoelementgerät nach ISO 4049 bestimmt.

**Verarbeitungszeit:** Die Verarbeitungszeiten wurden an einem Thermoelementgerät nach ISO 4049 bestimmt.

**Wasseraufnahme:** Die Wasseraufnahmen wurden nach ISO 4049 bestimmt.

**Löslichkeit:** Die Löslichkeiten wurden nach ISO 4049 bestimmt.

**Biegefestigkeit:** Die Biegefestigkeiten wurden analog zu ISO 4049 bestimmt. Für die provisorischen K&B-Materialien wurden abweichend von ISO 4049 Prüfkörper der Maße (25 ± 2) mm x (5,0 ± 0,1) mm x (5,0 ± 0,1) hergestellt. Zusätzlich zu den Biegefestigkeiten 24 Stunden nach Mischbeginn wurden für die provisorischen K&B-Materialien auch die Biegefestigkeiten 1 Stunde nach Mischbeginn bestimmt.

**Haftung:** Die Haftung der Unterfütterungsmaterialien wurde in einem Scherversuch an einer Universalprüfmaschine Zwick Z2.5 (Zwick GmbH & Co. KG, Ulm, Deutschland) bei einer Prüfgeschwindigkeit von 1 mm/min bestimmt. Prüfkörper mit einem Durchmesser von 25 mm und einer Höhe von 15 mm aus Paladur (Heraeus Kulzer GmbH, Hanau, Deutschland) wurden plangeschliffen (SiC-Schleifpapier, 180er Körnung). Auf die Prüfkörperoberfläche wurde Ufi Gel hard Adhäsiv (VOCO GmbH, Cuxhaven, Deutschland) aufgetragen und für 30 Sekunden an der Luft trocknengelassen. Anschließend wurde ein Formring (Durchmesser 8 mm, Höhe 6 mm) auf die Oberfläche aufgesetzt und mit dem angemischtem Unterfütterungsmaterial gefüllt. Die Proben wurden für 24 Stunden bei (37 ± 1) °C bei 100% Luftfeuchtigkeit ausgehärtet. Anschließend wurde der Formring entfernt und die Haftung zwischen Unterfütterungsmaterial und Paladur im Scherversuch als Quotient der Kraft beim Bruch und der Haftfläche bestimmt.

## Patentansprüche

1. Härtbares Gemisch umfassend
(a) eine oder mehrere während des Härtens mit den Bestandteilen (b) radikalisch nicht polymerisationsfähige Verbindung(en) der Struktur Q - [(Y)ₙ - X]ₒ, wobei hier und nachfolgend gilt:
- Q bedeutet ein polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten Yₙ - X substituierten Wasserstoffatome dieses polyalicyclischen Strukturelements Q durch Alkylgruppen, Alkoxygruppen, Halogenatome oder Trifluormethylgruppen substituiert sind;
- Y bedeutet Methylen (-CH₂-);
- n = 0 oder 1;
- X bedeutet -O-Z, -N-(Z)₂, -NH-Z, -O-C(=O)-Z, -C(=O)-O-Z, -O-C(=O)-NH-Z, -NH-C(=O)-O-Z, -NH-C(=O)-NH-Z, -C(=O)-NH-Z, -NH-C(=O)-Z, -C(=O)-N-(Z)₂, -N-(Z)-C(=O)-Z, -O-C(=O)-N(Z)-C(=O)-NH-Z, -NH-C(=O)-N(Z)-C(=O)-NH-Z, -N(C(=O)-NH-Z)₂, -C(=O)-N(Z)-C(=O)-NH-Z, -N(C(=O)-NH-Z)(C(=O)-Z), -N(C(=O)-NH-Z)(C(=O)-O-Z),
wobei die jeweils im Formelbild links angeordnete Bindung dem Strukturelement Q näher ist und wobei X so gewählt wird, dass Z möglichst wenige Atome zählt,
- Z bedeutet ein organischer Rest mit mindestens einem Kohlenstoffatom, wobei verschiedene Z unterschiedlich sein können,
- o = 2, 3, 4;
als Weichmacher
und
(b) weitere Bestandteile gewählt aus der Gruppe bestehend aus
(b-1) ein oder mehrere verschiedene Monomer(e),
(b-2) einen oder mehrere Füllstoffe,
(b-3) einen oder mehrere Photoinitiator(en) und/oder einen oder mehrere Initiator(en) für die chemische Aushärtung, und
(b-4) einen oder mehrere Polymerisationsinhibitor(en)
zur Anwendung in einem therapeutischen Verfahren bei der Herstellung eines dentalen Werkstoffs.

2. Härtbares Gemisch zur Anwendung in einem therapeutischen Verfahren bei der Herstellung eines dentalen Werkstoffs nach Anspruch 1, **dadurch gekennzeichnet, daß** Z ausgewählt ist aus der Gruppe bestehend aus:
a.) Kohlenwasserstoffresten [-R₁], wobei die Anzahl der C-Atome 1 bis 30 beträgt und wobei die Reste linear oder verzweigt sein können und
b.) Kohlenwasserstoffetherresten [(-R₂ - O)_{q} - R₃], wobei die Anzahl der C-Atome für R₂ 2 bis 6 beträgt und wobei die Anzahl der C-Atome für R₃ 1 bis 20 beträgt und wobei R₂ und R₃ linear oder verzweigt sein können und wobei q = 1 bis 15 ist und
c.) Kohlenwasserstoffesterresten [-R₄ - (C=O) - O - R₅] und [-R₄ - O - (C=O) - R₅], wobei die Anzahl der C-Atome für R₄ und R₅ 1 bis 15 beträgt und wobei R₄ und R₅ linear oder verzweigt sein können und
d.) Kohlenwasserstoffesterresten [-R₄ - ((C=O) - O-R₅)₂] und [-R₄ - (O - (C=O) - R₅)₂] und
e.) Kohlenwasserstoffaminoresten [-R₄ - N - (R₅)₂] und
f.) alkoxylierten Kohlenwasserstoffesterresten [(-R₂ - O)_{q} - R₄ - (C=O) - O - R₅] und [( - R₂ - O)_{q} - R₄ - O - (C=O) - R₅] und
g.) substituierten alkoxylierten Kohlenwasserstoffesterresten [(- R₂ - O)_{q} - CH₂ - O - (C=O) - NH - R₄ - (C=O) - O - R₅] und
h.) Kohlenwasserstoffalkoholresten mit endständiger Hydroxylgruppe [-R₄ - OH] und
i.) Kohlenwasserstoffalkoholresten mit einer, zwei oder mehreren nicht endständiger/endständigen Hydroxylgruppe(n) und bis zu 30 C-Atomen und
j.) alkoxylierten Kohlenwasserstoffalkoholresten [(-R₂ - O)_{q} - R₄ - OH] und
k.) Ketonresten [-R₆- (C=O) - R₇], wobei die Anzahl der C-Atome für R₆ 1 bis 30 beträgt und wobei die Anzahl der C-Atome für R₇ 1 bis 30 beträgt und wobei und wobei R₆ und R₇ linear oder verzweigt sein können.

3. Härtbares Gemisch zur Anwendung in einem therapeutischen Verfahren bei der Herstellung eines dentalen Werkstoffs nach Anspruch 1, **dadurch gekennzeichnet, daß** Z ausgewählt ist aus der Gruppe bestehend aus:
a.) Kohlenwasserstoffresten [-R₁], wobei die Anzahl der C-Atome 1 bis 9 beträgt und wobei die Reste linear oder verzweigt sein können und
b.) Kohlenwasserstoffetherresten [(-R₂ - O)_{q} -R₃], wobei die Anzahl der C-Atome für R₂ 2 bis 3 beträgt und wobei die Anzahl der C-Atome für R₃ 1 bis 5 beträgt und wobei R₂ und R₃ linear oder verzweigt sein können und wobei q = 1 bis 5 ist und
c.) Kohlenwasserstoffesterresten [-R₄ - (C=O) - O - R₅] und [-R₄ - O - (C=O) - R₅], wobei die Anzahl der C-Atome für R₄ und R₅ 1 bis 4 beträgt und wobei R₄ und R₅ linear oder verzweigt sein können und
d.) Kohlenwasserstoffesterresten [-R₄ - ((C=O) - O-R₅)₂] und [-R₄ - (O - (C=O) - R₅)₂] und
e.) Kohlenwasserstoffaminoresten [-R₄ - N - (R₅)₂] und
f.) alkoxylierten Kohlenwasserstoffesterresten [(-R₂ - O)_{q} - R₄ - (C=O) - O - R₅] und [( -R₂ - O)_{q} - R₄ - O - (C=O) - R₅] und
g.) substituierten alkoxylierten Kohlenwasserstoffesterresten [(-R₂ - O)_{q} - CH₂ - O - (C=O) - NH - R₄ - (C=O) - O - R₅] und
h.) Kohlenwasserstoffalkoholresten [- R₄ - OH] und
i.) Kohlenwasserstoffalkoholresten mit einer, zwei oder mehreren nicht endständiger/endständigen Hydroxylgruppe(n) und bis zu 30 C-Atomen und
j.) alkoxylierten Kohlenwasserstoffalkoholresten [(- R₂ - O)_{q} - R₄ - OH] und
k.) Ketonresten [- R₆ - (C=O) - R₇], wobei die Anzahl der C-Atome für R₆ 1 bis 10 beträgt und wobei die Anzahl der C-Atome für R₇ 1 beträgt und wobei R₆ linear oder verzweigt sein kann.

4. Härtbares Gemisch zur Anwendung in einem therapeutischen Verfahren bei der Herstellung eines dentalen Werkstoffs nach Anspruch 1, 2 oder 3 **dadurch gekennzeichnet, dass** Q ein gesättigtes polyalicyclisches Strukturelement, ausgewählt aus der Gruppe bestehend aus bicyclischen oder tricyclischen Kohlenwasserstoffresten, wobei keines der nicht durch Substituenten Yₙ - X substituierten Wasserstoffatome dieses polyalicyclischen Strukturelements Q substituiert ist.

5. Härtbares Gemisch zur Anwendung in einem therapeutischen Verfahren bei der Herstellung eines dentalen Werkstoffs nach Anspruch 1, 2, 3 oder 4 **dadurch gekennzeichnet, dass** Q einen Tricyclo[5.2.1.0^{2,6}]decanrest, Tricyclo[3.3.1.1^{3,7}]decanrest oder einen Bicyclo[2.2.1]heptanrest bedeutet.

6. Härtbares Gemisch zur Anwendung in einem therapeutischen Verfahren bei der Herstellung eines dentalen Werkstoffs nach einem der vorangehenden Ansprüche **dadurch gekennzeichnet, dass**
Bestandteil (a) gewählt ist aus der Gruppe bestehend aus 3(4),8(9)-Bis(acyloxymethyl)tricyclo[5.2.1.0^{2,6}]decan, dem alkoxylierten 3(4),8(9)-Bis(acyloxymethyl)tricyclo[5.2.1.0^{2,6}]decan, 2,3-Bis(acyloxymethyl)bicyclo[2.2.1]heptan, dem alkoxylierten 2,3-Bis(acyloxymethyl)bicyclo[2.2.1]heptan, 1,3,5-Triacyloxytricyclo[3.3.1.1^{3,7}]decan, dem alkoxylierten Triacyloxytricyclo[3.3.1.1^{3,7}]decan, Tricyclo[5.2.1.0^{2,6}]decan-3(4),8(9)-dicarbonsäureester, dem michaelartigen Adduct aus 3(4),8(9)-Bis(aminomethyl)tricyclo[5.2.1.0^{2,6}]decan und Acrylsäureester, 3(4), 8(9)-Bis-(alkyloxymethyl)tricyclo[5.2.1.0^{2,6}]decan, dem alkoxylierten 3(4), 8(9)-Bis-(alkyloxymethyl)tricyclo[5.2.1.0^{2,6}]decan, dem Additionsprodukt aus 3(4), 8(9)-Bis(hydroxymethyl)tricyclo[5.2.1.0^{2,6}]decan mit Isocyanat, dem Additionsprodukt aus alkoxyliertem 3(4), 8(9)-Bis(hydroxymethyl)tricyclo[5.2.1.0^{2,6}]decan mit Isocyanat, dem Additionsprodukt aus 3(4), 8(9)-Bis(aminomethyl)tricyclo[5.2.1.0^{2,6}]decan mit Isocyanat beziehungsweise dem Reaktionsprodukt aus 3(4), 8(9)-Bis(isocyanatomethyl)tricyclo[5.2.1.0^{2,6}]decan mit Aminen, dem Additionsprodukt aus alkoxyliertem 3(4), 8(9)-Bis(aminomethyl)tricyclo[5.2.1.0^{2,6}]decan mit Isocyanat, dem Additionsprodukt aus 3(4), 8(9)-Bis(isocyanatomethyl)tricyclo[5.2.1.0^{2,6}]decan mit Alkohol, dem Additionsprodukt aus 3(4), 8(9)-Bis(isocyanatomethyl)tricyclo[5.2.1.0^{2,6}]decan mit alkoxyliertem Alkohol, dem Umsetzungsprodukt aus 3(4), 8(9)-Bis(carbonsäurehalogenid)tricyclo[5.2.1.0^{2,6}]decan mit einem Amin, beziehungsweise dem Umsetzungsprodukt aus 3(4), 8(9)-Bis(carbonsäure)tricyclo[5.2.1.0^{2,6}]decan mit einem Isocyanat, dem Umsetzungsprodukt aus 3(4), 8(9)-Bis(isocyanatomethyl)tricyclo[5.2.1.0^{2,6}]decan mit einem Aminoalkohol, dem Umsetzungsprodukt aus 3(4), 8(9)-Bis(isocyanatomethyl)tricyclo[5.2.1.0^{2,6}]decan mit einem Aminoalkohol und anschließender Alkoxylierung des resultierenden Alkohols, dem Umsetzungsprodukt aus 3(4), 8(9)-Bis(isocyanatomethyl)tricyclo[5.2.1.0^{2,6}]decan mit einem Aminoalkohol, anschließender Alkoxylierung des resultierenden Alkohols und Reaktion dieser Verbindung mit Carbonsäure/Carbonsäureanhydrid, dem Umsetzungsprodukt aus 3(4), 8(9)-Bis(aminomethyl)tricyclo[5.2.1.0^{2,6}]decan mit Alkylhalogeniden, dem Umsetzungsprodukt aus 3(4), 8(9)-Bis(aminomethyl)tricyclo[5.2.1.0^{2,6}]decan mit Säurehalogeniden, beziehungsweise dem Umsetzungsprodukt aus 3(4), 8(9)-Bis(isocyanatomethyl)tricyclo[5.2.1.0^{2,6}]decan mit Carbonsäuren, dem Umsetzungsprodukt von 3(4), 8(9)-Bis(hydroxymethyl)tricyclo[5.2.1.0^{2,6}]decan, beziehungsweise von alkoxyliertem 3(4), 8(9)-Bis(hydroxymethyl)tricyclo[5.2.1.0^{2,6}]decan, mit Fettsäuren sowie deren weitere Umsetzungen im Falle von Reaktionsprodukten ungesättigter Fettsäuren zu den entsprechenden Ketonen gemäß dem Wacker Prozeß, dem Umsetzungsprodukt aus 3(4), 8(9)-Bis(aminomethyl)tricyclo[5.2.1.0^{2,6}]decan mit Epoxiden und deren weiteres Reaktionsprodukt mit Carbonsäuren, dem alkoxylierten 3(4), 8(9)-Bis(hydroxymethyl)tricyclo[5.2.1.0^{2,6}]decan und dem alkoxylierten 3(4), 8(9)-Bis(aminomethyl)tricyclo[5.2.1.0^{2,6}]decan,
Bestandteil (b-1) gewählt ist aus der Gruppe bestehend aus 3(4), 8(9)-Bis((meth)acryloyloxymethyl)tricyclo[5.2.1.0^{2,6}]decan, dem alkoxylierten 3(4),8(9)-Bis((meth)acryloyloxymethyl)tricyclo[5.2.1.0^{2,6}]decan, 2,3-Bis((meth)acryloyloxymethyl)bi-cyclo[2.2.1]heptan, dem alkoxylierten 2,3-Bis((meth)acryloyloxymethyl)bicyclo[2.2.1]heptan, 1,3,5-Tri(meth)acryloyloxytricyclo[3.3.1.1^{3,7}]decan, dem alkoxylierten Tri(meth)acyloyloxytricyclo[3.3.1.1^{3,7}]decan, (Meth)acrylsäureester von Tricyclo[5.2.1.0^{2,6}]decan-3(4),8(9)-dimethanol, alkoxyliertem Tricyclo[5.2.1.0^{2,6}]decan-3(4),8(9)-dimethanol, Bicyclo[2.2.1]-heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind, Ethylenglykoldi(meth)acrylat, 1,6-Hexandioldi(meth)acrylat (HEDMA), Triethylenglykoldi(meth)acrylat (TEDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglykoldi(meth)acrylat, 7,7,9-Trimethyl-4,13-dioxo-5,12-diazahexadecan-1,16-dioxydi(meth)acrylat (UDMA), Butandioldi(meth)acrylat, Tetraethylenglykoldi(meth)acrylat, Neopentylglykoldi(meth)acrylat, 2-Hydroxypropyl-1,3-di(meth)acrylat, 3-Hydroxypropyl-1,2-di(meth)acrylat, Pentaerythritol-di(meth)acrylat, Di(meth)acrylaten des Dihydroxymethyltricyclo[5.2.1.0^{2,6}]decans, 2-Hydroxyethyl(meth)acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, 2,2-Bis[4-[3-(meth)acryloyloxy-2-hydroxypropoxy]phenyl]propan (Bis-GMA), Trimethylolpropantri(meth)acrylat, Trimethylolethantri(meth)acrylat, Pentaerythritoltri(meth)-acrylat, Trimethylolmethantri(meth)acrylat, Pentaerythritoltetra(meth)acrylat, Ditrimethylolpropantetra(meth)acrylat, Pentaerythritolhexa(meth)acrylat, Butylenglykoldi(meth)acrylat, Propylenglykoldi(meth)acrylat, Nonandioldi(meth)acrylat, Decandioldi(meth)acrylat, Glycerolmono(meth)acrylat, Glyceroldi(meth)acrylat, Trimethylolpropanmono(meth)acrylat, Trimethylolpropandi(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)-acrylat, Allyl(meth)acrylat, Glycidyl(meth)acrylat, 2-Ethoxyethyl(meth)acrylat, Methoxypolyethylenglykol(meth)acrylat, Isobornyl(meth)acrylat, 2-(N,N-Dimethylamino)ethyl(meth)acrylat, N-Methylol(meth)acrylamid, Diaceton(meth)acrylamide, 2,2-Bis[4-(meth)acryloyloxyphenyl]-propan, 2,2-Bis[4-(meth)acryloyloxyethoxyphenyl]propan, 2,2-Bis[4-(meth)acryloyloxydiethoxyphenyl]propan, 2,2-Bis[4-(meth)acryloyloxytriethoxyphenyl]propan 2,2-Bis[4-(meth)acryloyloxytetraethoxyphenyl]propan, 2,2-Bis[4-(meth)acryloyloxypentaethoxyphenyl]-propan, 2,2-Bis[4-(meth)acryloyloxydipropoxyphenyl]propan, 2,2-Bis[4-(meth)acryloyloxyethoxyphenyl]-2-[4-(meth)acryloyloxydiethoxyphenyl]propan, 2-[4-(Meth)acryloyloxydiethoxyphenyl]-2-[4-(meth)acryloyloxytriethoxyphenyl]propan, 2-[4-(Meth)acryloyloxdipropoxyphenyl]-2-[4-(meth)acryloyloxytriethoxyphenyl]propan, 2,2-Bis[4-(meth)acryloyloxyisopropoxy phenyl]propan, Hydroxypivalinsäureneopentylglykoldi(meth)acrylat, Acetoacetoxyethyl-(meth)acrylat, Polypropylenglykoldi(meth)acrylat, Glycerolalkoxylatdimethacrylat, Neopentylglykol(meth)acrylat, N,N-(1,2-Dihydroxyethylen)bisacrylamid, 2,2-Bis[4-(meth)acryloyloxypentaethoxyphenyl]propan, 2,2-Bis[4-(meth)acryloyloxypolyethoxyphenyl]-propan, Diethylenglykoldi(meth)acrylat, Dipentaerythritoltetra(meth)acrylat, Dipentaerythritolhexa(meth)acrylat, N,N-(2,2,4-Trimethylhexamethylen)bis[2-(aminocarboxy)propan-1,3-diol]-tetra(meth)acrylat, das Kondensationsprodukt von 3, (4)- (Meth)acryloxymethyl-8, (9) - hydroxymethyltricyclo-[5.2.1.0^{2,6}]decan mit Dicarbonsäuren, 2-Ethylhexyl(meth)acrylat, Tridecyl(meth)acrylat, Stearyl(meth)acrylat, Cyclohexyl(meth)acrylat, Benzyl(meth)acrylat, Methoxydiethylenglykol(meth)acrylat, Dicyclopentenyl(meth)acrylat, Phenyl(meth)acrylat, Pentaerythritolmono(meth)acrylat, Dipentaerythritolmono(meth)acrylat, Caprolacton modifiziertes Tetrahydrofurfuryl(meth)acrylat sowie polymerisierbare Monomere auf Basis von Polysiloxanen,
Bestandteil (b-2) gewählt ist aus der Gruppe bestehend aus amorphen Materialien auf der Basis von Mischoxiden aus SiO₂, ZrO₂ und/oder TiO₂, mikrofeinen Füllstoffen wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid sowie nanoskaligen Feststoffteilchen mit einer mittleren Teilchengröße von nicht mehr als 200 nm, bevorzugt nicht mehr als 100 nm und insbesondere nicht mehr als 70 nm und die bevorzugt nicht agglomeriert und/oder nicht aggregiert sind, wobei die nanoskaligen anorganischen Feststoffteilchen bevorzugt solche von Oxiden, Sulfiden, Seleniden und Telluriden von Metallen, Halbmetallen und deren Mischungen wie SiO₂, TiO₂, ZrO₂, ZnO, SnO₂ und Al₂O₃, und organischen Polymerisaten sind,
Bestandteil (b-3) gewählt ist aus der Gruppe bestehend aus alpha-Diketonen, Benzoinalkylether, Thioxanthonen, Benzophenonen, Acylphosphinoxiden, Acetophenonen, Ketalen, Titanocenen, sensibilisierenden Farbstoffen sowie Boratsalzen und Benzoylperoxid, Lauroylperoxid in Kombination mit Aminen, Barbitursäuren bzw. Barbitursäurederivaten sowie Malonylsulfamiden, Natriumbenzolsulfinaten oder Natriumparatoluolsulfinaten in Kombination mit Schwermetallsalzen,
Bestandteil (b-4) gewählt ist aus der Gruppe bestehend aus Hydrochinonmonomethylether, 2,6-Di-tert.butyl-4-methylphenol, 2,2-Diphenyl-1-picrylhydrazyl-, Galvinoxyl-, Triphenylmethyl-Radikalen, 2,3,6,6,-Tetramethylpiperidinyl-1-oxylradikalens sowie deren Derivaten, oder Phenothiazin,
Bestandteil (b-5) gewählt ist aus der Gruppe bestehend aus Toluol, Xylol, Isooctan, Aceton, Butanon, Methylisobutylketon, Ethylacetat, Butylacetat, Tetrahydrofuran, N-Methylpyrrolidon, Dimethylacetamid, Dimethylformamid, Ethanol, Propanolen, Butanolen, Pentanolen, Hexanolen, Cyclohexanol, Heptanolen, Octanolen, Nonanolen, Decanolen, sowie cycloaliphatischen oder arylaliphatischen Alkoholen.

7. Härtbares Gemisch zur Anwendung in einem therapeutischen Verfahren bei der Herstellung eines dentalen Werkstoffs nach Anspruch 6 **dadurch gekennzeichnet, dass** die Stöchiometrie der Umsetzungen von
- 3(4), 8(9)-Bis(hydroxymethyl)tricyclo[5.2.1.0^{2,6}]decan mit Isocyanat oder von alkoxyliertem 3(4), 8(9)-Bis(hydroxymethyl)tricyclo[5.2.1.0^{2,6}]decan mit Isocyanat von 1:1 bis 1:2 reicht, so dass die Reaktionsprodukte Urethane, Allophanate sowie deren Gemische, in denen die Urethane nicht vollständig zu den Allophanaten abreagiert sind, enthalten können,
- 3(4), 8(9)-Bis(aminomethyl)tricyclo[5.2.1.0^{2,6}]decan mit Isocyanat oder von alkoxyliertem 3(4), 8(9)-Bis(aminomethyl)tricyclo[5.2.1.0^{2,6}]decan mit Isocyanat von 1:1 bis 1:2 reicht, so dass die Reaktionsprodukte Harnstoffe, Biurete sowie deren Gemische, in denen die Harnstoffe nicht vollständig zu den Biureten abreagiert sind, enthalten können,
- 3(4), 8(9)-Bis(carbonsäure)tricyclo[5.2.1.0^{2,6}]decan mit Isocyanat von 1:1 bis 1:2 reicht, so dass die Reaktionsprodukte Amide, Acylharnstoffe sowie deren Gemische, in denen die Amide nicht vollständige zu den Acylharnstoffen abreagiert sind, enthalten können,
- 3(4), 8(9)-Bis(carbonsäurehalogenid)tricyclo[5.2.1.0^{2,6}]decan mit Amin 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann,
- 3(4), 8(9)-Bis(isocyanatomethyl)tricyclo[5.2.1.0^{2,6}]decan mit Alkohol oder mit alkoxyliertem Alkohol 1:1 beträgt und ein Urethan ergibt und die zweite Umsetzungsstufe zum Allophanat mit einem Monoisocyanat in einer Stöchiometrie bis 1:1 erfolgt, so daß das Reaktionsprodukt Urethane, Allophanate sowie deren Gemische enthalten kann,
- 3(4), 8(9)-Bis(isocyanatomethyl)tricyclo[5.2.1.0^{2,6}]decan mit Amin 1:1 beträgt und einen Harnstoff ergibt und die zweite Umsetzungsstufe zum Biuret mit einem Monoisocyanat in einer Stöchiometrie bis 1:1 erfolgt, so dass das Reaktionsprodukt Harnstoffe, Biurete sowie deren Gemische enthalten kann,
- 3(4), 8(9)-Bis(isocyanatomethyl)tricyclo[5.2.1.0^{2,6}]decan mit Carbonsäure 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann,
- 3(4), 8(9)-Bis(aminomethyl)tricyclo[5.2.1.0^{2,6}]decan mit Säurehalogeniden 1:1 beträgt und ein Amid ergibt und die zweite Umsetzungsstufe zum Acylharnstoff mit einem Monoisocyanat in einer Stöchiometrie bis 1:1 erfolgt, so dass das Reaktionsprodukt Amide, Acylharnstoffe sowie deren Gemische enthalten kann.

8. Härtbares Gemisch zur Anwendung in einem therapeutischen Verfahren bei der Herstellung eines dentalen Werkstoffs nach einem der vorangehenden Ansprüche, **dadurch gekennzeichnet, daß**
(a) 0,5 bis 20 Gew.-%, vorzugsweise 2 bis 10 Gew.-%
(b-1) 5 bis 90 Gew.-%, vorzugsweise 25 bis 80 Gew.-%
(b-2) 1 bis 85 Gew.-%, vorzugsweise 20 bis 75 Gew.-%
(b-3) 0,05 bis 8 Gew.-%, vorzugsweise 0,5 bis 4 Gew.-%
(b-4) 0 bis 1 Gew.-%, vorzugsweise 0,01 bis 0,2 Gew.-%
(b-5) 0 bis 85 Gew.-%, vorzugsweise 0 bis 75 Gew.-%
beträgt.

9. Härtbares Gemisch zur Anwendung in einem therapeutischen Verfahren bei der Herstellung eines dentalen Werkstoffs nach einem der Ansprüche 1 bis 7 **dadurch gekennzeichnet, daß**
(a) 2 bis 10 Gew.-%
(b-1) 25 bis 80 Gew.-%
(b-2) 20 bis 75 Gew.-%
(b-3) 0,5 bis 4 Gew.-%
(b-4) 0,01 bis 0,2 Gew.-%
(b-5) 0 bis 75 Gew.-%
beträgt.

10. Härtbares Gemisch zur Anwendung in einem therapeutischen Verfahren bei der Herstellung eines dentalen Werkstoffs nach Anspruch 6 und 9, enthaltend
(a) 2 bis 10 Gew.-% eines Weichmachers, ausgewählt aus der Gruppe bestehend aus 3(4),8(9)-Bis(acetyloxymethyl)tricyclo[5.2.1.0²⁶]decan, dem alkoxylierten 3(4),8(9)-Bis(acetyloxymethyl)tricyclo[5.2.1.0^{2,6}]decan, 2,3-Bis(acetyloxymethyl)bicyclo[2.2.1]heptan, dem alkoxylierten 2,3-Bis(acetyloxymethyl)bicyclo[2.2.1]heptan, 1,3,5-Triacetyloxytricyclo[3.3.1.1^{3,7}]decan, dem alkoxylierten Triacetyloxytricyclo[3.3.1.1^{3,7}]decan, Tricyclo[5.2.1.0^{2,6}]decan-3(4),8(9)-dicarbonsäureethylester und dem michaelartigen Adduct aus 3(4),8(9)-Bis(aminomethyl)tricyclo[5.2.1.0^{2,6}]decan und Acrylsäuremethylester,
(b-1) 25 bis 80 Gew.-% eines radikalisch härtbaren Monomers, ausgewählt aus der Gruppe bestehend aus 7,7,9-Trimethyl-4,13-dioxo-5,12-diazahexadecan-1,16-dioxydi(meth)acrylat (UDMA), Triethylenglykoldi(meth)acrylat (TEDMA) und 3(4), 8(9)-Bis(methacryloyloxymethyl)tricyclo[5.2.1.0^{2,6}]decan,
(b-2) 20 bis 75 Gew.-% eines Füllstoffs, ausgewählt aus der Gruppe bestehend aus Glaskeramik, Kieselsäuren, röntgenopaken Füllstoffen, nanoskaligen Füllstoffen unter 200 nm, wobei letztere bevorzugt nicht-agglomeriert und/oder nicht-aggregiert sind sowie organischen Polymerisaten,
(b-3) 0,5 bis 4 Gew.-% eines Photoinitoators, ausgewählt aus der Gruppe bestehend aus Campherchinon/Amin und Phosphinoxid und/oder eines chemischen Initiators ausgewählt aus der Gruppe bestehend aus Peroxid/Amin und Barbitursäure/Barbitursäurederivaten in Kombination mit Schwermetallsalzen.

11. Härtbares Gemisch zur Anwendung in einem therapeutischen Verfahren bei der Herstellung eines dentalen Werkstoffs nach einem der vorangehenden Ansprüche **dadurch gekennzeichnet, dass** Bestandteil (b-2) organisch oberflächenmodifiziert ist.

12. Härtbares Gemisch zur Anwendung in einem therapeutischen Verfahren bei der Herstellung eines dentalen Werkstoffs nach Anspruch 11, **dadurch gekennzeichnet, daß** Bestandteil (b-2) silanisiert ist.

13. Härtbares Gemisch zur Anwendung in einem therapeutischen Verfahren bei der Herstellung eines dentalen Werkstoffs nach einem der vorangehenden Ansprüche, **dadurch gekennzeichnet, dass** der dentale Werkstoff ein gehärtetes Füllungsmaterial, Stumpfaufbaumaterial, provisorisches Kronen- und Brückenmaterial, ein Befestigungszement, ein Unterfütterungsmaterial, ein zahntechnischer Werkstoff, ein Unterfüllungsmaterial, eine Abdeckmasse zum Gingivaschutz, ein Prothesenmaterial, das Material einer provisorischen Suprakonstruktion für ein dentales Implantat oder eines Kerns für eine provisorische Suprakonstruktion oder ein Inlay, Onlay oder eine Verblendung ist.

14. Härtbares Gemisch zur Anwendung in einem therapeutischen Verfahren bei der Herstellung eines dentalen Werkstoffs nach einem der vorangehenden Ansprüche, **dadurch gekennzeichnet, dass** das härtbare Gemisch ein Zweikomponentensysten darstellt, wobei Bestandteil (b-3) ein Redoxsystem umfasst, enthaltend ein Reduktionsmittel und ein Oxidationsmittel und wobei das härtbare Gemisch in zwei räumlich voneinander getrennten Komponenten in Form von Pasten vorliegt und wobei das Reduktionsmittel in der ersten Komponente enthalten ist und das Oxidationsmittel in der zweiten Komponente enthalten ist und wobei die Bestandteile (a), (b-1), (b-2), gegebenenfalls (b-4) und gegebenenfalls (b-5) in der ersten und/oder zweiten Komponente vorliegen und wobei die Pasten in Mischungsverhältnissen von erster Komponente zu zweiter Komponente im Verhältnis von 10:1 bis 1:10 vorliegen.

15. Härtbares Gemisch zur Anwendung in einem therapeutischen Verfahren nach einem der Ansprüche 1 bis 12 und 14, das nach Härten ein Unterfütterungsmaterial ergibt, **dadurch gekennzeichnet, dass** das Unterfütterungsmaterial eine Biegefestigkeit, gemessen nach ISO 4049, von mehr als 73 MPa ergibt.

16. Härtbares Gemisch zur Anwendung in einem therapeutischen Verfahren nach einem der Ansprüche 1 bis 12 und 14, das nach Härten ein provisorisches Kronen und Brückenmaterial ergibt, **dadurch gekennzeichnet, dass** das provisorische Kronen und Brückenmaterial eine Löslichkeit, gemessen nach ISO 4049, von weniger als 2,5 µg/mm³ ergibt.

## Claims

1. Curable mixture comprising
(a) one or more compound(s) of the structure Q - [(Y)ₙ - X]ₒ which are not free-radically polymerizable during the curing with the constituents (b), wherein, here and hereinafter:
- Q is a polyalicyclic structural element selected from the group consisting of bicyclic, tricyclic, tetracyclic, pentacyclic and hexacyclic hydrocarbyl rests, wherein one, two or more of the hydrogen atoms not substituted by Yₙ - X substituents in this polyalicyclic structural element Q are optionally replaced by alkyl groups, alkoxy groups, halogen atoms or trifluoromethyl groups;
- Y is methylene (-CH₂-);
- n = 0 or 1;
- X is -O-Z, -N-(Z)₂, -NH-Z, -O-C(=O)-Z, -C(=O)-O-Z, -O-C(=O)-NH-Z, -NH-C(=O)-O-Z, -NH-C(=O)-NH-Z, -C(=O)-NH-Z, -NH-C(=O)- Z, -C(=O)-N-(Z)₂, -N-(Z)-C(=O)-Z, -O-C(=O)-N(Z)-C(=O)-NH-Z, -NH-C(=O)-N(Z)-C(=O)-NH-Z, -N(C(=O)-NH-Z)₂, -C(=O)-N(Z)-C(=O)-NH-Z, -N(C(=O)-NH-Z)(C(=O)-Z), -N(C(=O)-NH-Z)(C(=O)-O-Z), wherein the bond arranged on the left in each formula is closer to the structural element Q and wherein X is selected such that Z has a minimum number of atoms,
- Z is an organic radical having at least one carbon atom, and different Z may be different,
- o = 2, 3, 4,
as a plasticiser
and
(b) further constituents selected from the group consisting of
(b-1) one or more different monomer(s),
(b-2) one or more fillers,
(b-3) one or more photoinitiator(s) and/or one or more initiator(s) for chemical curing,
(b-4) one or more polymerization inhibitor(s) for use in a therapeutic method in the production of a dental material.

2. Curable mixture for use in a therapeutic method in the production of a dental material according to claim 1, wherein Z is selected from the group consisting of:
a.) hydrocarbyl radicals [-R₁] wherein the number of carbon atoms is 1 to 30 and wherein the radicals may be linear or branched, and
b.) hydrocarbyl ether radicals [(-R₂ - O)_{q} - R₃] wherein the number of carbon atoms for R₂ is 2 to 6, and wherein the number of carbon atoms for R₃ is 1 to 20, and wherein R₂ and R₃ may be linear or branched and wherein q = 1 to 15, and
c.) hydrocarbyl ester radicals [- R₄ - (C=O) - O - R₅] and [-R₄ - O - (C=O) - R₅] wherein the number of carbon atoms for R₄ and R₅ is 1 to 15, and wherein R₄ and R₅ may be linear or branched and
d.) hydrocarbyl ester radicals [- R₄ - ((C=O) - O - R₅)₂] and [-R₄ - (O - (C=O) - R₅)₂] and
e.) hydrocarbyl amino radicals [- R₄ - N - (R₅)₂] and
f.) alkoxylated hydrocarbyl ester radicals [(-R₂ - O)_{q} - R₄ - (C=O) - O - R₅] and [(-R₂ - O)_{q} - R₄ - O - (C=O) - R₅] and
g.) substituted alkoxylated hydrocarbyl ester radicals [(-R₂ - O)_{q} - CH₂ - O - (C=O) - NH - R₄ - (C=O) - O - R₅] and
h.) hydrocarbyl alcohol radicals having a terminal hydroxyl group [- R₄ - OH] and
i.) hydrocarbyl alcohol radicals having one, two or more non-terminal hydroxyl group(s) and up to 30 carbon atoms and
j.) alkoxylated hydrocarbyl alcohol radicals [(-R₂ - O)_{q} - R₄ - OH] and
k.) ketone radicals [-R₆ - (C=O) - R₇] wherein the number of carbon atoms for R₆ is 1 to 30, and wherein the number of carbon atoms for R₇ is 1 to 30, and wherein R₆ and R₇ may be linear or branched.

3. Curable mixture for use in a therapeutic method in the production of a dental material according to claim 1, wherein Z is selected from the group consisting of:
a.) hydrocarbyl radicals [-R₁] wherein the number of carbon atoms is 1 to 9 and wherein the radicals may be linear or branched and
b.) hydrocarbyl ether radicals [(-R₂ - O)_{q} - R₃] wherein the number of carbon atoms for R₂ is 2 to 3 and where the number of carbon atoms for R₃ is 1 to 5 and wherein R₂ and R₃ may be linear or branched and wherein q = 1 to 5 and
c.) hydrocarbyl ester radicals [-R₄ - (C=O) - O - R₅] and [-R₄ - O - (C=O) - R₅] wherein the number of carbon atoms for R₄ and R₅ is 1 to 4 and wherein R₄ and R₅ may be linear or branched and
d.) hydrocarbyl ester radicals [-R₄ - ((C=O) - O - R₅)₂] and [-R₄ - (O - (C=O) - R₅)₂] and
e.) hydrocarbyl amino radicals [- R₄ - N - (R₅)₂] and
f.) alkoxylated hydrocarbyl ester radicals [(-R₂ - O)_{q} - R₄ - (C=O) - O - R₅] and [(-R₂ - O)_{q} - R₄ - O - (C=O) - R₅] and
g.) substituted alkoxylated hydrocarbyl ester radicals [(-R₂ - O)_{q} - CH₂ - O - (C=O) - NH - R₄ - (C=O) - O - R₅] and
h.) hydrocarbyl alcohol radicals [- R₄ - OH] and
i.) hydrocarbyl alcohol radicals having one, two or more non-terminal hydroxyl group(s) and up to 30 carbon atoms and
j.) alkoxylated hydrocarbyl alcohol radicals [(- R₂ - O)_{q} - R₄ - OH] and
k.) ketone radicals [- R₆ - (C=O) - R₇] wherein the number of carbon atoms for R₆ is 1 to 10, and wherein the number of carbon atoms for R₇ is 1 and wherein R₆ may be linear or branched.

4. Curable mixture for use in a therapeutic method in the production of a dental material according to claims 1, 2 or 3, wherein Q is a saturated polyalicyclic structural element selected from the group consisting of bicyclic and tricyclic hydrocarbyl rests, wherein none of the hydrogen atoms unsubstituted by Yₙ - X substituents in the polyalicyclic structural element Q is substituted.

5. Curable mixture for use in a therapeutic method in the production of a dental material according to claims 1, 2, 3 or 4, wherein Q is a tricyclo[5.2.1.0^{2,6}]decane radical, a tricyclo[3.3.1.1^{3,7}]decane radical or a bicyclo[2.2.1]heptane radical.

6. Curable mixture for use in a therapeutic method in the production of a dental material according to any of the preceding claims, wherein
constituent (a) is selected from the group consisting of 3(4),8(9)-bis(acyloxymethyl)-tricyclo[5.2.1.0^{2,6}]decane, alkoxylated 3(4),8(9)-bis(acyloxymethyl)tricyclo[5.2.1.0^{2,6}]decane, 2,3-bis(acyloxymethyl)bicyclo[2.2.1]heptane, alkoxylated 2,3-bis(acyloxymethyl)bicyclo[2.2.1]heptane, 1,3,5-triacyloxytricyclo[3.3.1.1^{3,7}]decane, alkoxylated triacyloxytricyclo[3.3.1.1³,⁷]decane, tricyclo[5.2.1.0^{2,6}]decane-3(4),8(9)-dicarboxylic ester, the Michael-type adduct of 3(4),8(9)-bis(aminomethyl)-tricyclo[5.2.1.0^{2,6}] decane and acrylic ester, 3(4),8(9)-bis(alkyloxymethyl)tricyclo[5.2.1.0^{2,6}]decane, alkoxylated 3(4),8(9)-bis(alkyloxymethyl)tricyclo[5.2.1.0^{2,6}]decane, the addition product of 3(4),8(9)-bis(hydroxymethyl)tricyclo[5.2.1.0^{2,6}] decane with isocyanate, the addition product of alkoxylated 3(4),8(9)-bis(hydroxymethyl)tricyclo[5.2.1.0^{2,6}]decane with isocyanate, the addition product of 3(4),8(9)-bis(aminomethyl)tricyclo[5.2.1.0^{2,6}]decane with isocyanate or the reaction product of 3(4),8(9)-bis(isocyanatomethyl)-tricyclo[5.2.1.0^{2,6}]decane with amines, the addition product of alkoxylated 3(4),8(9)-bis-(aminomethyl)tricyclo[5.2.1.0^{2,6}]decane with isocyanate, the addition product of 3(4),8(9)-bis(isocyanatomethyl)tricyclo[5.2.1.0^{2,6}]decane with alcohol, the addition product of 3(4),8(9)-bis(isocyanatomethyl)tricyclo[5.2.1.0^{2,6}]decane with alkoxylated alcohol, the reaction product of 3(4),8(9)-bis(carbonyl halide)tricyclo[5.2.1.0^{2,6}]decane with an amine, or the reaction product of 3(4),8(9)-bis(carboxylic acid)tricyclo[5.2.1.0^{2,6}]decane with an isocyanate, the reaction product of 3(4),8(9)-bis(isocyanatomethyl)tricyclo[5.2.1.0^{2,6}]decane with an amino alcohol, the reaction product of 3(4),8(9)-bis(isocyanatomethyl)tricyclo[5.2.1.0^{2,6}]decane with an amino alcohol and subsequent alkoxylation of the resulting alcohol, the reaction product of 3(4),8(9)-bis(isocyanatomethyl)tricyclo[5.2.1.0^{2,6}] decane with an amino alcohol, subsequent alkoxylation of the resulting alcohol and reaction of this compound with carboxylic acid/carboxylic anhydride, the reaction product of 3(4),8(9)-bis(aminomethyl)tricyclo[5.2.1.0^{2,6}]decane with alkyl halides, the reaction product of 3(4),8(9)-bis(aminomethyl)tricyclo[5.2.1.0^{2,6}]decane with acid halides, or the reaction product of 3(4),8(9)-bis(isocyanatomethyl)tricyclo[5.2.1.0^{2,6}]decane with carboxylic acids, the reaction product of 3(4),8(9)-bis(hydroxymethyl)tricyclo[5.2.1.0^{2,6}]decane, or of alkoxylated 3(4),8(9)- bis(hydroxymethyl)tricyclo[5.2.1.0^{2,6}]decane, with fatty acids and the further reactions thereof, in the case of reaction products of unsaturated fatty acids, to give the corresponding ketones by the Wacker process, the reaction product of 3(4),8(9)-bis(aminomethyl)tricyclo[5.2.1.0^{2,6}]decane with epoxides and the further reaction product thereof with carboxylic acids, alkoxylated 3(4),8(9)-bis(hydroxymethyl)tricyclo[5.2.1.0^{2,6}]decane and alkoxylated 3(4),8(9)-bis(aminomethyl)tricyclo[5.2.1.0^{2,6}]decane,
constituent (b-1) is selected from the group consisting of 3(4),8(9)-bis((meth)acryloyloxymethyl)tricyclo[5.2.1.0^{2,6}]-decane, alkoxylated 3(4),8(9)-bis((meth)acryloyloxymethyl)-tricyclo[5.2.1.0^{2,6}]decane, 2,3-bis((meth)acryloyloxymethyl)-bicyclo[2.2.1]heptane, alkoxylated 2,3-bis((meth)acryloyloxymethyl)bicyclo[2.2.1]heptane, 1,3,5-tri(meth)acryloyloxytricyclo[3.3.1.1^{3,7}]decane, alkoxylated tri(meth)-acryloyloxytricyclo[3.3.1.1^{3,7}]decane, (meth)acrylic esters of tricyclo[5.2.1.0^{2,6}]decane-3(4),8(9)-dimethanol, alkoxylated tricyclo[5.2.1.0^{2,6}]decane-3(4),8(9)-dimethanol, bicyclo[2.2.1]heptane-2,3-dimethanol, alkoxylated bicyclo[2.2.1]heptane-2,3-dimethanol, 1,3,5-adamantanetriol alkoxylated 1,3,5-adamantanetriol, with arrangement of urethane, urea, amide, allophanate, acylurea or biuret groups between the polyalicyclic structural element and the (meth)acrylic esters, ethylene glycol di(meth)acrylate, 1,6-hexanediol di(meth)acrylate (HEDMA), triethylene glycol di(meth)acrylate (TEDMA), 1,12-dodecanediol di(meth)acrylate, bisphenol A di(meth)acrylate, alkoxylated bisphenol A di(meth)acrylate, bisphenol B di(meth)acrylate, alkoxylated bisphenol B di(meth)acrylate, bisphenol C di(meth)acrylate, alkoxylated bisphenol C di(meth)acrylate, bisphenol F di(meth)acrylate, alkoxylated bisphenol F di(meth)acrylate, polyethylene glycol di(meth)acrylate, 7,7,9-trimethyl-4,13-dioxo-5,12-diazahexadecane-1,16-dioxydi(meth)acrylate (UDMA), butanediol di(meth)acrylate, tetraethylene glycol di(meth)acrylate, neopentyl glycol di(meth)acrylate, 2-hydroxypropyl-1,3-di(meth)acrylate, 3-hydroxypropyl-1,2-di(meth)acrylate, pentaerythritol di(meth)acrylate, di(meth)acrylates of dihydroxymethyltricyclo[5.2.1.0^{2,6}]decane, 2-hydroxyethyl (meth)acrylate, 2-hydroxypropyl (meth)acrylate, 3-hydroxypropyl (meth)acrylate, 1,2-dihydroxypropyl (meth)acrylate, 1,3-dihydroxypropyl (meth)acrylate, 2,2-bis[4-[3-(meth)acryloyloxy-2- hydroxypropoxy]phenyl]propane (Bis-GMA), trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, ditrimethylolpropane tetra(meth)acrylate, pentaerythritol hexa(meth)acrylate, butylene glycol di(meth)acrylate, propylene glycol di(meth)acrylate, nonanediol di(meth)acrylate, decanediol di(meth)acrylate, glyceryl mono(meth)acrylate, glyceryl di(meth)acrylate, trimethylolpropane mono(meth)acrylate, trimethylolpropane di(meth)acrylate, sorbitol mono-, di-, tri-, tetra- or penta(meth)acrylate, methyl (meth)acrylate, ethyl (meth)acrylate, propyl (meth)acrylate, butyl (meth)acrylate, hexyl (meth)acrylate, tetrahydrofurfuryl (meth)acrylate, lauryl (meth)acrylate, cyclohexyl (meth)acrylate, allyl (meth)acrylate, glycidyl (meth)acrylate, 2-ethoxyethyl (meth)acrylate, methoxy polyethylene glycol (meth)acrylate, isobornyl (meth)acrylate, 2-(N,N-dimethylamino)ethyl (meth)acrylate, N-methylol(meth)acrylamide, diacetone(meth)acrylamide, 2,2-bis[4-(meth)acryloyloxyphenyl]propane, 2,2-bis[4-(meth)acryloyloxyethoxyphenyl]propane, 2,2-bis[4-(meth)acryloyloxydiethoxyphenyl]propane, 2,2-bis[4-(meth)acryloyloxytriethoxyphenyl]propane, 2,2-bis[4-(meth)acryloyloxytetraethoxyphenyl]propane, 2,2-bis[4-(meth)acryloyloxypentaethoxyphenyl]propane, 2,2-bis[4-(meth)acryloyloxydipropoxyphenyl]propane, 2,2-bis[4-(meth)acryloyloxyethoxyphenyl]-2-[4-(meth)acryloyloxydiethoxyphenyl]propane, 2-[4-(meth)acryloyloxydiethoxyphenyl]-2-[4-(meth)acryloyloxytriethoxyphenyl]propane, 2-[4-(meth)acryloyloxdipropoxyphenyl]-2-[4-(meth)acryloyloxytriethoxyphenyl]propane, 2,2-bis[4-meth)acryloyloxyisopropoxyphenyl]propane, hydroxypivalic acid neopentyl glycol di(meth)acrylate, acetoacetoxyethyl (meth)acrylate, polypropylene glycol di(meth)acrylate, glyceryl alkoxylate dimethacrylate, neopentyl glycol (meth)acrylate, N,N-(1,2-dihydroxyethylene)bisacrylamide, 2,2-bis[4-(meth)acryloyloxypentaethoxyphenyl]propane, 2,2-bis[4-(meth)acryloyloxypolyethoxyphenyl]propane, diethylene glycol di(meth)acrylate, dipentaerythritol tetra(meth)acrylate, dipentaerythritol hexa(meth)acrylate, N,N-(2,2,4-trimethylhexamethylene)bis[2-(aminocarboxy)propane-1,3-diol] tetra(meth)acrylate, the condensation product of 3,(4)-(meth)acryloxymethyl-8,(9)-hydroxymethyltricyclo-[5.2.1.0^{2,6}]decane with dicarboxylic acids, 2-ethylhexyl (meth)acrylate, tridecyl (meth)acrylate, stearyl (meth)acrylate, cyclohexyl (meth)acrylate, benzyl (meth)acrylate, methoxy diethylene glycol (meth)acrylate, dicyclopentenyl (meth)acrylate, phenyl (meth)acrylate, pentaerythritol mono(meth)acrylate, dipentaerythritol mono(meth)acrylate, caprolactone modified tetrahydrofurfuryl (meth)acrylate, and light-curable monomers based on polysiloxanes,
constituent (b-2) is selected from the group consisting of amorphous materials based on mixed oxides of SiO₂, ZrO₂ and/or TiO₂, microfine fillers such as fumed silica or precipitated silica, and macro- or mini-fillers such as quartz glass ceramic or glass powder, barium silicate glasses, barium fluorosilicate glasses, strontium silicate glasses, strontium borosilicate, Li/Al silicate glasses, barium glasses, calcium silicates, sodium aluminum silicates, fluoroaluminum silicate glasses, oxides of aluminum or silicon, zeolites, apatite, zirconium silicates, sparingly soluble metal salts such as barium sulfate or calcium fluoride, and X-ray-opaque fillers such as ytterbium fluoride, and nanoscale solid particles having a mean particle size of not more than 200 nm, preferably not more than 100 nm and especially not more than 70 nm, and which are preferably non-agglomerated and/or non-aggregated, the nanoscale inorganic solid particles preferably being those of oxides, sulfides, selenides and tellurides of metals, semimetals and mixtures thereof, such as SiO₂, TiO₂, ZrO₂, ZnO, SnO₂ and Al₂O₃, and organic polymers,
constituent (b-3) is selected from the group consisting of alpha-diketones, benzoin alkyl ethers, thioxanthones, benzophenones, acylphosphine oxides, acetophenones, ketals, titanocenes, sensitizing dyes, and borate salts and benzoyl peroxide, lauroyl peroxide in combination with amines, barbituric acids or barbituric acid derivatives and malonylsulfamides, sodium benzenesulfinates or sodium paratoluenesulfinates in combination with heavy metal salts, constituent (b-4) is selected from the group consisting of hydroquinone monomethyl ether, 2,6-di-tert-butyl-4-methylphenol, 2,2-diphenyl-1-picrylhydrazyl, galvinoxyl and triphenylmethyl radicals, 2,3,6,6-tetramethylpiperidinyl-1-oxyl radicals and derivatives thereof, or phenothiazine,
constituent (b-5) is selected from the group consisting of toluene, xylene, isooctane, acetone, butanone, methyl isobutyl ketone, ethyl acetate, butyl acetate, tetrahydrofuran, N-methylpyrrolidone, dimethylacetamide, dimethylformamide, ethanol, propanols, butanols, pentanols, hexanols, cyclohexanol, heptanols, octanols, nonanols, decanols, and cycloaliphatic or arylaliphatic alcohols.

7. Curable mixture for use in a therapeutic method in the production of a dental material according to claim 6, wherein the stoichiometry of the reactions of
- 3(4),8(9)-bis(hydroxymethyl)tricyclo[5.2.1.0^{2,6}]decane with isocyanate or of alkoxylated 3(4),8(9)-bis(hydroxymethyl)tricyclo[5.2.1.0^{2,6}]decane with isocyanate ranges from 1:1 to 1:2, such that the reaction products may comprise urethanes, allophanates and mixtures thereof in which the urethanes have not reacted fully to give the allophanates,
- 3(4),8(9)-bis(aminomethyl)tricyclo[5.2.1.0^{2,6}]decane with isocyanate or of alkoxylated 3(4),8(9)-bis(aminomethyl)tricyclo[5.2.1.0^{2,6}]decane with isocyanate ranges from 1:1 to 1:2, such that the reaction products may comprise ureas, biurets and mixtures thereof in which the ureas have not reacted fully to give the biurets,
- 3(4),8(9)-bis(carboxylic acid) tricyclo [5.2.1.0^{2,6}] decane with isocyanate ranges from 1:1 to 1:2, such that the reaction products may comprise amides, acylureas and mixtures thereof in which the amides have not reacted fully to give the acylureas,
- 3(4),8(9)-bis(carbonyl halide) tricyclo [5.2.1.0^{2,6}] decane with amine is 1:1 and gives an amide, and the second reaction stage to give the acylurea with a monoisocyanate is effected in a stoichiometry up to 1:1, such that the reaction product may comprise amides, acylureas and mixtures thereof,
- 3(4),8(9)-bis(isocyanatomethyl)tricyclo[5.2.1.0^{2,6}]-decane with alcohol or with alkoxylated alcohol is 1:1 and gives a urethane, and the second reaction stage to give the allophanate with a monoisocyanate is effected in a stoichiometry up to 1:1, such that the reaction product may comprise urethanes, allophanates and mixtures thereof,
- 3(4),8(9)-bis(isocyanatomethyl)tricyclo[5.2.1.0^{2,6}]-decane with amine is 1:1 and gives a urea, and the second reaction stage to give the biuret with a monoisocyanate is effected in a stoichiometry up to 1:1, such that the reaction product may comprise ureas, biurets and mixtures thereof,
- 3(4),8(9)-bis(isocyanatomethyl)tricyclo[5.2.1.0^{2,6}]-decane with carboxylic acid is 1:1 and gives an amide, and the second reaction stage to give the acylurea with a monoisocyanate is effected in a stoichiometry up to 1:1, such that the reaction product may comprise amides, acylureas and mixtures thereof,
- 3(4),8(9)-bis(aminomethyl)tricyclo[5.2.1.0^{2,6}]decane with acid halides is 1:1 and gives an amide, and the second reaction stage to give the acylurea with a monoisocyanate is effected in a stoichiometry up to 1:1, such that the reaction product may comprise amides, acylureas and mixtures thereof.

8. Curable mixture for use in a therapeutic method in the production of a dental material according to any of the preceding claims, wherein
(a) is 0.5 to 20% by weight, preferably 2 to 10% by weight
(b-1) is 5 to 90% by weight, preferably 25 to 80% by weight
(b-2) is 1 to 85% by weight, preferably 20 to 75% by weight
(b-3) is 0.05 to 8% by weight, preferably 0.5 to 4% by weight
(b-4) is 0 to 1% by weight, preferably 0.01 to 0.2% by weight
(b-5) is 0 to 85% by weight, preferably 0 to 75% by weight.

9. Curable mixture for use in a therapeutic method in the production of a dental material according to any of the claims 1 to 7, wherein
(a) is 2 to 10% by weight
(b-1) is 25 to 80% by weight
(b-2) is 20 to 75% by weight
(b-3) is 0.5 to 4% by weight
(b-4) is 0.01 to 0.2% by weight
(b-5) is 0 to 75% by weight.

10. Curable mixture for use in a therapeutic method in the production of a dental material according to claims 6 and 9, comprising
(a) 2 to 10% by weight of a plasticiser selected from the group consisting of 3(4),8(9)-bis(acetyloxymethyl)-tricyclo [5.2.1.0^{2,6}] decane, alkoxylated 3(4),8(9)-bis(acetyloxymethyl)tricyclo[5.2.1.0^{2,6}]decane, 2,3-bis(acetyloxymethyl)bicyclo[2.2.1]heptane, alkoxylated 2,3-bis(acetyloxymethyl)bicyclo[2.2.1]heptane, 1,3,5-triacetyloxytricyclo[3.3.1.1^{3,7}]decane, alkoxylated triacetyloxytricyclo[3.3.1.1^{3,7}]decane, tricyclo[5.2.1.0^{2,6}]decane-3(4),8(9)-dicarboxylic acid ethyl ester and the Michael-type adduct of 3(4),8(9)-bis(aminomethyl)tricyclo[5.2.1.0^{2,6}]decane and methyl acrylate,
(b-1) 25 to 80% by weight of a free-radically curable monomer selected from the group consisting of 7,7,9-trimethyl-4,13-dioxo-5,12-diazahexadecane-1,16-dioxy di(meth)acrylate (UDMA), triethylene glycol di(meth)acrylate (TEDMA) and 3(4),8(9)-bis(methacryloyloxymethyl)tricyclo[5.2.1.0^{2,6}]decane,
(b-2) 20 to 75% by weight of a filler selected from the group consisting of glass ceramic, silicas, X-ray-opaque fillers, nanoscale fillers below 200 nm, the latter preferably being non-agglomerated and/or non-aggregated, and organic polymers,
(b-3) 0.5 to 4% by weight of a photoinitiator selected from the group consisting of camphorquinone/amine and phosphine oxide and/or a chemical initiator selected from the group consisting of peroxide/amine and barbituric acid/barbituric acid derivatives in combination with heavy metal salts.

11. Curable mixture for use in a therapeutic method in the production of a dental material according to any of the preceding claims, wherein constituent (b-2) is organically surface-modified.

12. Curable mixture for use in a therapeutic method in the production of a dental material according claim 11, wherein constituent (b-2) is silanized.

13. Curable mixture for use in a therapeutic method in the production of a dental material according to any of the preceding claims, wherein the dental material is a cured filling material, core buildup material, temporary crown and bridge material, a luting cement, a relining material, a dental material, a modeling material, a base material, a covering composition for gingiva protection, a prosthetic material, the material for a temporary supraconstruction for a dental implant or a core for a temporary supraconstruction, or an inlay, onlay or veneer.

14. Curable mixture for use in a therapeutic method in the production of a dental material according to any of the preceding claims, wherein the curable mixture is a two-component system, constituent (b-3) comprising a redox system comprising a reducing agent and an oxidizing agent, and the curable mixture being in the form of two spatially separate components in the form of pastes, and the reducing agent being present in the first component and the oxidizing agent in the second component, and constituents (a), (b-1), (b-2), optionally (b-4) and optionally (b-5) being present in the first and/or second component, and the pastes being present in mixing ratios of first component to second component in the ratio of 10:1 to 1:10.

15. Curable mixture for use in a therapeutic method according to any of the claims 1 to 12 and 14, which gives a relining material after curing, wherein the relining material has a flexural strength, measured according to ISO 4049, of more than 73 MPa.

16. Curable mixture for use in a therapeutic method according to any of the claims 1 to 12 and 14, which gives a temporary crown and bridge material after curing, wherein the temporary crown and bridge material gives a water solubility, measured according to ISO 4049, of less than 2.5 µg/mm³.

## Revendications

1. Mélange durcissable, comprenant :
(a) un ou plusieurs composés non polymérisables par voie radicalaire pendant le durcissement avec les constituants (b), de structure Q-[(Y)ₙ-X]ₒ, dans laquelle, ici et ultérieurement :
- Q signifie élément structural polyalicyclique choisi dans le groupe constitué par les radicaux hydrocarbonés bicycliques, tricycliques, tétracycliques, pentacycliques et hexacycliques ; facultativement un, deux ou davantage des atomes d'hydrogène non substitués par des substituants Yₙ-X de cet élément structural polyalicyclique Q étant substitués par des groupes alkyle, des groupes alcoxy, des atomes d'halogène ou des groupes trifluorométhyle ;
- Y signifie méthylène (-CH₂-) ;
- n = 0 ou 1 ;
- X signifie -O-Z, -N-(Z)₂, -NH-Z, -O-C(=O)-Z,-C(=O)-O-Z, -O-C(=O)-NH-Z, -NH-C(=O)-O-Z, -NH-C(=O)-NH-Z, -C(=O)-NH-Z, -NH-C(=O)-Z, -C(=O)-N-(Z)₂, -N-(Z)-C(=O)-Z, -O-C(=O)-N(Z)-C(=O)-NH-Z, -NH-C(=O)-N(Z)-C(=O)-NH-Z, -N(C(=O)-NH-Z)₂, -C(=O)-N(Z)-C(=O)-NH-Z, - N(C(=O)-NH-Z)(C(=O)-Z), -N(C(=O)-NH-Z)(C(=O)-O-Z),
la liaison agencée à gauche dans la formule étant à chaque fois plus proche de l'élément structural Q, et X étant choisi de telle sorte que Z contienne le moins d'atomes possible,
- Z signifie un radical organique contenant au moins un atome de carbone, différents Z pouvant être différents,
- o = 2, 3, 4 ;
en tant que plastifiant
et
(b) d'autres constituants choisis dans le groupe constitué par :
(b-1) un ou plusieurs monomères différents,
(b-2) une ou plusieurs charges,
(b-3) un ou plusieurs photoinitiateurs et/ou un ou plusieurs initiateurs pour le durcissement chimique, et
(b-4) un ou plusieurs inhibiteurs de polymérisation, destiné à une utilisation dans un procédé thérapeutique lors de la fabrication d'un matériau dentaire.

2. Mélange durcissable destiné à une utilisation dans un procédé thérapeutique lors de la fabrication d'un matériau dentaire selon la revendication 1, **caractérisé en ce que** Z est choisi dans le groupe constitué par :
a) les radicaux hydrocarbonés [-R₁], le nombre d'atomes C étant de 1 à 30 et les radicaux pouvant être linéaires ou ramifiés, et
b) les radicaux hydrocarbonés éther [(-R₂-O)_{q}-R₃], le nombre d'atomes C pour R₂ étant de 2 à 6, et le nombre d'atomes C pour R₃ étant de 1 à 20, et R₂ et R₃ pouvant être linéaires ou ramifiés, et q = 1 à 15, et
c) les radicaux hydrocarbonés ester [-R₄-(C=O)-O-R₅] et [-R₄-O-(C=O)-R₅], le nombre d'atomes C pour R₄ et R₅ étant de 1 à 15, et R₄ et R₅ pouvant être linéaires ou ramifiés, et
d) les radicaux hydrocarbonés ester [-R₄-((C=O)-O-R₅)₂] et [-R₄-(O-(C=O)-R₅)₂], et
e) les radicaux hydrocarbonés amino [-R₄-N-(R₅)₂] et
f) les radicaux hydrocarbonés ester alcoxylés [(-R₂-O)_{q}-R₄-(C=O)-O-R₅] et [(-R₂-O)_{q}-R₄-O-(C=O)-R₅] et
g) les radicaux hydrocarbonés ester alcoxylés substitués [(-R₂-O)_{q}-CH₂-O-(C=O)-NH-R₄-(C=O)-O-R₅] et
h) les radicaux hydrocarbonés alcool contenant un groupe hydroxyle terminal [-R₄-OH] et
i) les radicaux hydrocarbonés alcool contenant un, deux ou davantage de groupes hydroxyle non terminaux/terminaux et jusqu'à 30 atomes C, et
j) les radicaux hydrocarbonés alcool alcoxylés [(-R₂-O)_{q}-R₄-OH] et
k) les radicaux cétone [-R₆-(C=O)-R₇], le nombre d'atomes C pour R₆ étant de 1 à 30, et le nombre d'atomes C pour R₇ étant de 1 à 30, et R₆ et R₇ pouvant être linéaires ou ramifiés.

3. Mélange durcissable destiné à une utilisation dans un procédé thérapeutique lors de la fabrication d'un matériau dentaire selon la revendication 1, **caractérisé en ce que** Z est choisi dans le groupe constitué par :
a) les radicaux hydrocarbonés [-R₁], le nombre d'atomes C étant de 1 à 9 et les radicaux pouvant être linéaires ou ramifiés, et
b) les radicaux hydrocarbonés éther [(-R₂-O)_{q}-R₃], le nombre d'atomes C pour R₂ étant de 2 à 3, et le nombre d'atomes C pour R₃ étant de 1 à 5, et R₂ et R₃ pouvant être linéaires ou ramifiés, et q = 1 à 5, et
c) les radicaux hydrocarbonés ester [-R₄-(C=O)-O-R₅] et [-R₄-O-(C=O)-R₅], le nombre d'atomes C pour R₄ et R₅ étant de 1 à 4, et R₄ et R₅ pouvant être linéaires ou ramifiés, et
d) les radicaux hydrocarbonés ester [-R₄-((C=O)-O-R₅)₂] et [-R₄-(O-(C=O)-R₅)₂], et
e) les radicaux hydrocarbonés amino [-R₄-N-(R₅)₂] et
f) les radicaux hydrocarbonés ester alcoxylés [(-R₂-O)_{q}-R₄-(C=O)-O-R₅] et [(-R₂-O)_{q}-R₄-O-(C=O)-R₅] et
g) les radicaux hydrocarbonés ester alcoxylés substitués [(-R₂-O)_{q}-CH₂-O-(C=O)-NH-R₄-(C=O)-O-R₅] et
h) les radicaux hydrocarbonés alcool [-R₄-OH] et
i) les radicaux hydrocarbonés alcool contenant un, deux ou davantage de groupes hydroxyle non terminaux/terminaux et jusqu'à 30 atomes C, et
j) les radicaux hydrocarbonés alcool alcoxylés [(-R₂-O)_{q}-R₄-OH] et
k) les radicaux cétone [-R₆-(C=O)-R₇], le nombre d'atomes C pour R₆ étant de 1 à 10, et le nombre d'atomes C pour R₇ étant de 1, et R₆ pouvant être linéaire ou ramifié.

4. Mélange durcissable destiné à une utilisation dans un procédé thérapeutique lors de la fabrication d'un matériau dentaire selon la revendication 1, 2 ou 3, **caractérisé en ce que** Q est un élément structural polyalicyclique saturé, choisi dans le groupe constitué par les radicaux hydrocarbonés bicycliques ou tricycliques, aucun des atomes d'hydrogène non substitués par des substituants Yₙ-X de cet élément structural polyalicyclique Q n'étant substitué.

5. Mélange durcissable destiné à une utilisation dans un procédé thérapeutique lors de la fabrication d'un matériau dentaire selon la revendication 1, 2, 3 ou 4, **caractérisé en ce que** Q signifie un radical tricyclo[5.2.1.0^{2,6}]décane, un radical tricyclo[3.3.1.1^{3,7}]décane ou un radical bicyclo[2.2.1]heptane.

6. Mélange durcissable destiné à une utilisation dans un procédé thérapeutique lors de la fabrication d'un matériau dentaire selon l'une quelconque des revendications précédentes, **caractérisé en ce que**
le constituant (a) est choisi dans le groupe constitué par le 3(4),8(9)-bis(acyloxyméthyl)tricyclo[5.2.1.0^{2,6}]décane, le 3(4),8(9)-bis(acyloxyméthyl)tricyclo[5.2.1,0^{2,6}]décane alcoxylé, le 2,3-bis(acyloxyméthyl)bicyclo[2.2.1]heptane, le 2,3-bis(acyloxyméthyl)bicyclo[2.2.1]heptane alcoxylé, le 1,3,5-triacyloxytricyclo[3.3.1.1^{3,7}]décane, le triacyloxytricyclo[3.3.1.1^{3,7}]décane alcoxylé, l'ester de l'acide tricyclo[5.2.1,0^{2,6}]décane-3(4),8(9)-dicarboxylique, l'adduit de type Michael de 3(4),8(9)-bis(aminométhyl)tricyclo[5.2.1.0^{2,6}] décane et d'un ester de l'acide acrylique, le 3(4),8(9)-bis-(alkyloxyméthyl)tricyclo[5.2.1.0^{2,6}]décane, le 3(4),8(9)-bis-(alkyloxyméthyl)tricyclo[5.2.1.0^{2,6}]décane alcoxylé, le produit d'addition de 3(4),8(9)-bis(hydroxyméthyl)tricyclo[5.2.1.0^{2,6}] décane avec un isocyanate, le produit d'addition de 3(4),8(9)-bis(hydroxyméthyl)tricyclo[5.2.1.0^{2,6}]décane alcoxylé avec un isocyanate, le produit d'addition de 3(4),8(9)-bis(aminométhyl)tricyclo[5.2.1.0^{2,6}] décane avec un isocyanate ou le produit de réaction de 3(4),8(9)-bis(isocyanatométhyl)tricyclo[5.2.1,0^{2,6}]décane avec des amines, le produit d'addition de 3(4),8(9)-bis(aminométhyl)tricyclo[5.2.1.0^{2,6}]décane alcoxylé avec un isocyanate, le produit d'addition de 3(4),8(9)-bis(isocyanatométhyl)tricyclo[5.2.1,0^{2,6}]décane avec un alcool, le produit d'addition de 3(4),8(9)-bis(isocyanatométhyl)tricyclo[5.2.1.0^{2,6}]décane avec un alcool alcoxylé, le produit de réaction de 3(4),8(9)-bis(halogénure d'acide carboxylique)tricyclo[5.2.1.0^{2,6}]décane avec une amine, ou le produit de réaction de 3(4),8(9)-bis(acide carboxylique)tricyclo[5.2.1.0^{2,6}]décane avec un isocyanate, le produit de réaction de 3(4),8(9)-bis(isocyanatométhyl)tricyclo[5.2.1,0^{2,6}]décane avec un alcool aminé, le produit de réaction de 3(4),8(9)-bis(isocyanatométhyl)tricyclo[5.2.1.0^{2,6}]décane avec un alcool aminé et alcoxylation ultérieure de l'alcool résultant, le produit de réaction de 3(4),8(9)-bis(isocyanatométhyl)tricyclo[5.2.1.0^{2,6}]décane avec un alcool aminé, alcoxylation ultérieure de l'alcool résultant et réaction de ce composé avec un acide carboxylique/anhydride d'acide carboxylique, le produit de réaction de 3(4),8(9)-bis(aminométhyl)tricyclo[5.2.1.0^{2,6}]décane avec des halogénures d'alkyle, le produit de réaction de 3(4), 8(9)-bis(aminométhyl)tricyclo[5.2.1.0^{2,6}]décane avec des halogénures d'acides, ou le produit de réaction de 3(4),8(9)-bis(isocyanatométhyl)tricyclo[5.2.1,0^{2,6}]décane avec des acides carboxyliques, le produit de réaction de 3(4),8(9)-bis(hydroxyméthyl)tricyclo[5.2.1,0^{2,6}]décane ou de 3(4),8(9)-bis(hydroxyméthyl)tricyclo[5.2.1.0^{2,6}]décane alcoxylé avec des acides gras, ainsi que leurs réactions supplémentaires dans le cas de produits de réaction d'acides gras insaturés en les cétones correspondantes selon le procédé de Wacker, le produit de réaction de 3(4),8(9)-bis(aminométhyl)tricyclo[5.2.1.0^{2,6}]décane avec des époxydes et son produit de réaction supplémentaire avec des acides carboxyliques, le 3(4),8(9)-bis(hydroxyméthyl)tricyclo[5.2.1.0^{2,6}]décane alcoxylé et le 3(4),8(9)-bis(aminométhyl)tricyclo[5.2.1.0^{2,6}]décane alcoxylé,
le constituant (b-1) est choisi dans le groupe constitué par le 3(4),8(9)-bis((méth)acryloyloxyméthyl)tricyclo[5.2.1.0^{2,6}]décane, le 3(4),8(9)-bis((méth)acryloyloxyméthyl)tricycle[5.2.1.0^{2,6}]décane alcoxylé, le 2,3-bis((méth)acryloyloxyméthyl)bicyclo[2.2.1]heptane, le 2,3-bis((méth)acryloyloxyméthyl)bicyclo[2.2.1]heptane alcoxylé, le 1,3,5-tri(méth)acryloyloxytricyclo[3.3.1.1^{3,7}]décane, le tri(méth)acryloyloxytricyclo[3.3.1.1^{3,7}]décane alcoxylé, l'ester de l'acide (méth)acrylique de tricyclo[5.2.1.0^{2,6}]décane-3(4),8(9)-diméthanol, le tricyclo[5.2.1.0^{2,6}]décane-3(4),8(9)-diméthanol alcoxylé, le bicyclo[2.2.1]-heptane-2,3-diméthanol, le bicyclo[2.2.1]heptane-2,3-diméthanol alcoxylé, le 1,3,5-adamantane-triol, le 1,3,5-adamantane-triol alcoxylé, des groupes uréthane, urée, amide, allophanate, acylurée ou biuret étant agencés entre l'élément structural polyalicyclique et les esters de l'acide (méth)acrylique, le di(méth)acrylate d'éthylène glycol, le di(méth)acrylate de 1,6-hexane-diol (HEDMA), le di(méth)acrylate de triéthylène glycol (TEDMA), le di(méth)acrylate de 1,12-dodécane-diol, le di(méth)acrylate de bisphénol A, le di(méth)acrylate de bisphénol A alcoxylé, le di(méth)acrylate de bisphénol B, le di(méth)acrylate de bisphénol B alcoxylé, le di(méth)acrylate de bisphénol C, le di(méth)acrylate de bisphénol C alcoxylé, le di(méth)acrylate de bisphénol F, le di(méth)acrylate de bisphénol F alcoxylé, le di(méth)acrylate de polyéthylène glycol, le 1,16-dioxydi(méth)acrylate de 7,7,9-triméthyl-4,13-dioxo-5,12-diazahexadécane (UDMA), le di(méth)acrylate de butanediol, le di(méth)acrylate de tétraéthylène glycol, le di(méth)acrylate de néopentylglycol, le 1,3-di(méth)acrylate de 2-hydroxypropyle, le 1,2-di(méth)acrylate de 3-hydroxypropyle, le di(méth)acrylate de pentaérythritol, les di(méth)acrylates de dihydroxyméthyltricyclo[5.2.1,0^{2,6}]décane, le (méth)acrylate de 2-hydroxyéthyle, le (méth)acrylate de 2-hydroxypropyle, le (méth)acrylate de 3-hydroxypropyle, le (méth)acrylate de 1,2-dihydroxypropyle, le (méth)acrylate de 1,3-dihydroxypropyle, le 2,2-bis[4-[3-(méth)acryloyloxy-2-hydroxypropoxy]phényl]propane (bis-GMA), le tri(méth)acrylate de triméthylolpropane, le tri(méth)acrylate de triméthyloléthane, le tri(méth)acrylate de pentaérythritol, le tri(méth)acrylate de triméthylolméthane, le tétra(méth)acrylate de pentaérythritol, le tétra(méth)acrylate de ditriméthylolpropane, l'hexa(méth)acrylate de pentaérythritol, le di(méth)acrylate de butylène glycol, le di(méth)acrylate de propylène glycol, le di(méth)acrylate de nonane-diol, le di(méth)acrylate de décane-diol, le mono(méth)acrylate de glycérol, le di(méth)acrylate de glycérol, le mono(méth)acrylate de triméthylolpropane, le di(méth)acrylate de triméthylolpropane, le mono-, di-, tri-, tétra- ou penta(méth)acrylate de sorbitol, le (méth)acrylate de méthyle, le (méth)acrylate d'éthyle, le (méth)acrylate de propyle, le (méth)acrylate de butyle, le (méth)acrylate d'hexyle, le (méth)acrylate de tétrahydrofurfuryle, le (méth)acrylate de lauryle, le (méth)acrylate de cyclohexyle, le (méth)acrylate d'allyle, le (méth)acrylate de glycidyle, le (méth)acrylate de 2-éthoxyéthyle, le (méth)acrylate de méthoxypolyéthylène glycol, le (méth)acrylate d'isobornyle, le (méth)acrylate 2-(N,N-diméthylamino)éthyle, le N-méthylol(méth)acrylamide, le diacétone-(méth)acrylamide, le 2,2-bis[4-(méth)acryloyloxyphényl]-propane, le 2,2-bis[4-(méth)acryloyloxyéthoxyphényl]propane, le 2,2-bis[4-(méth)acryloyloxydiéthoxyphényl]propane, le 2,2-bis[4-(méth)acryloyloxytriéthoxyphényl]propane, le 2,2-bis[4-(méth)acryloyloxytétraéthoxyphényl]propane, le 2,2-bis[4-(méth)acryloyloxypentaéthoxyphényl]propane, le 2,2-bis[4-(méth)acryloyloxydipropoxyphényl]propane, le 2,2-bis[4-(méth)acryloyloxy-éthoxyphényl]-2-[4-(méth)acryloyloxydiéthoxyphényl]propane, le 2-[4-(méth)acryloyloxydiéthoxyphényl]-2-[4-(méth)acryloyloxytriéthoxyphényl]propane, le 2-[4-(méth)acryloyloxdipropoxyphényl]-2-[4-(méth)acryloyloxytriéthoxyphényl]propane, le 2,2-bis[4-(méth)acryloyloxyisopropoxyphényl]propane, le di(méth)acrylate de néopentylglycol de l'acide hydroxypivalique, le (méth)acrylate d'acétoacétoxyéthyle, le di(méth)acrylate de polypropylène glycol, l'alcoxylate-diméthacrylate de glycérol, le (méth)acrylate de néopentyl glycol, le N,N-(1,2-dihydroxyéthylène)bisacrylamide, le 2,2-bis[4-(méth)acryloyloxypentaéthoxyphényl]propane, le 2,2-bis[4-(méth)acryloyloxypolyéthoxyphényl]-propane, le di(méth)acrylate de diéthylène glycol, le tétra(méth)acrylate de dipentaérythritol, l'hexa(méth)acrylate de dipentaérythritol, le tétra(méth)acrylate de N,N-(2,2,4-triméthylhexaméthylène)bis[2-(aminocarboxy)propane-1,3-diol], le produit de condensation de 3,(4)-(méth)acryloxyméthyl-8, (9)-hydroxyméthyltricyclo-[5.2.1.0^{2,6}] décane avec des acides dicarboxyliques, le (méth)acrylate de 2-éthylhexyle, le (méth)acrylate de tridécyle, le (méth)acrylate de stéaryle, le (méth)acrylate de cyclohexyle, le (méth)acrylate de benzyle, le (méth)acrylate de méthoxydiéthylène glycol, le (méth)acrylate de dicyclopentényle, le (méth)acrylate de phényle, le mono(méth)acrylate de pentaérythritol, le mono(méth)acrylate de dipentaérythritol, le (méth)acrylate de tétrahydrofurfuryle modifié par de la caprolactone, ainsi que les monomères polymérisables à base de polysiloxanes,
le constituant (b-2) est choisi dans le groupe constitué par les matériaux amorphes à base d'oxydes mixtes de SiO₂, ZrO₂ et/ou TiO₂, les charges microfines telles que la silice pyrogénée ou la silice précipitée, ainsi que les macro- ou mini-charges telles que la vitrocéramique de quartz ou la poudre de verre, les verres de silicate de baryum, les verres de fluorosilicate de baryum, les verres de silicate de strontium, le borosilicate de strontium, les verres de silicate de Li/Al, les verres de baryum, les silicates de calcium, les silicates de sodium et d'aluminium, les verres de silicate de fluor et d'aluminium, les oxydes d'aluminium ou de silicium, les zéolithes, l'apatite, les silicates de zirconium, les sels de métaux difficilement solubles tels que le sulfate de baryum ou le fluorure de calcium, ainsi que les charges opaques aux rayons X telles que le fluorure d'ytterbium, ainsi que les particules solides nanométriques ayant une taille de particule moyenne non supérieure à 200 nm, de préférence non supérieure à 100 nm et notamment non supérieure à 70 nm, et qui sont de préférence non agglomérées et/ou non agrégées, les particules solides inorganiques nanométriques étant de préférence celles d'oxydes, de sulfures, de séléniures et de tellurures de métaux, de semi-métaux et leurs mélanges, tels que SiO₂, TiO₂, ZrO₂, ZnO, SnO₂ et Al₂O₃, et de polymères organiques,
le constituants (b-3) est choisi dans le groupe constitué par les alpha-dicétones, l'éther alkylique de benzoïne, les thioxanthones, les benzophénones, les oxydes d'acylphosphine, les acétophénones, les cétals, les titanocènes, les colorants sensibilisants, ainsi que les sels de borate et le peroxyde de benzoyle, le peroxyde de lauroyle en combinaison avec des amines, des acides barbituriques ou des dérivés d'acides barbituriques, ainsi que les malonylsulfamides, les benzène-sulfinates de sodium ou les paratoluène-sulfinates de sodium en combinaison avec des sels de métaux lourds,
le constituant (b-4) est choisi dans le groupe constitué par l'éther monométhylique d'hydroquinone, le 2,6-di-tert.-butyl-4-méthylphénol, les radicaux 2,2-diphényl-1-picrylhydrazyle, galvinoxyle, triphénylméthyle, le radical 2,3,6,6-tétraméthylpipéridinyl-1-oxyle, ainsi que leurs dérivés, ou la phénothiazine,
le constituant (b-5) est choisi dans le groupe constitué par le toluène, le xylène, l'isooctane, l'acétone, la butanone, la méthylisobutylcétone, l'acétate d'éthyle, l'acétate de butyle, le tétrahydrofurane, la N-méthylpyrrolidone, le diméthylacétamide, le diméthylformamide, l'éthanol, les propanols, les butanols, les pentanols, les hexanols, le cyclohexanol, les heptanols, les octanols, les nonanols, les décanols, ainsi que les alcools cycloaliphatiques ou arylaliphatiques.

7. Mélange durcissable destiné à une utilisation dans un procédé thérapeutique lors de la fabrication d'un matériau dentaire selon la revendication 6, **caractérisé en ce que** la stoechiométrie des réactions de
- 3(4),8(9)-bis(hydroxyméthyl)tricyclo[5.2.10^{2,6}]décane avec un isocyanate ou de 3(4),8(9)-bis(hydroxyméthyl)tricyclo[5.2.1.0^{2,6}]décane alcoxylé avec un isocyanate est de 1:1 à 1:2, de telle sorte que les produits de réaction puissent contenir des uréthanes, des allophanates et leurs mélanges dans lesquels les uréthanes n'ont pas été entièrement transformés en allophanates,
- 3(4),8(9)-bis(aminométhyl)tricyclo[5.2.1.0^{2,6}]décane avec un isocyanate ou de 3(4),8(9)-bis(aminométhyl)tricyclo[5.2.10^{2,6}]décane alcoxylé avec un isocyanate est de 1:1 à 1:2, de telle sorte que les produits de réaction puissent contenir des urées, des biurets et leurs mélanges dans lesquels les urées n'ont pas été entièrement transformées en biurets,
- 3(4),8(9)-bis(acide carboxylique)tricyclo[5.2.1.0^{2,6}]décane avec un isocyanate est de 1:1 à 1:2, de telle sorte que les produits de réaction puissent contenir des amides, des acylurées et leurs mélanges dans lesquels les amides n'ont pas été entièrement transformés en acylurées,
- 3(4),8(9)-bis(halogénure d'acide carboxylique)tricyclo[5.2.1.0^{2,6}]décane avec une amine est de 1:1 et donne un amide, et la deuxième étape de réaction en acylurée a lieu avec un monoisocyanate en une stoechiométrie de jusqu'à 1:1, de telle sorte que le produit de réaction puisse contenir des amides, des acylurées et leurs mélanges,
- 3(4),8(9)-bis(isocyanatométhyl)tricyclo[5.2.1.0^{2,6}]décane avec un alcool ou un alcool alcoxylé est de 1:1 et donne un amide, et la deuxième étape de réaction en allophanate a lieu avec un monoisocyanate en une stoechiométrie de jusqu'à 1:1, de telle sorte que le produit de réaction puisse contenir des uréthanes, des allophanates et leurs mélanges,
- 3(4),8(9)-bis(isocyanatométhyl)tricyclo[5.2.1.0^{2,6}]décane avec une amine est de 1:1 et donne une urée, et la deuxième étape de réaction en biuret a lieu avec un monoisocyanate en une stoechiométrie de jusqu'à 1:1, de telle sorte que le produit de réaction puisse contenir des urées, des biurets et leurs mélanges,
- 3(4),8(9)-bis(isocyanatométhyl)tricyclo[5.2.1.0^{2,6}]décane avec un acide carboxylique est de 1:1 et donne un amide, et la deuxième étape de réaction en acylurée a lieu avec un monoisocyanate en une stoechiométrie de jusqu'à 1:1, de telle sorte que le produit de réaction puisse contenir des amides, des acylurées et leurs mélanges,
- 3(4),8(9)-bis(aminométhyle)tricyclo[5.2.1.0^{2,6}]décane avec des halogénures d'acides est de 1:1 et donne un amide, et la deuxième étape de réaction en acylurée a lieu avec un monoisocyanate en une stoechiométrie de jusqu'à 1:1, de telle sorte que le produit de réaction puisse contenir des amides, des acylurées et leurs mélanges.

8. Mélange durcissable destiné à une utilisation dans un procédé thérapeutique lors de la fabrication d'un matériau dentaire selon l'une quelconque des revendications précédentes, **caractérisé en ce que**
(a) est de 0,5 à 20 % en poids, de préférence 2 à 10 % en poids,
(b-1) est de 5 à 90 % en poids, de préférence 25 à 80 % en poids,
(b-2) est de 1 à 85 % en poids, de préférence 20 à 75 % en poids,
(b-3) est de 0,05 à 8 % en poids, de préférence 0,5 à 4 % en poids,
(b-4) est de 0 à 1 % en poids, de préférence 0,01 à 0,2 % en poids,
(b-5) est de 0 à 85 % en poids, de préférence 0 à 75 % en poids.

9. Mélange durcissable destiné à une utilisation dans un procédé thérapeutique lors de la fabrication d'un matériau dentaire selon l'une quelconque des revendications 1 à 7, **caractérisé en ce que**
(a) est de 2 à 10 % en poids,
(b-1) est de 25 à 80 % en poids,
(b-2) est de 20 à 75 % en poids,
(b-3) est de 0,5 à 4 % en poids,
(b-4) est de 0,01 à 0,2 % en poids,
(b-5) est de 0 à 75 % en poids.

10. Mélange durcissable destiné à une utilisation dans un procédé thérapeutique lors de la fabrication d'un matériau dentaire selon les revendications 6 et 9, contenant :
(a) 2 à 10 % en poids d'un plastifiant, choisi dans le groupe constitué par le 3(4),8(9)-bis(acétyloxyméthyl)tricyclo[5.2.1.0^{2,6}]décane, le 3(4),8(9)-bis(acétyloxyméthyl)tricyclo[5.2.1.0^{2,6}]décane alcoxylé, le 2,3-bis(acétyloxyméthyl)bicyclo[2.2.1]heptane, le 2,3-bis(acétyloxyméthyl)bicyclo[2.2.1]heptane alcoxylé, le 1,3,5-triacétyloxytricyclo[3.3.1.1^{3,7}]décane, le triacétyloxytricyclo[3.3.1.1^{3,7}]décane alcoxylé, l'ester éthylique de l'acide tricyclo[5.2.1,0^{2,6}]décane-3(4),8(9)-dicarboxylique et l'adduit de type Michael de 3(4),8(9)-bis(aminométhyl)tricyclo[5.2.1.0^{2,6}]décane et d'ester méthylique de l'acide acrylique,
(b-1) 25 à 80 % en poids d'un monomère durcissable par voie radicalaire, choisi dans le groupe constitué par le 1,16-dioxydi(méth)acrylate de 7,7,9-triméthyl-4,13-dioxo-5,12-diazahexadécane (UDMA), le di(méth)acrylate de triéthylène glycol (TEDMA) et le 3(4),8(9)-bis(méthacryloyloxyméthyl)tricyclo[5.2.1.0^{2,6}]décane,
(b-2) 20 à 75 % en poids d'une charge, choisie dans le groupe constitué par la vitrocéramique, les silices, les charges opaques aux rayons X, les charges nanométriques d'une taille inférieure à 200 nm, ces dernières étant de préférence non agglomérées et/ou non agrégées, ainsi que les polymères organiques,
(b-3) 0,5 à 4 % en poids d'un photoinitiateur, choisi dans le groupe constitué par la camphre-quinone/amine et l'oxyde de phosphine, et/ou un initiateur chimique, choisi dans le groupe constitué par un peroxyde/amine et l'acide barbiturique/dérivés de l'acide barbiturique en combinaison avec des sels de métaux lourds.

11. Mélange durcissable destiné à une utilisation dans un procédé thérapeutique lors de la fabrication d'un matériau dentaire selon l'une quelconque des revendications précédentes, **caractérisé en ce que** le constituant (b-2) est modifié organiquement en surface.

12. Mélange durcissable destiné à une utilisation dans un procédé thérapeutique lors de la fabrication d'un matériau dentaire selon la revendication 11, **caractérisé en ce que** le constituant (b-2) est silanisé.

13. Mélange durcissable destiné à une utilisation dans un procédé thérapeutique lors de la fabrication d'un matériau dentaire selon l'une quelconque des revendications précédentes, **caractérisé en ce que** le matériau dentaire est un matériau de remplissage durci, un matériau de reconstitution de moignon, un matériau de couronne ou de bridge provisoire, un ciment de fixation, un matériau de rebasage, un matériau technique dentaire, un matériau de fond de cavité, une matière de recouvrement pour la protection de gencives, un matériau de prothèse, le matériau d'une superstructure provisoire pour un implant dentaire ou un noyau pour une superstructure provisoire, ou un inlay, un onlay ou une facette.

14. Mélange durcissable destiné à une utilisation dans un procédé thérapeutique lors de la fabrication d'un matériau dentaire selon l'une quelconque des revendications précédentes, **caractérisé en ce que** le mélange durcissable est un système bicomposant, le constituant (b-3) comprenant un système redox, contenant un réducteur et un oxydant, et le mélange durcissable se présentant en deux composants séparés l'un de l'autre dans l'espace sous la forme de pâtes, et le réducteur étant contenu dans le premier composant et l'oxydant étant contenu dans le deuxième composant, et les constituants (a), (b-1), (b-2), éventuellement (b-4) et éventuellement (b-5) étant présents dans le premier et/ou le deuxième composant, et les pâtes étant présentes en des rapports de mélange entre le premier composant et le deuxième composant de 10:1 à 1:10.

15. Mélange durcissable destiné à une utilisation dans un procédé thérapeutique selon l'une quelconque des revendications 1 à 12 et 14, qui donne après le durcissement un matériau de rebasage, **caractérisé en ce que** le matériau de rebasage présente une résistance à la flexion, mesurée selon ISO 4049, de plus de 73 MPa.

16. Mélange durcissable destiné à une utilisation dans un procédé thérapeutique selon l'une quelconque des revendications 1 à 12 et 14, qui donne après le durcissement un matériau de couronne et de bridge provisoire, **caractérisé en ce que** le matériau de couronne et de bridge provisoire présente une solubilité, mesurée selon ISO 4049, de moins de 2,5 µg/mm³.
